# EUROPEAN PATENT SPECIFICATION

(11) **EP 2 663 559 B1**
(45) Date of publication and mention of the grant of the patent: **01.07.2015**
(21) Application number: 12700232.7
(22) Date of filing: 11.01.2012
(51) Int. Cl.: C07D 413/12, C07D 471/04, A61K 31/5377, A61K 31/437, A61K 31/444, A61K 31/497, A61P 25/00

(54) **OXAZINE DERIVATIVES AND THEIR USE IN THE TREATMENT OF NEUROLOGICAL DISORDERS**
OXAZINE-DERIVATE UND IHRE VERWENDUNG ZUR BEHANDLUNG VON NEUROLOGISCHEN ERKRANKUNGEN
DÉRIVÉS D'OXAZINE ET LEUR UTILISATION DANS LE TRAITEMENT DE MALADIES NEUROLOGIQUES

(30) Priority: 12.01.2011 US 201161432037 P
(43) Date of publication of application: 20.11.2013
(73) Proprietor: Novartis AG, 4056 Basel (CH)
(72) Inventor: HURTH, Konstanze, CH-4002 Basel (CH); LUEOEND, Rainer Martin, CH-4002 Basel (CH); MACHAUER, Rainer, CH-4002 Basel (CH); NEUMANN, Ulf, CH-4002 Basel (CH); RUEEGER, Heinrich, CH-4002 Basel (CH); SCHAEFER, Michael, CH-4002 Basel (CH); TINTELNOT-BLOMLEY, Marina, CH-4002 Basel (CH); VEENSTRA, Siem Jacob, CH-4002 Basel (CH); VOEGTLE, Markus, CH-4002 Basel (CH)
(74) Representative: Campbell, Lachlan Clive
(86) International application number: PCT/EP2012/050387
(87) International publication number: WO 2012/095463

(56) References cited:
- EP-A1- 2 360 155
- WO-A1-2011/009943
- WO-A1-2012/006953

## Description

Alzheimer's Disease is a devastating neurodegenerative disorder. Its sporadic forms affect an elderly population (sharp increase in incidence at >75 years of age), in addition, there are various familial forms with an onset of the disease in the fourth or fifth decade of life. Pathologically, it is characterized by the presence of extracellular senile plaques, and intracellular neurofibrillar tangles in patient's brains. The core constituent of the senile plaques are small, 4 kDa amyloid peptides. They are generated by the proteolytic processing of a large transmembrane protein, amyloid precursor protein (APP). Cleavage of APP by beta-secretase (BACE-1) releases the soluble APP-beta fragment, while the 99-amino acid long C-terminus remains tethered to the membrane. This C-terminal fragment is subsequently proteolytically processed by gamma-secretase (an membrane multi-enzyme complex) to generate amyloid peptides of various length, predominantly 40 and 42 amino acids long (Hardy J, Selkoe DJ (2002) Science; 297 (5580):353-356).

If, under pathologic conditions, the generation of these peptides occurs at an increased rate, or if their removal from the brain is disturbed, increased brain amyloid peptide concentrations leads to the formation of oligomers, fibrils and eventually plaques (Farris W, et al (2007) Am.J. Pathol.; 171 (1):241-251). It has been shown, that deposition of amyloid peptides and plaques in the brain is the first measurable event in the pathogenesis of Alzheimers Disease, and that it is the trigger for loss of synapses, synaptic contacts, and neurons (Grimmer T, et al (2009) Neurobiology of Aging; 30 (12):1902-1909). Brain atrophy caused by massive neuron loss is followed by impairments in cognition, memory, orientation and the ability to perform the tasks of daily living, i.e. clinically manifest dementia (Okello A, et al (2009) Neurology; 73 (10):754-760).

BACE-1, also known as Asp2 or Memapsin 2, is a transmembrane aspartic protease highly expressed in neurons. It co-localizes with its substrate APP in Golgi and endocytic compartments (Willem M, Lammich S, Haass C (2009) Semin.Cell Dev.Biol; 20 (2):175-182). Knock-out studies in mice have demonstrated the absence of amyloid peptide formation, while the animals are healthy and fertile (Ohno M, et al (2007) Neurobiol.Dis.; 26 (1):134-145). Genetic ablation of BACE-1 in APP-overexpressing mice has demonstrated absence of plaque formation and the reversal of cognitive deficits (Ohno M, et al (2004) Neuron; 41 (1):27-33). BACE-1 levels are elevated in the brains of sporadic Alzheimer's Disease patients (Hampel H, Shen Y (2009) Scand. J. Clin. Lab. Invest.; 69 (1):8-12).

Taken together, these findings suggest that the inhibition of BACE-1 may be a favourable therapeutic strategy for Alzheimer's Disease.
EP2360155A1 describes 2-aminopyridin-4-one derivatives having BACE-1 inhibitory activity.

The present invention relates to novel oxazine derivatives having BACE inhibitory activity, to their preparation, to their medical use and to medicaments comprising them.

More particularly, in a first aspect, the invention relates to compounds of the formula (I) 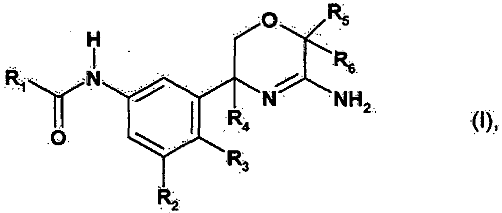
wherein R₁, R₂, R₃, R₄, R₅ and R₆ are defined so as to provide a compound selected from: 5-Chloro-3-methoxymethyl-pyridine-2-carboxylic acid [3-(5-amino-3-difluoromethyl-3,6-dihydro-2H-[1,4] oxazin-3-yl)-4-fluoro-phenyl]-amide;
3-Chloro-5-cyano-pyridine-2-carboxylic acid [3-(5-amino-3-difluormethyl-3,6-dihydro-2H [1,4]oxazin-3-yl)-4-fluoro-phenyl]-amide;
3-Amino-5-tris-deutero-methoxy-pyrazine-2-carboxylic acid [3-(5-amino-3-difluoromethyl-3,6-dihydro-2H-[1,4]oxazin-3-yl)-4-fluoro-phenyl]-amide;
3-Amino-5-prop-2-ynyloxy-pyrazine-2-carboxylic acid [3-(5-amino-3-difluoromethyl-3,6-dihydro-2H-[1,4]oxazin-3-yl)-4-fluoro-phenyl]-amide;
3-Chloro-1H-pyrrolo[2,3-b]pyridine-6-carboxylic acid [3-(5-amino-3-difluoromethyl-3,6-dihydro-2H-[1,4]oxazin-3-yl)-4-fluoro-phenyl]-amide;
3-Amino-5-difluoromethyl-pyrazine-2-carboxylic acid [3-(5-amino-3-difluoromethyl-3,6-dihydro-2H-[1,4]oxazin-3-yl)-4-fluoro-phenyl]-amide;
5-Methoxy-3-methyl-pyridine-2-carboxylic acid [3-(5-amino-3-difluoromethyl-3,6-dihydro-2H-[1,4]oxazin-3-yl)-4-fluoro-phenyl]-amide;
5-Difluoromethyl-3-methyl-pyridine-2-carboxilic acid [3-(5-amino-3-difluoromethyl-3,6-dihydro-2H-[1,4]oxazin-3-yl)-4-fluoro-phenyl]-amide;
5-Fluoro-3-trideuteromethoxymethyl-pyridine-2-carboxylic acid [3-(5-amino-3-difluoromethyl-3,6-dihydro-2H-[1,4]oxazin-3-yl)-4-fluoro-phenyl]-amide;
5-Trideuteromethoxy-3-trideuteromethoxymethyl-pyridine-2-carboxylic acid [3-(5-amino-3-difluoromethyl-3,6-dihydro-2H-[1,4]oxazin-3-yl)-4-fluoro-phenyl]-amide; 3-Amino-5-cyano-pyridine-2-carboxylic acid [3-(5-amino-3-difluoromethyl-3,6-dihydro-2H-[1,4]oxazin-3-yl)-4-fluoro-phenyl]-amide;
3-Amino-5-difluoromethyl-pyridine-2-carboxylic acid [3-(5-amino-3-difluoromethyl-3,6-dihydro-2H-[1,4]oxazin-3-yl)-4-fluoro-phenyl]-amide;
5-Trideuteromethoxy-3-methyl-pyridine-2-carboxylic acid [3-(5-amino-3-difluoromethyl-3,6-dihydro-2H-[1,4]oxazin-3-yl)-4-fluoro-phenyl]-amide;
5-Cyano-3-methyl-pyridine-2-carboxylic acid [3-(5-amino-3-difluoromethyl-3,6-dihydro-2H-[1,4]oxazin-3-yl)-4,5-difluoro-phenyl]-amide;
3-Chloro-5-trideuteromethoxy-pyridine-2-carboxylic acid [3-(5-amino-3-difluoromethyl-3,6-dihydro-2H-[1,4]oxazin-3-yl)-4,5-difluoro-phenyl]-amide;
4,6-Dideutero-5-chloro-3-trideuteromethyl-pyridine-2-carboxylic acid [3-(5-amino-3-difluoromethyl-3,6-dihydro-2H-[1,4]oxazin-3-yl)-4,5-difluoro-phenyl]-amide;
3-Chloro-5-cyano-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-methoxy-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-difluoromethoxy-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
5-Chloro-3-methyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
5-Chloro-3-fluoro-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-trideutero-methoxy-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
2,5-Dimethyl-oxazole-4-carboxylic acid [3-(5-amino-3,6-dimethyl-6-trifluoromethyl-3,6-dihydro-2H-[1,4]oxazin-3-yl)-4-fluoro-phenyl]-amide;
5-Difluoromethoxy-3-methyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Amino-5-prop-2-ynyloxy-pyrazine-2-carboxylic acid [3-(5-amino-3,6-dimethyl-6-trifluoromethyl-3,6-dihydro-2H-[1,4]oxazin-3-yl)-4-fluoro-phenyl]-amide;
3-Amino-5-cyano-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
5-Difluoromethyl-3-methyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Amino-5-difluoromethyl-pyrazine-2-carboxylic acid [3-(5-amino-3,6-dimethyl-6-trifluoromethyl-3,6-dihydro-2H-[1,4]oxazin-3-yl)-4-fluoro-phenyl]-amide;
3-Amino-5-difluoromethyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
4-Difluoromethyl-6-methoxy-pyridazine-3-carboxylic acid [3-(5-amino-3,6-dimethyl-6-trifluoromethyl-3,6-dihydro-2H-[1,4]oxazin-3-yl)-4-fluoro-phenyl]-amide;
5-Cyano-3-trideuteromethoxymethyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-difluormethyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-trideuteromethoxy-dideuteromethyl-1 H-pyrrolo[2,3-b]pyridine-6-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-trifluoromethyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
5-Fluoro-3-methyl-pyridine-2-carboxylic acid [3-(5-amino-3,6-dimethyl-6-trifluoro-methyl-3,6-dihydro-2H-[1,4]oxazin-3-yl)-4-fluoro-phenyl]-amide;
5-Trideuteromethoxy-3-methyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
5-Cyano-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
5-Difluoromethoxy-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
5-Chloro-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
5-Cyano-3-methyl-pyridine-2-carboxylic acid [3-(5-amino-3,6,6-tris-fluoromethyl-3,6-dihydro-2H-[1,4]oxazin-3-yl)-4-fluoro-phenyl]-amide;
5-Difluoromethyl-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
3-Amino-5-difluoromethyl-pyrazine-2-carboxylic acid [3-(5-amino-6,6-bis-fluoromethyl-3-methyl-3,6-dihydro-2H-[1,4]oxazin-3-yl)-4-fluoro-phenyl]-amide;
3-Amino-5-difluoromethyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
3-Amino-5-cyano-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
5-Difluoromethyl-3-methyl-pyridine-2-carboxylic acid [3-(5-amino-3,6,6-tris-fluoromethyl-3,6-dihydro-2H-[1,4]oxazin-3-yl)-4-fluoro-phenyl]-amide;
3-Chloro-5-difluoromethyl-pyridine-2-carboxylic acid [3-(5-amino-3,6,6-tris-fluoromethyl-3,6-dihydro-2H-[1,4]oxazin-3-yl)-4-fluoro-phenyl]-amide;
3-Chloro-5-trifluoromethyl-pyridine-2-carboxylic acid [3-(5-amino-3,6,6-tris-fluoromethyl-3,6-dihydro-2H-[1,4]oxazin-3-yl)-4-fluoro-phenyl]-amide;
3,5-Dichloro-pyridine-2-carboxylic acid [3-(5-amino-3,6,6-tris-fluoromethyl-3,6-dihydro-2H-[1,4]oxazin-3-yl)-4-fluoro-phenyl]-amide;
3-Amino-5-cyano-pyridine-2-carboxylic acid [3-(5-amino-3,6,6-tris-fluoromethyl-3,6-dihydro-2H-[1,4]oxazin-3-yl)-4-fluoro-phenyl]-amide;
3-Chloro-5-cyano-pyridine-2-carboxylic acid [3-(5-amino-3,6,6-tris-fluoromethyl-3,6-dihydro-2H-[1,4]oxazin-3-yl)-4-fluoro-phenyl]-amide;
N-[3-(5-Amino-3,6-dimethyl-6-trifluoromethyl-3,6-dihydro-2H-[1,4]oxazin-3-yl)-4-fluoro-phenyl]-6-methoxy-2-methyl-nicotinamide;
N-[3-(5-Amino-3,6-dimethyl-6-trifluoromethyl-3,6-dihydro-2H-[1,4]oxazin-3-yl)-4-fluorophenyl]-6-trideuteromethoxy-2-methyl-nicotinamide;
2-Amino-N-[3-(5-amino-3,6-dimethyl-6-trifluoromethyl-3,6-dihydro-2H-[1,4]oxazin-3-yl)-4-fluoro-phenyl]-6-ethoxy-nicotinamide;
2-Amino-N-[3-(5-amino-3,6-dimethyl-6-trifluoromethyl-3,6-dihydro-2H-[1,4]oxazin-3-yl)-4-fluoro-phenyl]-6-methoxy-nicotinamide;
2-Amino-N-[3-(5-amino-3,6-dimethyl-6-trifluoromethyl-3,6-dihydro-2H-[1,4]oxazin-3-yl)-4-fluoro-phenyl]-6-trideuteromethoxy-nicotinamide;
2-Amino-N-[3-(5-amino-3,6-dimethyl-6-trifluoromethyl-3,6-dihydro-2H-[1,4]oxazin-3-yl)-4-fluoro-phenyl]-6-pentadeuteroethoxy-nicotinamide;
N-[3-(5-Amino-3,6-dimethyl-6-trifluoromethyl-3,6-dihydro-2H-[1,4]oxazin-3-yl)-4-fluoro-phenyl]-2-chloro-6-methoxy-nicotinamide;
N-[3-(5-Amino-3,6-dimethyl-6-trifluoromethyl-3,6-dihydro-2H-[1,4]oxazin-3-yl)-4-fluoro-phenyl]-2-chloro-6-ethoxy-nicotinamide;
2-Amino-N-[3-(5-amino-3,6-dimethyl-6-trifluoromethyl-3,6-dihydro-2H-[1,4]oxazin-3-yl)-4-fluoro-phenyl]-6-cyclopropylmethoxy-nicotinamide; and
2-Amino-N-[3-(5-amino-3,6-dimethyl-6-trifluoromethyl-3,6-dihydro-2H-[1,4]oxazin-3-yl)-4-fluoro-phenyl]-6-(2,2,2-trifluoro-ethoxy)-nicotinamide;
and pharmaceutically acceptable salts thereof.

The terms "a compound of the invention", "a compound of the formula I" and "agents of the invention" are used interchangeably throughout the description and are intended to mean the same thing, namely any compound, or a pharmaceutically acceptable salt thereof, falling within the definition of the first aspect of the invention described hereinbefore.

On account of one or more than one asymmetrical carbon atom, which may be present in a compound of the formula I, a corresponding compound of the formula I may exist in pure optically active form or in the form of a mixture of optical isomers, for example in the form of a racemic mixture. All of such pure optical isomers and all of their mixtures, including the racemic mixtures, are part of the present invention as defined in the first aspect of the invention described hereinbefore.

In one embodiment, there is provided a compound of the invention as an isolated stereoisomer wherein the compound has one stereocenter and the stereoisomer is in the R configuration:

In one embodiment, there is provided a compound of the invention as an isolated stereoisomer wherein the compound has one stereocenter and the stereoisomer is in the S configuration.

In one embodiment, there is provided a compound of the invention as an isolated stereoisomer wherein the compound has two stereocenters and the stereoisomer is in the R R configuration.

In one embodiment, there is provided a compound of the invention as an isolated stereoisomer wherein the compound has two stereocenters and the stereoisomer is in the R S configuration.

In one embodiment, there is provided a compound of the invention as an isolated stereoisomer wherein the compound has two stereocenters and the stereoisomer is in the S R configuration.

In one embodiment, there is provided a compound of the invention as an isolated stereoisomer wherein the compound has two stereocenters and the stereoisomer is in the S S configuration.

In one embodiment, there is provided a compound of the invention, wherein the compound has one or two stereocenters, as a racemic mixture.

As used herein, the term "isomers" refers to different compounds that have the same molecular formula but differ in arrangement and configuration of the atoms. Also as used herein, the term "an optical isomer" or "a stereoisomer" refers to any of the various stereo isomeric configurations which may exist for a given compound of the present invention and includes geometric isomers. It is understood that a substituent may be attached at a chiral center of a carbon atom. Therefore, the invention includes enantiomers, diastereomers or racemates of the compound. "Enantiomers" are a pair of stereoisomers that are non-superimposable mirror images of each other. A 1:1 mixture of a pair of enantiomers is a "racemic" mixture. The term is used to designate a racemic mixture where appropriate. "Diastereoisomers" are stereoisomers that have at least two asymmetric atoms, but which are not mirror-images of each other. The absolute stereochemistry is specified according to the Cahn- Ingold- Prelog R-S system. When a compound is a pure enantiomer the stereochemistry at each chiral carbon may be specified by either R or S. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers or axes and may thus give rise to enantiomers, diastereomers, and other stereoisomeric forms that may be defined, in terms of absolute stereochemistry, as (R)-or (S)-. The present invention is meant to include all such possible isomers, including racemic mixtures, optically pure forms and intermediate mixtures. Optically active (R)- and (S)- isomers may be prepared using chiral synthons or chiral reagents, or resolved using conventional techniques. If the compound contains a double bond, the substituent may be E or Z configuration. If the compound contains a disubstituted cycloalkyl, the cycloalkyl substituent may have a cis- or trans-configuration.

A compound of the formula I may exist in tautomeric form. All such tautomers are part of the present invention.

In one embodiment of the invention, the invention relates to compounds of the formula (I) 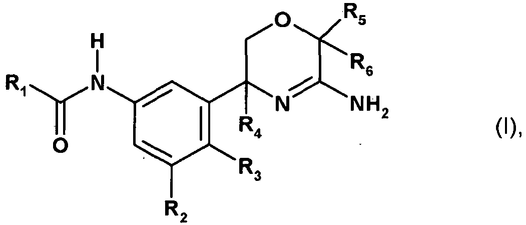
wherein R₁, R₂, R₃, R₄, R₅ and R₆ are defined so as to provide a compound selected from: 5-Chloro-3-methoxymethyl-pyridine-2-carboxylic acid [3-((R)-5-amino-3-difluoromethyl-3,6-dihydro-2H-[1,4] oxazin-3-yl)-4-fluoro-phenyl]-amide;
3-Chloro-5-cyano-pyridine-2-carboxylic acid [3-((R)-5-amino-3-difluoromethyl-3,6-dihydro-2H-[1,4]oxazin-3-yl)-4-fluoro-phenyl]-amide;
3-Amino-5-tris-deutero-methoxy-pyrazine-2-carboxylic acid [3-((R)-5-amino-3-difluoromethyl-3,6-dihydro-2H-[1,4]oxazin-3-yl)-4-fluoro-phenyl]-amide;
3-Amino-5-prop-2-ynyloxy-pyrazine-2-carboxylic acid [3-((R)-5-amino-3-difluoromethyl-3,6-dihydro-2H-[1,4]oxazin-3-yl)-4-fluoro-phenyl]-amide;
3-Chloro-1H-pyrrolo[2,3-b]pyridine-6-carboxylic acid [3-((R)-5-amino-3-difluoromethyl-3,6-dihydro-2H-[1,4]oxazin-3-yl)-4-fluoro-phenyl]-amide;
3-Amino-5-difluoromethyl-pyrazine-2-carboxylic acid [3-((R)-5-amino-3-difluoromethyl-3,6-dihydro-2H-[1,4]oxazin-3-yl)-4-fluoro-phenyl]-amide;
5-Methoxy-3-methyl-pyridine-2-carboxylic acid [3-((R)-5-amino-3-difluoromethyl-3,6-dihydro-2H-[1,4]oxazin-3-yl)-4-fluoro-phenyl]-amide;
5-Difluoromethyl-3-methyl-pyridine-2-carboxylic acid [3-((R)-5-amino-3-difluoromethyl-3,6-dihydro-2H-[1,4]oxazin-3-yl)-4-fluoro-phenyl]-amide;
5-Fluoro-3-trideuteromethoxymethyl-pyridine-2-carboxylic acid [3-((R)-5-amino-3-difluoromethyl-3,6-dihydro-2H-[1,4]oxazin-3-yl)-4-fluoro-phenyl]-amide;
5-Trideuteromethoxy-3-trideuteromethoxymethyl-pyridine-2-carboxylic acid [3-((R)-5-amino-3-difluoromethyl-3,6-dihydro-2H-[1,4]oxazin-3-yl)-4-fluoro-phenyl]-amide;
3-Amino-5-cyano-pyridine-2-carboxylic acid [3-((R)-5-amino-3-difluoromethyl-3,6-dihydro-2H-[1,4]oxazin-3-yl)-4-fluoro-phenyl]-amide;
3-Amino-5-difluoromethyl-pyridine-2-carboxylic acid [3-((R)-5-amino-3-difluoromethyl-3,6-dihydro-2H-[1,4]oxazin-3-yl)-4-fluoro-phenyl]-amide;
5-Trideuteromethoxy-3-methyl-pyridine-2-carboxylic acid [3-((R)-5-amino-3-difluoromethyl-3,6-dihydro-2H-[1,4]oxazin-3-yl)-4-fluoro-phenyl]-amide;
5-Cyano-3-methyl-pyridine-2-carboxylic acid [3-((R)-5-amino-3-difluoromethyl-3,6-dihydro-2H-[1,4]oxazin-3-yl)-4,5-difluoro-phenyl]-amide;
3-Chloro-5-trideuteromethoxy-pyridine-2-carboxylic acid [3-((R)-5-amino-3-difluoromethyl-3,6-dihydro-2H-[1,4]oxazin-3-yl)-4,5-difluoro-phenyl]-amide;
4,6-Dideutero-5-chloro-3-trideuteromethyl-pyridine-2-carboxylic acid [3-((R)-5-amino-3-difluoromethyl-3,6-dihydro-2H-[1,4]oxazin-3-yl)-4,5-difluoro-phenyl]-amide;
3-Chloro-5-cyano-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Chloro-5-methoxy-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Chloro-5-difluoromethoxy-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Chloro-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Chloro-3-fluoro-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Chloro-5-trideutero-methoxy-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
2,5-Dimethyl-oxazole-4-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Difluoromethoxy-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Amino-5-prop-2-ynyloxy-pyrazine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Amino-5-cyano-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Difluoromethyl-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Amino-5-difluoromethyl-pyrazine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Amino-5-difluoromethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
4-Difluoromethyl-6-methoxy-pyridazine-3-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Cyano-3-trideuteromethoxymethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Chloro-5-difluormethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Chloro-5-trideuteromethoxy-dideuteromethyl-1H-pyrrolo[2,3-b]pyridine-6-carboxylic acid [3-((3R,6R)-5-amino-3,6-dimethyl-6-trifluoromethyl-3,6-dihydro-2H-[1,4]oxazin-3-yl)-4-fluorophenyl]-amide;
3-Chloro-5-trifluoromethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Fluoro-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Trideuteromethoxy-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Cyano-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
5-Cyano-3-methyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide;
5-Difluoromethoxy-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
5-Difluoromethoxy-3-methyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide;
5-Chloro-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
5-Cyano-3-methyl-pyridine-2-carboxylic acid [3-((R)-5-amino-3,6,6-tris-fluoromethyl-3,6-dihydro-2H-[1,4]oxazin-3-yl)-4-fluoro-phenyl]-amide;
5-Difluoromethyl-3-methyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide;
3-Amino-5-difluoromethyl-pyrazine-2-carboxylic acid [3-((R)-5-amino-6,6-bis-fluoromethyl-3-methyl-3,6-dihydro-2H-[1,4]oxazin-3-yl)-4-fluoro-phenyl]-amide;
3-Amino-5-difluoromethyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide;
3-Amino-5-cyano-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide;
5-Difluoromethyl-3-methyl-pyridine-2-carboxylic acid [3-((R)-5-amino-3,6,6-tris-fluoromethyl-3,6-dihydro-2H-[1,4]oxazin-3-yl)-4-fluoro-phenyl]-amide;
3-Chloro-5-difluoromethyl-pyridine-2-carboxylic acid [3-((R)-5-amino-3,6,6-tris-fluoromethyl-3,6-dihydro-2H-[1,4]oxazin-3-yl)-4-fluoro-phenyl]-amide;
3-Chloro-5-trifluoromethyl-pyridine-2-carboxylic acid [3-((R)-5-amino-3,6,6-tris-fluoromethyl-3,6-dihydro-2H-[1,4]oxazin-3-yl)-4-fluoro-phenyl]-amide;
3,5-Dichloro-pyridine-2-carboxylic acid [3-((R)-5-amino-3,6,6-tris-fluoromethyl-3,6-dihydro-2H-[1,4]oxazin-3-yl)-4-fluoro-phenyl]-amide;
3-Amino-5-cyano-pyridine-2-carboxylic acid [3-((R)-5-amino-3,6,6-tris-fluoromethyl-3,6-dihydro-2H-[1,4]oxazin-3-yl)-4-fluoro-phenyl]-amide;
3-Chloro-5-cyano-pyridine-2-carboxylic acid [3-((R)-5-amino-3,6,6-tris-fluoromethyl-3,6-dihydro-2H-[1,4]oxazin-3-yl)-4-fluoro-phenyl]-amide;
N-[3-((3R,6R)-5-Amino-3,6-dimethyl-6-trifluoromethyl-3,6-dihydro-2H-[1,4]oxazin-3-yl)-4-fluoro-phenyl]-6-methoxy-2-methyl-nicotinamide;
N-[3-((3R,6R)-5-Amino-3,6-dimethyl-6-trifluoromethyl-3,6-dihydro-2H-[1,4]oxazin-3-yl)-4-fluoro-phenyl]-6-trideuteromethoxy-2-methyl-nicotinamide;
2-Amino-N-[3-((3R,6R)-5-amino-3,6-dimethyl-6-trifluoromethyl-3,6-dihydro-2H-[1,4]oxazin-3-yl)-4-fluoro-phenyl]-6-ethoxy-nicotinamide;
2-Amino-N-[3-((3R,6R)-5-amino-3,6-dimethyl-6-trifluoromethyl-3,6-dihydro-2H-[1,4]oxazin-3-yl)-4-fluoro-phenyl]-6-methoxy-nicotinamide;
2-Amino-N-[3-((3R,6R)-5-amino-3,6-dimethyl-6-trifluoromethyl-3,6-dihydro-2H-[1,4]oxazin-3-yl)-4-fluoro-phenyl]-6-trideuteromethoxy-nicotinamide;
2-Amino-N-[3-((3R,6R)-5-amino-3,6-dimethyl-6-trifluoromethyl-3,6-dihydro-2H-[1,4]oxazin-3-yl)-4-fluoro-phenyl]-6-pentadeuteroethoxy-nicotinamide;
N-[3-((3R,6R)-5-Amino-3,6-dimethyl-6-trifluoromethyl-3,6-dihydro-2H-[1,4]oxazin-3-yl)-4-fluoro-phenyl]-2-chloro-6-methoxy-nicotinamide;
N-[3-((3R,6R)-5-Amino-3,6-dimethyl-6-trifluoromethyl-3,6-dihydro-2H-[1,4]oxazin-3-yl)-4-fluoro-phenyl]-2-chloro-6-ethoxy-nicotinamide;
2-Amino-N-[3-((3R,6R)-5-amino-3,6-dimethyl-6-trifluoromethyl-3,6-dihydro-2H-[1,4]oxazin-3-yl)-4-fluoro-phenyl]-6-cyclopropylmethoxy-nicotinamide; and
2-Amino-N-[3-((3R,6R)-5-amino-3,6-dimethyl-6-trifluoromethyl-3,6-dihydro-2H-[1,4]oxazin-3-yl)-4-fluoro-phenyl]-6-(2,2,2-trifluoro-ethoxy)-nicotinamide;
and pharmaceutically acceptable salts thereof.

A compound of the formula I may exist in free form or in pharmaceutically acceptable salt form. All of such free compounds and pharmaceutically acceptable salts are part of the present invention.

Salts may be prepared from free compounds in a known manner, and vice-versa.

In one embodiment, the invention relates to any one of the compounds of the invention in free form. In another embodiment, the invention relates to any one of the compounds of the invention in pharmaceutically acceptable salt form. In a further embodiment, the invention relates to any one of the compounds of the invention in pharmaceutically acceptable acid addition salt form. In yet a further embodiment, the invention relates to any one of the compounds of the invention in hydrochloride salt form.

As used herein, the terms "salt" or "salts" refers to an acid addition salt of a compound of the invention. "Salts" include in particular "pharmaceutically acceptable salts". The term "pharmaceutically acceptable salts" refers to salts that retain the biological effectiveness and properties of the compounds of this invention and, which typically are not biologically or otherwise undesirable. In many cases, the compounds of the present invention are capable of forming acid salts by virtue of the presence of amino groups or groups similar thereto.

Pharmaceutically acceptable acid addition salts may be formed with inorganic acids and organic acids, e.g., acetate, aspartate, benzoate, besylate, bromide/hydrobromide, bicarbonate/carbonate, bisulfate/sulfate, camphorsulfornate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts. Inorganic acids from which salts can be derived include, for example, hydrochloric acid, hydrobromic acid, sulfuric acid, nitric acid and phosphoric acid. Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid and sulfosalicylic acid.

The pharmaceutically acceptable salts of the present invention can be synthesized from a parent compound by conventional chemical methods. Generally, such salts can be prepared by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

Furthermore, the compounds of the present invention, including their salts, may also be obtained in the form of their hydrates, or include other solvents used for their crystallization. The compounds of the present invention may inherently or by design form solvates with pharmaceutically acceptable solvents (including water); therefore, it is intended that the invention embrace both solvated and unsolvated forms. The term "solvate" refers to a molecular complex of a compound of the present invention (including pharmaceutically acceptable salts thereof) with one or more solvent molecules. Such solvent molecules are those commonly used in the pharmaceutical art, which are known to be innocuous to the recipient, e.g., water, ethanol, and the like. The term "hydrate" refers to the complex where the solvent molecule is water.

The compounds of the present invention, including salts, hydrates and solvates thereof, may inherently or by design form polymorphs. All such polymorphs are part of the present invention.

The present invention includes all pharmaceutically acceptable isotope-labeled compounds of the formula I, wherein one or more than one atom is / are replaced by one or more than one atom having the same atomic number as, but an atomic mass different from, the one(s) usually found in nature. Examples of such isotopes are those of carbon, such as ¹¹C, ¹³C or ¹⁴C, chlorine, such as ³⁸Cl, fluorine, such as ¹⁸F, bromine, such as ⁷⁶Br, hydrogen, such as ²H or ³H, iodine, such as ¹²³I,¹²⁴I, ¹²⁵I or ¹³¹I, nitrogen, such as ¹³N or ¹⁵N, oxygen, such as ¹⁵O, ¹⁷O or ¹⁸O, phosphorus, such as ³²P, or sulphur, such as ³⁵S. An isotope-labeled compound of the formula I can be prepared by a process analogous to those described in the Examples or by a conventional technique known to those skilled in the art using an appropriate isotopically-labeled reagent or starting material. The incorporation of a heavier isotope, such as ²H, may provide greater metabolic stability to a compound of the formula I, which may result in, for example, an increased *in vivo*-half-life of the compound or in reduced dosage requirements. Certain isotope-labeled compounds of the formula I, for example those incorporating a radioactive isotope, such as ³H or ¹⁴C, may be used in drug or substrate-tissue distribution studies. Compounds of the formula I with a positron emitting isotope, such as ¹¹C, ¹⁸F, ¹³N or ¹⁵O, may be useful in positron emission tomography (PET) or single photon emission computed tomography (SPECT) studies, e. g. to examine substrate-receptor occupancies.

Pharmaceutically acceptable solvates in accordance with the invention include those wherein the solvent of crystallization may be isotopically substituted, e.g. D2O, d6-acetone, d6-DMSO.

Compounds of the invention that contain groups capable of acting as donors and/or acceptors for hydrogen bonds may be capable of forming co-crystals with suitable co-crystal formers. These co-crystals may be prepared from compounds of formula I by known co-crystal forming procedures. Such procedures include grinding, heating, co-subliming, comelting, or contacting in solution compounds of formula I with the co-crystal former under crystallization conditions and isolating co-crystals thereby formed. Suitable co-crystal formers include those described in WO 2004/078163. Hence the invention further provides co-crystals comprising a compound of formula I.

Compounds of the formula I can also be prepared by further conventional processes, which processes are further aspects of the invention, for example as described in the Examples. The starting materials are known, may be prepared according to conventional procedures starting from known compounds, may be prepared from known compounds as described in the Examples, or may be prepared using procedures analogous to those described in the Examples.

Compounds of the formula I, in free form, or in pharmaceutically acceptable salt form, hereinafter often referred to as "agents of the invention", exhibit valuable pharmacological properties, when tested *in vitro* or *in vivo,* and are, therefore, useful in medicaments, in therapy or for use as research chemicals, for example as tool compounds.

For example, agents of the invention are inhibitors of aspartic proteases and can be used for the treatment or prevention of a condition, disease or disorder involving processing by such enzymes. Particularly, agents of the invention inhibit beta-secretase and, thus, the generation of beta-amyloid and the subsequent aggregation into oligomers and fibrils.

The inhibiting properties of an agent of the invention towards proteases can be evaluated in tests as described hereinafter.

### Test 1: Inhibition of human BACE-1

Recombinant BACE-1 (extracellular domain, expressed in baculovirus and purified using standard methods) at 0.1 to 10 nM concentrations is incubated with the test compound at various concentrations for 1 hour at room temperature in 10 to 100 mM acetate buffer, pH 4.5, containing 0.1 % CHAPS. Synthetic fluorescence-quenched peptide substrate, derived from the sequence of APP and containing a suitable fluorophore-quencher pair, is added to a final concentration of 1 to 5 µM, and the increase in fluorescence is recorded at a suitable excitation / emission wavelength in a microplate spectro-fluorimeter for 5 to 30 minutes in 1-minute intervals. IC₅₀ values are calculated from percentage of inhibition of BACE-1 activity as a function of the test compound concentration.

### Test 2: Inhibition of human BACE-2

Recombinant BACE-2 (extracellular domain, expressed in baculovirus and purified using standard methods) at 0.1 to 10 nM concentrations is incubated with the test compound at various concentrations for 1 hour at room temperature in 10 to 100 mM acetate buffer, pH 4.5, containing 0.1 % CHAPS. Synthetic fluorescence-quenched peptide substrate, derived from the sequence of APP and containing a suitable fluorophore-quencher pair, is added to a final concentration of 1 to 5 µM, and the increase in fluorescence is recorded at a suitable excitation / emission wavelength in a microplate spectro-fluorimeter for 5 to 30 minutes in 1-minute intervals. IC₅₀ values are calculated from percentage of inhibition of BACE-2 activity as a function of the test compound concentration.

### Test 3: Inhibition of human cathepsin D

Recombinant cathepsin D (expressed as procathepsin D in baculovirus, purified using standard methods and activated by incubation in sodium formate buffer pH 3.7) is incubated with the test compound at various concentrations for 1 hour at room temperature in sodium formate or sodium acetate buffer at a suitable pH within the range of pH 3.0 to 5.0. Synthetic peptide substrate Mca-Gly-Lys-Pro-Ile-Leu-Phe-Phe-Arg-Leu-Lys(DNP)-D-Arg-NH₂ is added to a final concentration of 1 to 5 µM, and the increase in fluorescence is recorded at excitation of 325 nm and emission at 400 nm in a microplate spectro-fluorimeter for 5 to 30 minutes in 1-minute intervals. IC₅₀ values are calculated from the percentage of inhibition of cathepsin D-activity as a function of the test compound concentration.

### Test 4: Inhibition of cellular release of amyloid peptide 1-40

Chinese hamster ovary cells are transfected with the human gene for amyloid precursor protein. The cells are plated at a density of 8000 cells/well into 96-well microtiter plates and cultivated for 24 hours in DMEM cell culture medium containing 10 % FCS. The test compound is added to the cells at various concentrations, and the cells are cultivated for 24 hours in the presence of the test compound. The supernatants are collected, and the concentration of amyloid peptide 1-40 is determined using state of the art immunoassay techniques, for example sandwich ELISA, homogenous time-resolved fluorescence (HTRF) immunoassay, or electro-chemiluminescence immunoassay. The potency of the compound is calculated from the percentage of inhibition of amyloid peptide release as a function of the test compound concentration.

Agents of the invention were tested in at least one of the above-described tests.

The compounds of the Examples show the following mean IC₅₀ values in Test 1 described hereinbefore:

**Table A**

| **Example** | **Bace IC₅₀ [µM]** | **Example** | **Bace IC₅₀ [µM]** |
|---|---|---|---|
| 1 | 0.018 | 2 | 0.032 |
| 3 | 0.025 | 4 | 0.002 |
| 5 | 0.012 | 6 | 0.025 |
| 7 | 0.13 | 8 | 0.054 |
| 9 | 0.11 | 10 | 0.19 |
| 11 | 0.018 | 12 | 0.038 |
| 13 | 0.11 | 14 | 0.02 |
| 15 | 0.032 | 16 | 0.014 |
| 17 | 0.009 | 18 | 0.02 |
| 19 | 0.016 | 20 | 0.005 |
| 21 | 0.012 | 22 | 0.02 |
| 23 | 0.067 | 24 | 0.014 |
| 25 | 0.001 | 26 | 0.005 |
| 27 | 0.016 | 28 | 0.01 |
| 29 | 0.014 | 30 | 0.15 |
| 31 | 0.011 | 32 | 0.02 |
| 33 | 0.013 | 34 | 0.016 |
| 35 | 0.027 | 36 | 0.028 |
| 37 | 0.032 | 38 | 0.024 |
| 39 | 0.3 | 40 | 0.029 |
| 41 | 0.048 | 42 | 0.012 |
| 43 | 0.024 | 44 | 0.22 |
| 45 | 2.6 | | |

The compounds of the Examples show the following mean IC₅₀ values in Test 4 described hereinbefore:

**Table B**

| **Example** | **Amyloid-β1-40 release IC₅₀ [µM]** | **Example** | **Amyloid-β1-40 release IC₅₀ [µM]** |
|---|---|---|---|
| 1 | 0.006 | 2 | 0.011 |
| 3 | 0.009 | 4 | 0.001 |
| 5 | 0.014 | 6 | 0.008 |
| 7 | 0.011 | 8 | 0.008 |
| 9 | 0.036 | 10 | 0.032 |
| 11 | 0.005 | 12 | 0.01 |
| 13 | 0.022 | 14 | 0.009 |
| 15 | 0.021 | 16 | 0.014 |
| 17 | 0.004 | 18 | 0.009 |
| 19 | 0.009 | 20 | 0.026 |
| 21 | 0.009 | 22 | 0.007 |
| 23 | 0.16 | 24 | 0.006 |
| 25 | 0.001 | 26 | 0.004 |
| 27 | 0.01 | 28 | 0.008 |
| 29 | 0.01 | 30 | 0.062 |
| 31 | 0.009 | 32 | 0.011 |
| 33 | 0.041 | 34 | 0.01 |
| 35 | 0.088 | 36 | 0.018 |
| 37 | 0.004 | 38 | 0.003 |
| 39 | 0.022 | 40 | 0.004 |
| 41 | 0.013 | 42 | 0.013 |
| 43 | 0.022 | 44 | 0.079 |
| 45 | 0.72 | | |

As used herein, the term "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.

The term "a therapeutically effective amount" of a compound of the present invention refers to an amount of the compound of the present invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the compound of the present invention that, when administered to a subject, is effective to (1) at least partially alleviating, inhibiting, preventing and/or ameliorating a condition, or a disorder or a disease (i) mediated by BACE-1 or (ii) associated with BACE-1 activity, or (iii) characterized by activity (normal or abnormal) of BACE-1; or (2) reducing or inhibiting the activity of BACE-1. In another non-limiting embodiment, the term "a therapeutically effective amount" refers to the amount of the compound of the present invention that, when administered to a cell, or a tissue, or a non-cellular biological material, or a medium, is effective to at least partially reduce or inhibit the activity of BACE-1. The meaning of the term "a therapeutically effective amount" as illustrated in the above embodiments for BACE-1 also applies by the same means to any other relevant proteins/peptides/enzymes, such as BACE-2, or cathepsin D.

As used herein, the term "subject" refers to an animal. Typically the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e.g*., stabilization of a discernible symptom), physiologically, (*e.g*., stabilization of a physical parameter), or both.

As used herein, the term "prevention" of any particular disease or disorder refers to the administration of a compound of the present invention to a subject before any symptoms of that disease or disorder are apparent.

As used herein, a subject is "in need of" a treatment if such subject would benefit biologically, medically or in quality of life from such treatment.

As used herein, the term an "agent" of the invention is used interchangeably with the term a "compound" of the invention and has no difference in meaning therefrom.

As used herein, the term "a," "an," "the" and similar terms used in the context of the present invention (especially in the context of the claims) are to be construed to cover both the singular and plural unless otherwise indicated herein or clearly contradicted by the context. The use of any and all examples, or exemplary language (*e.g*. "such as") provided herein is intended merely to better illuminate the invention and does not pose a limitation on the scope of the invention otherwise claimed.

Due to their inhibiting properties towards proteases, agents of the invention are useful, e. g., in the treatment or prevention of a variety of disabilitating psychiatric, psychotic, neurological or vascular states, e. g. of a condition, disease or disorder of the vascular system or of the nervous system, in which beta-amyloid generation or aggregation plays a role, or, based on the inhibition of BACE-2 (beta-site APP-cleaving enzyme 2) or cathepsin D, which are close homologues of the pepsin-type aspartyl proteases and beta-secretase, and the correlation of the BACE-2 or cathepsin D expression with a more tumorigenic or metastatic potential of tumor cells, as anti-cancer medicaments, e. g. in the suppression of the metastasis process associated with tumor cells. The said condition, disease or disorder of the vascular system or of the nervous system is exemplified by, and includes, without limitation, an anxiety disorder, such as panic disorder with or without agoraphobia, agoraphobia without history of panic disorder, an animal or other specific phobia, including a social phobia, social anxiety disorder, anxiety, obsessive-compulsive disorder, a stress disorder, including post-traumatic or acute stress disorder, or a generalized or substance-induced anxiety disorder; a neurosis; seizures; epilepsy, especially partial seizures, simple, complex or partial seizures evolving to secondarily generalized seizures or generalized seizures [absence (typical or atypical), myoclonic, clonic, tonic, tonic-clonic or atonic seizures]; convulsions; migraine; an affective disorder, including a depressive or bipolar disorder, e. g. single-episode or recurrent major depressive disorder, major depression, a dysthymic disorder, dysthymia, depressive disorder NOS, bipolar I or bipolar II manic disorder or cyclothymic disorder; a psychotic disorder, including schizophrenia or depression; neurodegeneration, e. g. neurodegeneration arising from cerebral ischemia; an acute, traumatic or chronic degenerative process of the nervous system, such as Parkinson's disease, Down's syndrome, dementia, e. g. senile dementia, dementia with Lewy bodies or a fronto-temporal dementia, a cognitive disorder, cognitive impairment, e. g. mild cognitive impairment, memory impairment, an amyloid neuropathy, a peripheral neuropathy, Alzheimer's disease, Gerstmann-Straeussler-Scheinker syndrome, Niemann-Pick disease, e. g. Niemann-Pick type C disease, brain inflammation, a brain, spinal cord or nerve injury, e. g. traumatic brain injury (TBI), a nerve trauma or a brain trauma, vascular amyloidosis, cerebral haemorrhage with amyloidosis, Huntington's chorea, amyotrophic lateral sclerosis, multiple sclerosis or fragile X syndrome; scrapie; cerebral amyloid angiopathy; an encephalopathy, e. g. transmissible spongiform encephalopathy; stroke; an attention disorder, e. g. attention deficit hyperactivity disorder; Tourette's syndrome; a speech disorder, including stuttering; a disorder of the circadian rhythm, e. g. in subjects suffering from the effects of jet lag or shift work; pain; nociception; itch; emesis, including acute, delayed or anticipatory emesis, such as emesis induced by chemotherapy or radiation, motion sickness, or post-operative nausea or vomiting; an eating disorder, including anorexia nervosa or bulimia nervosa; premenstrual syndrome; a muscle spasm or spasticity, e. g. in paraplegic patients; a hearing disorder, e. g. tinnitus or age-related hearing impairment; urinary incontinence; glaucoma; inclusion-body myositis; or a substance-related disorder, including substance abuse or dependency, including a substance, such as alcohol, withdrawal disorder. Agents of the invention may also be useful in enhancing cognition, e. g. in a subject suffering from a dementing condition, such as Alzheimer's disease; as premedication prior to anaesthesia or a minor medical intervention, such as endoscopy, including gastric endoscopy; or as ligands, e. g. radioligands or positron emission tomography (PET) ligands.

For the above-mentioned indications, the appropriate dosage will vary depending on, e. g., the compound employed as active pharmaceutical ingredient, the host, the mode of administration, the nature and severity of the condition, disease or disorder or the effect desired. However, in general, satisfactory results in animals are indicated to be obtained at a daily dosage of from about 0.1 to about 100, preferably from about 1 to about 50, mg/kg of animal body weight. In larger mammals, for example humans, an indicated daily dosage is in the range of from about 0.5 to about 2000, preferably from about 2 to about 200, mg of an agent of the invention conveniently administered, for example, in divided doses up to four times a day or in sustained release form.

An agent of the invention may be administered by any conventional route, in particular enterally, preferably orally, e. g. in the form of a tablet or capsule, or parenterally, e. g. in the form of an injectable solution or suspension.

In a further aspect, the invention relates to a pharmaceutical composition comprising an agent of the invention as active ingredient in association with at least one pharmaceutically acceptable carrier or diluent and optionally in association with other auxiliary substances, such as inhibitors of cytochrome P450 enzymes, agents preventing the degradation of active pharmaceutical ingredients by cytochrome P450, agents improving or enhancing the pharmacokinetics of active pharmaceutical ingredients, agents improving or enhancing the bioavailability of active pharmaceutical ingredients, and so on, e. g. grapefruit juice, ketoconazole or, preferably, ritonavir. Such a composition may be manufactured in conventional manner, e. g. by mixing its components. Unit dosage forms contain, e. g., from about 0.1 to about 1000, preferably from about 1 to about 500, mg of an agent of the invention.

Thus in one embodiment of the invention there is provided a pharmaceutical composition comprising an agent of the invention as active ingredient and a pharmaceutically acceptable carrier or diluent.

In addition, the pharmaceutical compositions of the present invention can be made up in a solid form (including without limitation capsules, tablets, pills, granules, powders or suppositories), or in a liquid form (including without limitation solutions, suspensions or emulsions). The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifers and buffers, etc.

Typically, the pharmaceutical compositions are tablets or gelatin capsules comprising the active ingredient together with
a) diluents, *e.g*., lactose, dextrose, sucrose, mannitol, sorbitol, cellulose and/or glycine;
b) lubricants, *e.g*., silica, talcum, stearic acid, its magnesium or calcium salt and/or polyethyleneglycol; for tablets also
c) binders, *e.g*., magnesium aluminum silicate, starch paste, gelatin, tragacanth, methylcellulose, sodium carboxymethylcellulose and/or polyvinylpyrrolidone; if desired
d) disintegrants, *e.g*., starches, agar, alginic acid or its sodium salt, or effervescent mixtures; and/or
e) absorbents, colorants, flavors and sweeteners.

Tablets may be either film coated or enteric coated according to methods known in the art.

Suitable compositions for oral administration include an effective amount of a compound of the invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets may contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Certain injectable compositions are aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, or contain about 1-50%, of the active ingredient.

Suitable compositions for transdermal application include an effective amount of a compound of the invention with a suitable carrier. Carriers suitable for transdermal delivery include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Suitable compositions for topical application, *e.g*., to the skin and eyes, include aqueous solutions, suspensions, ointments, creams, gels or sprayable formulations, *e.g*., for delivery by aerosol or the like. Such topical delivery systems will in particular be appropriate for dermal application, *e.g.,* for the treatment of skin cancer, *e.g.,* for prophylactic use in sun creams, lotions, sprays and the like. They are thus particularly suited for use in topical, including cosmetic, formulations well-known in the art. Such may contain solubilizers, stabilizers, tonicity enhancing agents, buffers and preservatives.

As used herein a topical application may also pertain to an inhalation or to an intranasal application. They may be conveniently delivered in the form of a dry powder (either alone, as a mixture, for example a dry blend with lactose, or a mixed component particle, for example with phospholipids) from a dry powder inhaler or an aerosol spray presentation from a pressurised container, pump, spray, atomizer or nebuliser, with or without the use of a suitable propellant.

The present invention further provides anhydrous pharmaceutical compositions and dosage forms comprising the compounds of the present invention as active ingredients, since water may facilitate the degradation of certain compounds.

Anhydrous pharmaceutical compositions and dosage forms of the invention can be prepared using anhydrous or low moisture containing ingredients and low moisture or low humidity conditions. An anhydrous pharmaceutical composition may be prepared and stored such that its anhydrous nature is maintained. Accordingly, anhydrous compositions are packaged using materials known to prevent exposure to water such that they can be included in suitable formulary kits. Examples of suitable packaging include, but are not limited to, hermetically sealed foils, plastics, unit dose containers (*e.g*., vials), blister packs, and strip packs.

The invention further provides pharmaceutical compositions and dosage forms that comprise one or more agents that reduce the rate by which the compound of the present invention as an active ingredient will decompose. Such agents, which are referred to herein as "stabilizers," include, but are not limited to, antioxidants such as ascorbic acid, pH buffers, or salt buffers, etc.

In accordance with the foregoing, in a further aspect, the invention relates to an agent of the invention for use as a medicament, for example for the treatment or prevention of a neurological or vascular condition, disease or disorder, in which beta-amyloid generation or aggregation plays a role, or for the suppression of the metastasis process associated with tumor cells. In a further embodiment, the invention relates to an agent of the invention for use in the treatment or prevention of a disease or disorder mediated by BACE-1, BACE-2 or cathepsin D activity. In one embodiment, the invention relates to an agent of the invention for use in the treatment or prevention of Alzheimer's Disease or mild cognitive impairment.

In a further aspect, the invention relates to the use of an agent of the invention as an active pharmaceutical ingredient in a medicament, for example for the treatment or prevention of a neurological or vascular condition, disease or disorder, in which beta-amyloid generation or aggregation plays a role, or for the suppression of the metastasis process associated with tumor cells. In a further embodiment, the invention relates to the use of an agent of the invention as an active pharmaceutical ingredient in a medicament for the treatment or prevention of a disease or disorder mediated by BACE-1, BACE-2 or cathepsin D activity. In one embodiment, the invention relates to the use of an agent of the invention as an active pharmaceutical ingredient in a medicament for the treatment or prevention of Alzheimer's Disease or mild cognitive impairment.

In a further aspect, the invention relates to the use of an agent of the invention for the manufacture of a medicament for the treatment or prevention of a neurological or vascular condition, disease or disorder, in which beta-amyloid generation or aggregation plays a role, or for the suppression of the metastasis process associated with tumor cells. In a further embodiment, the invention relates to the use of an agent of the invention for the manufacture of a medicament for the treatment or prevention of a disease or disorder mediated by BACE-1, BACE-2 or cathepsin D activity. In one embodiment, the invention relates to the use of an agent of the invention for the manufacture of a medicament for the treatment or prevention of Alzheimer's Disease or mild cognitive impairment.

An agent of the invention can be administered as sole active pharmaceutical ingredient or as a combination with at least one other active pharmaceutical ingredient effective, e. g., in the treatment or prevention of a neurological or vascular condition, disease or disorder, in which beta-amyloid generation or aggregation plays a role, or in the suppression of the metastasis process associated with tumor cells. Such a pharmaceutical combination may be in the form of a unit dosage form, which unit dosage form comprises a predetermined quantity of each of the at least two active components in association with at least one pharmaceutically acceptable carrier or diluent. Alternatively, the pharmaceutical combination may be in the form of a package comprising the at least two active components separately, e.g. a pack or dispenser-device adapted for the concomitant or separate administration of the at least two active components, in which these active components are separately arranged. In a further aspect, the invention relates to such pharmaceutical combinations.

In a further aspect, the invention therefore relates to a combination comprising a therapeutically effective amount of an agent of the invention and a second drug substance, for simultaneous or sequential administtation.

In one embodiment, the invention provides a product comprising an agent of the invention and at least one other therapeutic agent as a combined preparation for simultaneous, separate or sequential use in therapy. In one embodiment, the therapy is the treatment of a disease or condition mediated by BACE-1, BACE-2 or cathepsin D activity. In a further embodiment, the therapy is the treatment of Alzheimer's Disease or mild cognitive impairment.

In one embodiment, the invention provides a pharmaceutical composition comprising an agent of the invention and another therapeutic agent(s). Optionally, the pharmaceutical composition may comprise a pharmaceutically acceptable excipient, as described above.

In one embodiment, the invention provides a kit comprising two or more separate pharmaceutical compositions, at least one of which contains an agent of the invention. In one embodiment, the kit comprises means for separately retaining said compositions, such as a container, divided bottle, or divided foil packet. An example of such a kit is a blister pack, as typically used for the packaging of tablets, capsules and the like. The kit of the invention may be used for administering different dosage forms, for example, oral and parenteral, for administering the separate compositions at different dosage intervals, or for titrating the separate compositions against one another. To assist compliance, the kit of the invention typically comprises directions for administration.

In the combination therapies of the invention, the agent of the invention and the other therapeutic agent may be manufactured and/or formulated by the same or different manufacturers. Moreover, the compound of the invention and the other therapeutic may be brought together into a combination therapy: (i) prior to release of the combination product to physicians (e.g. in the case of a kit comprising the compound of the invention and the other therapeutic agent); (ii) by the physician themselves (or under the guidance of the physician) shortly before administration; (iii) in the patient themselves, e.g. during sequential administration of the compound of the invention and the other therapeutic agent. Accordingly, the invention provides an agent of the invention for use in the treatment of a disease or condition mediated by BACE-1, BACE-2 or cathepsin D activity, in particular Alzheimer's Disease or mild cognitive impairment, wherein the medicament is prepared for administration with another therapeutic agent. The invention also provides the use of another therapeutic agent for treating a disease or condition mediated by BACE-1, BACE-2 or cathepsin D activity, in particular Alzheimer's Disease or mild cognitive impairment, wherein the medicament is administered with an agent of the invention.

The application also describes the use of an agent of the invention for treating a disease or condition mediated by BACE-1, BACE-2 or cathepsin D activity, in partcular Alzheimer's Disease or mild cognitive impairment, wherein the patient has previously (*e.g*. within 24 hours) been treated with another therapeutic agent. The application also describes the use of another therapeutic agent for treating a disease or condition mediated by BACE-1, BACE-2 or cathepsin D activity, in particular Alzheimer's Disease or mild cognitive impairment, wherein the patient has previously (*e.g*. within 24 hours) been treated with an agent of the invention.

In one embodiment, the invention relates to a compound of the invention in combination with another therapeutic agent wherein the other therapeutic agent is selected from:
(a) acetylcholinesterase inhibitors, such as donepezil (Aricept™), rivastigmine (Exelon™) and galantamine (Razadyne™);
(b) glutamate antagonists, such as memantine (Namenda™);
(c) antidepressant medications for low mood and irritability, such as citalopram (Celexa™), fluoxetine (Prozac™), paroxeine (Paxil™), sertraline (Zoloft™) and trazodone (Desyrel™);
(d) anxiolytics for anxiety, restlessness, verbally disruptive behavior and resistance, such as lorazepam (Ativan™) and oxazepam (Serax™);
(e) antipsychotic medications for hallucinations, delusions, aggression, agitation, hostility and uncooperativeness, such as aripiprazole (Abilify™), clozapine (Clozaril™), haloperidol (Haldol™), olanzapine (Zyprexa™), quetiapine (Seroquel™), risperidone (Risperdal™) and ziprasidone (Geodon™);
(f) mood stabilizers, such as carbamazepine (Tegretol™) and divalproex (Depakote™);
(g) nicotinic apha - 7 agonists;
(h) mGluR5 antagonists;
(i) H3 agonists; and
(j) amyloid therapy vaccines.

In another embodiment, the invention provides a pharmaceutical composition comprising:
i) a compound of the invention, or a pharmaceutically acceptable salt thereof;
ii) at least one therapeutic agent selected from:
   a) acetylcholinesterase inhibitors,
   b) glutamate antagonists,
   c) antidepressant medications,
   d) anxiolytics,
   e) antipsychotic medications,
   f) mood stabilizers,
   g) nicotinic apha - 7 agonists,
   h) mGluR5 antagonists,
   i) H3 agonists, and
   j) amyloid therapy vaccines; and
iii) one or more pharmaceutically acceptable carriers or diluents.

The following Examples illustrate the invention.

### Examples

### Abbreviations

- ACN: acetonitrile
- AcOH: acetic acid
- aq.: aqueous
- Boc: tert-butoxycarbonyl
- t-Bu: tert-butyl
- t-BuOH: tert-butanol
- conc.: concentrated
- DAST: diethylaminosulfurtrifluoride (Et₂N)₂SF₃
- DCM: dichloromethane
- DEAD: diethyl azodicarboxylate
- DIAD: diisopropyl azodicarboxylate
- DIPEA: diisopropylethylamine
- DMF: dimethylformamide
- DMSO: dimethylsulfoxide
- DPPF: 1,1'-bis(diphenylphosphino)ferrocene
- EDC: 1-(3-dimethylaminopropyl)-3-ethylcarbodiimide hydrochloride
- eq.: equivalent(s)
- ESI: electrospray ionisation
- Et₃N: triethylamine
- Et₂O: diethylether
- EtOAc: ethyl acetate
- EtOH: ethanol
- h: hour(s)
- Hex: hexane
- HMDS: hexamethyldisilazane
- HOAt: 1-hydroxy-7-aza-benztriazole
- HOBT: hydroxy-benztriazole
- HPLC: high performance liquid chromatography
- LCMS: liquid chromatography with mass spectrometry
- MeOH: methanol
- min: minute(s)
- MS: mass spectrometry
- NMR: nuclear magnetic resonance spectrometry
- NP: normal phase
- PE: petrolether
- PPh₃: triphenylphosphine
- Rf: retention factor (TLC)
- RP: reverse phase
- Rt: retention time
- rt: room temperature
- sat.: saturated
- soln.: solution
- TBME: tert-butyl-methyl-ether
- TFA: trifluoroacetic acid
- THF: tetrahydrofuran
- TLC: thin layer chromatography
- UPLC: ultra performance liquid chromatography

### General chromatography information

| **HPLC method H1 (Rt_{H1}):** | |
|---|---|
| HPLC-column dimensions: | 3.0 x 30 mm |
| HPLC-column type: | SB-C18, 1.8 µm |
| HPLC-eluent: | A) water + 0.05 Vol.-% TFA; B) ACN + 0.05 Vol.-% TFA |
| HPLC-gradient: | 30 - 100 % B in 3.25 min, flow = 0.7 ml / min |
| | |

| **HPLC method H2 (Rt_{H2}):** | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA; B) ACN + 0.05 Vol.-% TFA |
| HPLC-gradient: | 0 - 100 % B in 3.25 min, flow = 0.7 ml / min |
| | |

| **LCMS method H3 (Rt_{H3}):** | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA, B) ACN + 0.05 Vol.-% TFA |
| HPLC-gradient: | 10 - 100 % B in 3.25 min, flow = 0.7 ml / min |
| | |

| **LCMS method H4 (Rt_{H4}):** | |
|---|---|
| HPLC-column dimensions: | 3.0 x 30 mm |
| HPLC-column type: | Zorbax SB-C8, 1.8 µm |
| HPLC-eluent: | A) water + 0.05 Vol.-% TFA, B) ACN + 0.05 Vol.-% TFA |
| HPLC-gradient: | 10 - 95 % B in 2.00 min, 95 % B 2.00 min, flow = 0.7 ml / min |
| | |

| **UPLC method H5** (Rt_{H5})**:** | |
|---|---|
| HPLC-column dimensions: | 2.1 x 50 mm |
| HPLC-column type: | Acquity UPLC HSS T3 C18, 1.7 µm |
| HPLC-eluent: | A) water + 0.1 Vol.-% TFA, B) ACN + 0.1 Vol.-% TFA |
| H PLC-gradient: | 5 - 100 % B in 1.5 min, flow = 1.0 ml / min |
| | |

| **LCMS method H6 (Rt_{H6}):** | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA; B) ACN + 0.05 Vol.-% TFA |
| HPLC-gradient: | 40 - 100 % B in 3.25 min, flow = 0.7 ml / min |
| | |

| **LCMS method H7 (Rt_{H7}):** | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA; B) ACN + 0.05 Vol.-% TFA |
| HPLC-gradient: | 50 - 100 % B in 3.25 min, flow = 0.7 ml / min |
| | |

| **UPLC method H8 (Rt_{H8}):** | |
|---|---|
| HPLC-column dimensions: | 2.1 x 50 mm |
| HPLC-column type: | Acquity UPLC HSS T3, 1.8 µm |
| HPLC-eluent: | A) water + 0.1 Vol.-% formic acid, B) ACN + 0.1% formic acid |
| HPLC-gradient: | 10-95% B in 1.5 min, 1.0 min 95% B, flow = 1.2 ml / min |
| HPLC-column temperature: | 50 °C |
| | |

| **UPLC method H9 (Rt_{H9}):** | |
|---|---|
| HPLC-column dimensions: | 2.1 x 50 mm |
| HPLC-column type: | Acquity UPLC HSS T3, 1.8 µm |
| HPLC-eluent: | A) water + 0.05 Vol.-% formic acid + 3.75 mM ammonium acetate B) ACN + 0.04 Vol.-% formic acid |
| HPLC-gradient: | 2 - 98 % B in 1.4 min, 98% B 0.45 min, flow = 1.2 ml / min |
| HPLC-column temperature: | 50 °C |
| | |

| **UPLC method H10 (Rt_{H10}):** | |
|---|---|
| HPLC-column dimensions: | 2.1 x 50 mm |
| HPLC-column type: | Acquity UPLC HSS T3, 1.8 µm |
| HPLC-eluent: | A) water + 0.05 Vol.-% formic acid + 3.75 mM ammonium acetate B) ACN + 0.04 Vol.-% formic acid |
| HPLC-gradient: | 2 - 98 % B in 1.4 min, 98% B 0.75 min, flow = 1.2 ml / min |
| HPLC-column temperature: | 50 °C |
| | |

| **LCMS method H11 (Rt_{H11}):** | |
|---|---|
| HPLC-column dimensions: | 2.1 x 30 mm |
| HPLC-column type: | Ascentis Express C18, 2.8 µm |
| HPLC-eluent | A) water + 0.05 Vol.-% formic acid + 3.75 mM ammonium acetate, B) ACN + 0.04 Vol.-% formic acid |
| HPLC-gradient: | 2 - 98 % B in 1.4 min, 0.75 min 98% B, flow = 1.2 ml / min |
| HPLC-column temperature: | 50 °C |
| | |

| **UPLC method H12 (Rt_{H12}):** | |
|---|---|
| HPLC-column dimensions: | 2.1 x 50 mm |
| HPLC-column type: | Acquity UPLC HSS T3 C18, 1.8 µm |
| HPLC-eluent: | A) water + 0.1 Vol.-% TFA, B) ACN + 0.1 Vol.-% TFA |
| HPLC-gradient: | 10 - 100 % B in 1.5 min, flow = 1.0 ml / min |
| HPLC-column temperature: | 35 °C |

### Example 1: 5-Chloro-3-methoxymethyl-pyridine-2-carboxylic acid [3-((R)-5-amino-3-difluoromethyl-3,6-dihydro-2H-[1,4] oxazin-3-yl)-4-fluoro-phenyl]-amide

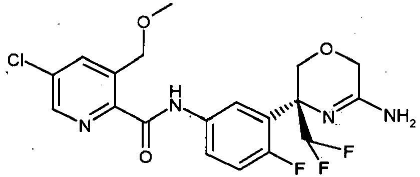

### a) 1-(5-Bromo-2-fluoro-phenyl)-ethanone

A solution of diisopropyl amine (17.78 ml, 126 mmol) in THF (375 ml) was cooled to -78 °C. A 1.6 M solution of BuLi in hexanes (79 ml, 126 mmol) was added drop wise. After 15 minutes 4-bromo-1-fluoro benzene (20 g, 114 mmol) was added dropwise while keeping the temperature below -60 °C. After stirring for 2.5 h at -70 °C ethyl difluoro acetate (13.22 ml) were added. The mixture was warmed to -40 °C and then quenched by pouring the mixture onto 1M HCl. The mixture was extracted with ligroine, dried with MgSO₄.H₂O, concentrated and purified by column chromatography (silica gel; hexane/5-15% TBME) to give the desired product as a yellow liquid.
¹H-NMR (CDCl₃, 360 MHz): δ 8.09 (dd, 1H), 7.82-7.77 (m, 1H), 7.17 (t, 1H), 6.45 (t, 1H, CHF₂).

### b) 1-(5-Bromo-2-fluoro-phenyl)-1-difluoromethyl-allyl]-carbamic acid tert-butyl ester

A mixture of 1-(5-bromo-2-fluoro-phenyl)-ethanone (16 g, 63.2 mmol) and N-tert-butyloxycarbonyl-triphenyliminophosphorane (26.3 g, 69.6 mmol) were heated at 90 °C in toluene for 18 h. The mixture was triturated with hexane and filtered to remove triphenyl phosphine oxide. The filtrate was purified by chromatography on silica gel (hexane/1-5% TBME) to give 11.37 g (32.3 mmol) of the desired product as a slightly impure yellow oil. TLC: Rf (Hexane / EtOAc 6:1) = 0.65.
The product was dissolved in THF (100 ml) and cooled to -78 °C. Vinylmagnesium bromide (48 ml of a 1M solution in THF) was added dropwise, while the reaction temperature was not allowed to exceed -60 °C. The mixture was stirred at -70 °C for 1 h before it was allowed to warm to 0 °C. The reaction was quenched with 10% aq. ammonium chloride and extracted with TBME. The organic layer was washed with brine, treated with activated charcoal and MgSO₄.H₂O and filtered over celite. The filtrated was concentrated and crystallized from hexane to give the desired product as colorless crystals.
HPLC: Rt_{H1}= 3.575 min; ESIMS [M+Na]⁺=402/404(1Br);
¹H-NMR (CDCl₃, 360 MHz): δ 7.57 (dd, 1H), 7.51-7.45 (m, 1H), 7.00 (dd, 1H), 6.49 (t, 1H, CHF₂), 6.21 (dd, 1H), 5.59 (d, 1H), 5.40 (dd, 1 H), 5.25 (br, 1 H), 1.40 (br s, 9H).

### c) [1-(5-Bromo-2-fluoro-phenyl)-2,2-difluoro-1-hydroxymethyl-ethyl]-carbamic acid tert-butyl ester

A suspension of 1-(5-bromo-2-fluoro-phenyl)-1-difluoromethyl-allyl]-carbamic acid tert-butyl ester (10.99 g, 28.9 mmol) and sodium hydrogen carbonate (3.84 g, 43.4 mmol) in DCM (200 ml) and MeOH (80 ml) was cooled to -78 °C. A mixture of O₃ in oxygen gas was introduced till the blue color persisted. The excess ozone was removed by bubbling through oxygen gas for 10 minutes. NaBH₄ (2.187 g, 57.8 mmol) was added as a solid in three portions. The mixture was stirred 10 min at -78 °C and then allowed to warm to 0 °C. After 30 min the mixture was poured onto ice-cold 1 N HCl and extracted with TBME. The organic phase was washed with 1 N HCl, brine, dried with MgSO₄.H₂O and evaporated. The crude product was crystallized from hexane to give the desired product as colorless crystals.
TLC: Rf (Hexane / EtOAc 4:1) = 0.29;
HPLC: Rt_{H1}= 3.000 min; ESIMS [M+Na]⁺=406/408(1Br);
¹H-NMR (DMSO-d6, 360 MHz): δ 7.60-7.49 (m, 2H), 7.42 (br s, 1 H), 7.180 (dd, 1 H), 6.49 (t, 1H, CHF₂), 5.27 (br s, 1H), 3.90 (br s, 2H), 1.35 (br s, 9H).

### d) N-[1-(5-Bromo-2-fluoro-phenyl)-2,2-difluoro-1-hydroxymethyl-ethyl]-2-chloro-acetamide

A suspension of [1-(5-bromo-2-fluoro-phenyl)-2,2-difluoro-1-hydroxymethyl-ethyl]-carbamic acid tert-butyl ester (10.22 g, 26.6 mmol) in 4N HCl in dioxane (133 ml) was stirred for two h at rt. The mixture was evaporated to give the hydrochloride salt of 2-amino-2-(5-bromo-2-fluoro-phenyl)-3,3-difluoro-propan-1-ol.
HPLC: Rt_{H3}= 2.550 min; ESIMS [M+H]⁺=284,286(1Br);
The crude product was taken up in DCM (63 ml) and 10% aq. soda (63 ml) and stirred vigorously with ice-cooling. A solution of chloroacetyl chloride (3.34 ml, 42 mmol) in DCM (10 ml) was added dropwise. The ice bath was taken away and stirring was continued for 1 h. The mixture was diluted with TBME and water. The organic phase was dried with MgSO₄.H₂O and purified via chromatography on silica gel (hexane/25-33% EtOAc) to give the desired product as a slightly impure resin.
HPLC: Rt_{H3}= 3.336 min; ESIMS [M+H]⁺=360/362/364 (1Br, 1CI);
¹H-NMR (DMSO-d6, 360 MHz): δ 8.78 (s, 1H), 7.62-7.53 (m, 2H), 7.19 (dd, 1H), 6.53 (t, 1H, CHF₂), 5.43 (t, 1 H), 4.27-4.02 (m, 4H).

### e) 5-(5-Bromo-2-fluoro-phenyl)-5-difluoromethyl-morpholin-3-one

A solution of N-[1-(5-bromo-2-fluoro-phenyl)-2,2-difluoro-1-hydroxymethyl-ethyl]-2-chloro-acetamide (9.59 g, 26.2 mmol) in t-butanol (134 ml) was treated with KOtBu (3.58 g). The mixture was heated at reflux for 3 h. After cooling down the mixture was diluted with EtOAc and 1 N HCl. The organic phase was washed with brine, dried with MgSO₄.H₂O, filtered and evaporated. The product was obtained as colorless crystal (TBME/hexane).
TLC: Rf (Hexane / EtOAc 2:1) = 0.29;
HPLC: Rt_{H3}= 2.950 min; ESIMS [M+H]⁺ =324/326(1 Br);
¹H-NMR (CDCl3, 360 MHz): δ 7.61-7.55 (m, 2H), 7.09 (dd, 1H), 6.80 (br, 1H), 6.35 (t, 1H, CHF2), 4.37-4.17 (m, 4H).

### f) 5-Difluoromethyl-5-(2-fluoro-phenyl)-morpholin-3-one

5-(5-Bromo-2-fluoro-phenyl)-5-difluoromethyl-morpholin-3-one (190 g, 586 mmol) and sodium acetate (57.7 g, 703 mmol) were suspended in 1850 mL methanol. Eventually, 10 % Pd on charcoal (18.7 g) were added and the rm was shaked in a Parr apparatus in an atmosphere of hydrogen at rt. After 60 minutes the reaction mixture was filtered over celite and evaporated. The residue was dissolved in 2 l TBME, washed with aq NaHCO₃ and brine. The organic layer was dried over MgSO₄.H₂O and evaporated to give 143.2 g of the title compound as a white solid.
HPLC: Rt_{H1} = 0.792 min; ESIMS [M+H]⁺= 246;
¹H-NMR (CDCl₃, 360 MHz): δ 7.50-7.43 (m, 2H), 7.32-7.27 (m, 1H), 7.19 (dd, 1H), 6.62 (br, 1 H), 6.37 (t, J = 54 Hz, 1 H), 4.34 (d, 1 H), 4.31 (d, 1 H), 4.22 (d, 1 H), 4.20 (d, 1 H).

### g) 5-Difluoromethyl-5-(2-fluoro-phenyl)-morpholine-3-thione

A mixture of 5-difluoromethyl-5-(2-fluoro-phenyl)-morpholin-3-one (141 g, 575 mmol) and Lawesson's reagent (132 g, 316 mmol) in 1400 ml of THF was heated at 68 °C for 1 h, cooled down and then evaporated. The residue was dissolved in 1 l DCM and filtered over 2 kg silica gel with 10 I DCM to give 161 g of the title compound in the form of a greenish resin that slowly crystallized. The compound was used without further purification.
HPLC: Rt_{H1} = 1.799 min; ESIMS [M+H]⁺= 262;
¹H-NMR (360 MHz, CDCl₃): δ 7.42-7.35 (m, 1 H), 7.28 (t, 1 H), 7.19 (t, 1 H), 7.11 (dd, 1 H), 6.29 (t, J = 54 Hz, 1H), 4.57 (d, 1H), 4.47 (d, 1H), 4.21 (d, 1 H), 4.18 (d,1H).

### h) 5-Difluoromethyl-5-(2-fluoro-phenyl)-5,6-dihydro-2H-[1,4]oxazin-3-ylamine

5-Difluoromethyl-5-(2-fluoro-phenyl)-morpholine-3-thione (160 g, 570 mmol) was dissolved in 2.4 l of a NH₃ solution 7 mol/l in methanol for 6.5 h and afterwards left standing overnight. The reaction mixture was evaporated and taken up in 2 l 1N aq HCl and 2 l TBME. The aq phase was washed with TBME and made basic through the addition of 30% aq. NaOH (300 ml) and some ice. The mixture was extracted with DCM three times and the combined organic layers were dried with Na₂SO₄ and concentrated in vacuo. The title compound was obtained by crystallization from DCM/ heptanes (128.45 g).
HPLC: Rt_{H3} = 2.059 min; ESIMS [M+H]⁺= 245;
¹H-NMR (CDCl₃, 360 MHz): δ 7.77 (t, 1 H), 7.38 - 7.30 (m, 1H), 7.21 (t, 1 H), 7.09 (dd, 1 H), 6.19 (t, J = 54 Hz, 1 H), 4.51 (br, 2H), 4.32, (d, 1H), 4.18 (d; 1 H), 4.05 (d, 1H), 3.96 (d, 1 H), 1.39 (s, 3H), 1.24 (s, 3H).

### i) 5-Difluoromethyl-5-(2-fluoro-5-nitro-phenyl)-5,6-dihydro-2H-[1,4]oxazin-3-ylamine

Potassium nitrate (60.3 g, 596 mmol) was added portionwise to 600 ml sulfuric acid (T <20 °C). This solution was added dropwise to a solution of 5-difluoromethyl-5-(2-fluoro-phenyl)-5,6-dihydro-2H-[1,4]oxazin-3-ylamine (112 g, 459 mmol) in sulfuric acid (600 ml), while keeping the reaction temperature <22 °C with an ice bath. After stirring for 1 h, the mixture was poured onto 10 kg ice. TBME (6 l) was added and the pH was adjusted to 12-14 by the addtion of 30% aq NaOH (ca. 5 l). The phases were separated and the aq. phase was extracted twice with TBME. The compined org layers were dried with sodium sulfate and evaporated to give 130 g of a yellow solid that was used further without purification.
HPLC: Rt_{H3}= 2.063 min; ESIMS [M+H]⁺= 290;
¹H-NMR (CDCl₃, 360 MHz): δ 8.71 (dd, 1H), 8.13 (dt, 1H), 7.13 (dd, 1H), 5.99 (t, J = 54 Hz, 1H), 4.55 (br, 2H), 4.33 (dd, 1H), 4.10 (d, 1H), 3.97 (d, 1H), 3.82 (dt, 1H).

### j) [5-Difluoromethyl-5-(2-fluoro-5-nitro-phenyl)-5,6-dihydro-2H-[1,4]oxazin-3-yl]-carbamic acid tert-butyl ester

A solution of 5-Difluoromethyl-5-(2-fluoro-5-nitro-phenyl)-5,6-dihydro-2H-[1,4]oxazin-3-ylamine (144.5 g, 500 mmol), Boc anhydride (142 g, 650 mmol) and DIPEA (131 ml, 749 mmol) in 2500 ml THF was stirred for 3 days at rt. There was still tarting material left. Boc anhydride (56 g, 325 mmol) was added, the mixture was heated to 60 °C and stirred for 10 h till the reaction was complete. The mixture was evaporated, dissolved in TBME, washed with ice-cold 1N aq HCl, water, 10% aq. NaHCO₃ and brine. The org phase was dried with sodium sulfate, filtered and evaporated. The product was purified by crystallization from DCM/ heptanes. Yield 182.8 g white crystals.
HPLC: Rt_{H1} = 3.259 min; ESIMS [M+Na]⁺= 412;
¹H-NMR (CDCl₃, 360 MHz): δ 8.70 (dd, 1H), 8.27 (dt, 1 H), 7.34 (br, 1H), 7.25 (dd, 1H), 6.09 (t, J = 54 Hz, 1H), 4.85 (d, 1H), 4.58 (d, 1H), 4.49 (dd, 1H), 3.94 (dt, 1H).

### k) [5-(5-Amino-2-fluoro-phenyl)-5-difluoromethyl-5,6-dihydro-2H-[1,4]oxazin-3-yl]-carbamic acid tert-butyl ester

[5-Difluoromethyl-5-(2-fluoro-5-nitro-phenyl)-5,6-dihydro-2H-[1,4]oxazin-3-yl]-carbamic acid tert-butyl ester (180 g, 462 mmol) and 17.61 g Pd-C 10% were suspended in THF (1760 ml). The mixture was shaked in a Parr apparatus in an atmosphere of hydrogen at rt. After 6 h the rm was filtered over celite and evaporated. The residue was crystallized from DCM/heptanes to provide 157.6 g of the title compound as beige crystals.
HPLC: Rt_{H3}= 2.748 min; ESIMS [M+H]⁺= 360;
¹H-NMR (CDCl₃, 360 MHz): δ Spectrum uninterpretable due to the presence of a complex mixture of rotamers.

### l) [(R)-5-(5-Amino-2-fluoro-phenyl)-5-difluoromethyl-5,6-dihydro-2H-[1,4]oxazin-3-yl]-carbamic acid tert-butyl ester

The racemic product ((rac.)[5-(5-Amino-2-fluoro-phenyl)-5-difluoromethyl-5,6-dihydro-2H-[1,4]oxazin-3-yl]-carbamic acid tert-butyl ester) was separated via prep. HPLC on a Chiralpak AD-H 20 µm (8 x 100 x 48mm HPLC colums), on a Bayer SMB CC50 instrument using SMB technology with heptane/EtOH/MeOH 70 : 20 : 10 as eluent. The desired compound was the slower eluting (R)-enantiomer. Yield 72.29 g of the title compound as a colorless foam. ee = 99.3 %; Opt. rotation: [α]_{D}-97.5° (c=1, CHCl₃)
HPLC: Rt_{H3} = 2.748 min; ESIMS [M+H]⁺ = 360;
¹H-NMR (CDCl₃, 360 MHz): δ Spectrum uninterpretable due to the presence of a complex mixture of rotamers.

### m) ((R)-5-{5-[(5-Chloro-3-methoxymethyl-pyridine-2-carbonyl)-amino]-2-fluoro-phenyl}-5-difluoromethyl-5,6-dihydro-2H-[1,4]oxazin-3-yl)-carbamic acid tert-butyl ester

5-Chloro-3-methoxymethyl-pyridine-2-carboxylic acid (56 mg, 0.278 mmol), [(R)-5-(5-Amino-2-fluoro-phenyl)-5-difluoromethyl-5,6-dihydro-2H-[1,4]oxazin-3-yl]-carbamic acid tert-butyl ester (example x, 100 mg, 0.278 mmol) and HOAt (68.2 mg, 0.50 mmol) were suspended in DMF (20 ml) and cooled down to 0° C. DIPEA (0.146 ml, 0.835 mmol) and EDC (80 mg, 0.417 mmol) were added and the reaction mixture was stirred at room temperature for 20 h. The reaction mixture was diluted with ethyl acetate, washed with water and brine, dried over sodium sulfate, filtered and evaporated. The crude product (592 mg) was chromatographed over silica gel (cyclohexane/ethyl acetate) to provide the title compound as a white glassy solid. TLC Rf (5:1 cyclohexane:ethyl acetate)=0.31;
MS: ESI+ 543, 545);¹H-NMR (360 MHz, CDCl₃): δ 10.03 (s, br. 1H), 8.45 (m, 1H), 8.21 (m, 1H), 8.01 (m, 1H), 7.66 (m, 1H), 7.09 (m, 1 H), 6.14 (t, 1H, CHF2), 5.09 (s, 2H), 4.79 (d, 1H), 4.56 (d, 1 H), 4.38 (d, 1 H), 3.95 (d, 1 H), 3.55 (s, 3H), 1.49 (s, 9H).

### n) 5-Chloro-3-methoxymethyl-pyridine-2-carboxylic acid [3-((R)-5-amino-3-difluoromethyl-3,6-dihydro-2H-[1,4] oxazin-3-yl)-4-fluoro-phenyl]-amide

To a solution of ((R)-5-{5-[(5-Chloro-3-methoxymethyl-pyridine-2-carbonyl)-amino]-2-fluorophenyl}-5-difluoromethyl-5,6-dihydro-2H-[1,4]oxazin-3-yl)-carbamic acid tert-butyl ester (150 mg, 0.276 mmol) in dichloromethane (4 ml) was added TFA (0.35 ml, 4.54 mmol) and the reaction mixture was stirred for 18 h at room temperature. The solvent was removed in vacuo and the residue diluted with ethyl acetate and poured onto a mixture of ammonia 2N/ice. The layers were separated and the organic phase was washed with water and brine, dried over sodium sulfate, filtered and evaporated. 116 mg. Silica gel chromatography (dichloromethane/methanol 95:5 + 1% ammonia) afforded the title compound. 102 mg.
TLC R_{f}=0.48 (dichloromethane/methanol 95:5 + 1% ammonia); ESI+ MS 443, 445; HPLC-MS: Rt_{H8}= 1.87min. (99% purity, ESI+ 443, 445);
¹H-NMR (600 MHz, DMSO-D₆): δ 10.65 (s, 1H), 8.68 (s, 1H), 8.10 (s, 1H), 8.02 (m, 1H), 7.80 (m, 1H), 7.18 (m, 1H), 6.17 (m, 3H, CHF2, NH2 (amidine)), 4.88 (s, 2H), 4.14 (d, 1H), 4.02 (d, 1H), 3.95 (d, 1H), 3.88 (d, 1 H), 3.41 (s, 3H).

**Examples 2 to 13**: The compounds listed in Table 1 were prepared by a procedure analogous to that used in Example 1.

Hydrochloride salts were obtained from solutions of the corresponding free base by addition of hydrochloric acid in dioxane or hydrochloric acid in diethylether and evaporation of the solvents.

**Table 1**

| **Example** | **Compound** | **¹H-NMR (δ; DMSO-d₆)** | **MS [m/z; (M+1)⁺]** |
|---|---|---|---|
| **2** | 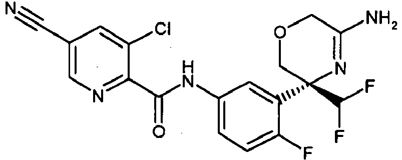 | 10.92 (s, 1H, NH), 9.10 (s, 1H), 8.80 (s, 1H), 7.91 (m, 1H), 7.80 (m, 1H), 7.20 (triplettoid, 1H), 6.17 (broad, 2H, NH₂ (amidine)), 6.17 (t, 1 H, CHF2), 4.11. (d, 1H), 4.01 (d, 1H), 3.91 (d, 1H), 3.82 (d, 1H). | 424, 426 |
| | 3-Chloro-5-cyano-pyridine-2-carboxylic acid [3-((R)-5-amino-3-difluoromethyl-3,6-dihydro-2H-[1,4]oxazin-3-yl)-4-fluoro-phenyl]-amide | | |
| **3** | 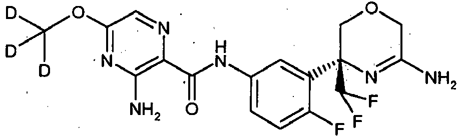 | 10.10 (s, 1H), 8.01 (dd, 1H), 7.80-7.67 (m, 1H), 7.50 (s, 1H), 7.11 (dd, 1H), 6.12 (br. t, 2H, CHF2 + 1H), 4.13 (dd, 1H), 4.04-3.95 (m, 1H), 3.93-3.85 (m, 1H), 3.79 (d, 1H). | 414 |
| | 3-Amino-5-tris-deutero-methoxy-pyrazine-2-carboxylic acid [3-((R)-5-amino-3-difluoromethyl-3,6-dihydro-2H-[1,4]oxazin-3-yl)-4-fluoro-phenyl]-amide | | |
| **4** | 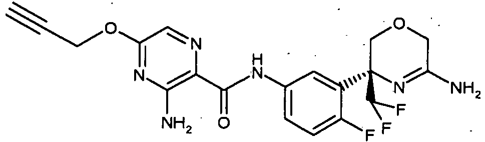 | 10.17 (s, 1H), 8.05 (dd, 1H), 7.82-7.69 (m, 2H), 7.57 (s, 1H), 7.14 (dd, 1H), 6.14 (br. t, 2H, CHF2 + 1H), 5.02 (d, 2H), 4.15 (dd, 1H), 4.07-3.97 (m, 1H), 3.97-3.87 (m, 1H), 3.82 (d, 1H), 3.62 (t, 1H). | 435 |
| | 3-Amino-5-prop-2-ynyloxy-pyrazine-2-carboxylic acid [3-((R)-5-amino-3-difluoromethyl-3,6-dihydro-2H-[1,4]oxazin-3-yl)-4-fluoro-phenyl]-amide | | |
| **5** | 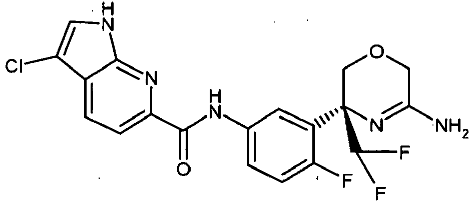 | 12.30 (s, 1H), 10.35 (s, 1H), 8.18 (d, 1H), 8.03 (broad, 1H), 7.98 (m, 2H), 7.90 (broad, 1H), 7.21 (triplettoid, 1H), 6.20 (broad, 2H, NH2), 6.18 (t, 1H, CHF2), 4.12 (d, 1H), 4.04 (d, 1H), 3.95 (d, 1H), 3.87 (d, 1H). | 438, 440 |
| | 3-Chloro-1H-pyrrolo[2,3-b]pyridine-6-carboxylic acid [3-((R)-5-amino-3-difluoromethyl-3,6-dihydro-2H-[1,4]oxazin-3-yl)-4-fluoro-phenyl]-amide | | |
| **6** | 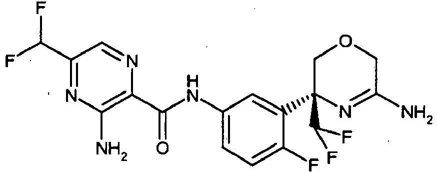 | 10.66 (s, 1H), 8.14 (s, 1H), 8.10 (d, 1H), 7.91 (br. s, 2H), 7.76 (br. s, 1H), 7.17 (t, 1H), 6.96 (t, 1H, CHF2), 6.23-6.04 (m, 3H, CHF2 + 1H), 4.14 (d, 1H), 4.05-3.97 (m, 1H), 3.96-3.87 (m, 1H), 3.82 (d, 1H). | 431 |
| | 3-Amino-5-difluoromethyl-pyrazine-2-carboxylic acid [3-((R)-5-amino-3-difluoromethyl-3,6-dihydro-2H-[1,4]oxazin-3-yl)-4-fluoro-phenyl]-amide | | |
| **7** | 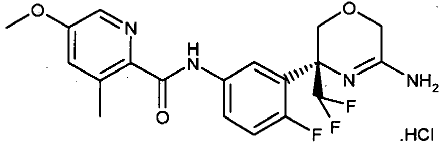 | 10.99 (s, 1H), 10.63 (s, 1H), 9.76 (br s, 1H), 8.76 (s, 1H), 8.23 (d, 1H), 8.05-8.00 (m, 1H), 7.97 (dd, 1H), 7.44 (d, 1H), 7.35 (dd, 1H), 6.78 (t, 1H), 4.71 (d, 1H), 4.64 (d, 1H), 4.35 (d, 1H), 4.17 (d, 1H), 3.91 (s, 3H), 2.63 (s, 3H). | 409 |
| | 5-Methoxy-3-methyl-pyridine-2-carboxylic acid [3-((R)-5-amino-3-difluoromethyl-3,6-dihydro-2H-[1,4]oxazin-3-yl)-4-fluoro-phenyl]-amide hydrochloride | | |
| **8** | 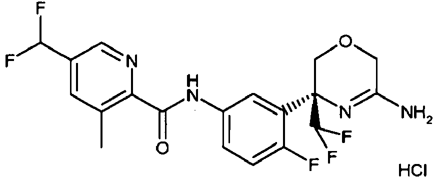 | 11.04 (br s, 1H), 10.87 (s, 1H), 9.78 (br s, 1H), 8.80 (s, 1H), 8.74 (s, 1H), 8.08 (s, 1H), 8.02-7.96 (m, 2H), 7.38 (dd, 1H), 7.25 (t, 1H), 6.79 (t, 1H), 4.72 (d, 1H), 4.65 (d, 1H), 4.35 (d, 1H), 4.18 (d, 1H), 2.61 (s, 3H). | 429 |
| | 5-Difluoromethyl-3-methyl-pyridine-2-carboxylic acid [3-((R)-5-amino-3-difluoromethyl-3,6-dihydro-2H-[1,4 ]oxazin-3-yl)-4-fluoro-phenyl]-amide hydrochloride | | |
| **9** | 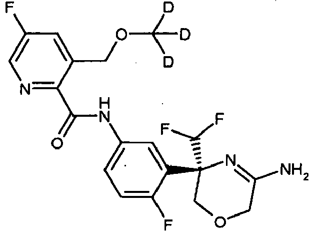 | 10.59 (s, 1H, NH), 8.62 (d, 1H), 8.01 (m, 1H), 7.88 (dd, 1H), 7.78 (broad, 1H), 7.16 (triplettoid, 1H), 6.15 (broad, 2H, NH2), 6.12 (t, 1H, CHF2), 4.85 (s, 2H), 4.11 (d, 1H), 4.00 (d, 1H), 3.90 (d, 1H), 3.83 (d, 1H). | 430 |
| | 5-Fluoro-3-trideuteromethoxymethyl-pyridine-2-carboxylic acid [3-((R)-5-amino-3-difluoromethyl-3,6-dihydro-2H-[1,4]oxazin-3-yl)-4-fluoro-phenyl]-amide | | |
| **10** | 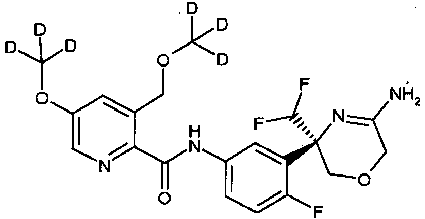 | 11.0 (s, 1 H, NH), 10.69 (s, 1H, NH, amide), 9.63 (s, 1H, NH₂, amidine), 8.76 (s, 1 H, NH₂, amidine), 8.29 (d, 1H), 8.02 (m, 1H), 7.96 (dd, 1H), 7.60 (d, 1H), 7.33 (dd, 1H), 6.76(t, 1H, CHF₂), 4.90 (s, 2H), 4.65 (d, 1H, AB), 4.61 (d, 1H, AB), 4.32 (d, 1H, AB), 4.14 (d, 1H, AB). | 445 |
| | 5-Trideuteromethoxy-3-trideuteromethoxymethyl-pyridine-2-carboxylic acid [3-((R)-5-amino-3-difluoromethyl-3,6-dihydro-2H-[1,4]oxazin-3-yl)-4-fluoro-phenyl]-amide | | |
| **11** | 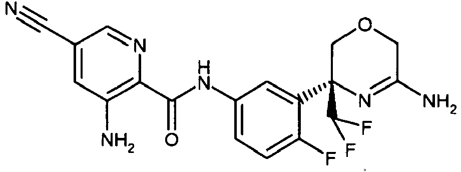 | 10.53 (s, 1H), 8.21 (d, 1H), 8.05 (dd, 1H), 7.77 (d, 1H), 7.64 (d, 1H), 7.25 (br. s, 2H), 7.16 (dd, 1H), 6.16 (br. s, 2H), 6.13 (t, 1H, CHF2), 4.14 (d, 1H), 4.01 (d, 1H), 3.91 (d, 1H), 3.81 (d, 1H). | 405 |
| | 3-Amino-5-cyano-pyridine-2-carboxylic acid [3-((R)-5-amino-3-difluoromethyl-3,6-dihydro-2H-[1,4]oxazin-3-yl)-4-fluoro-phenyl]-amide | | |
| **12** | 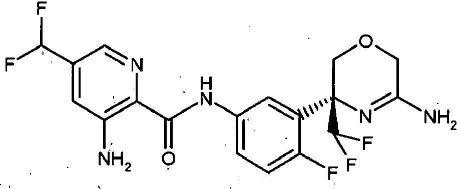 | 10.47 (s, 1H), 8.08-8.03 (m, 1H), 8.02 (s, 1H), 7.79 (d, 1H), 7.41 (s, 1H), 7.18-7.13 (m, 3H), 7.12 (t, 1H, CHF2), 6.17 (br. s, 2H), 6.13 (t, 1H, CHF2), 4.22-4.11 (m, 1H), 4.07-3.97 (m, 1H), 3.91 (d, 1H), 3.81 (d, 1H). | 430 |
| | 3-Amino-5-difluoromethyl-pyridine-2-carboxylic acid [3-((R)-5-amino-3-difluoromethyl-3,6-dihydro-2H-[1,4]oxazin-3-yl)-4-fluoro-phenyl]-amide | | |
| **13** | 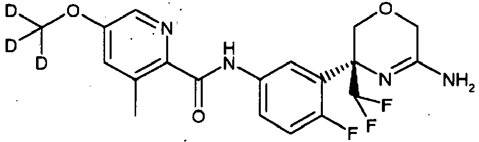 | 10.38 (s, 1H), 8.22 (d, 1H), 7.99 (dd, 1H), 7.85-7.80 (m, 1H), 7.40 (dd, 1H), 7.15 (dd, 1H), 6.29-5.98 (m, 3H), 4.13 (dd, 1H), 4.02 (d, 1H), 3.92 (d, 1H), 3.83 (d, 1H), 2.62 (s, 3H). | 412 |
| | 5-Trideuteromethoxy-3-methyl-pyridine-2-carboxylic acid [3-((R)-5-amino-3-difluoromethyl-3,6-dihydro-2H-[1,4]oxazin-3-yl)-4-fluoro-phenyl]-amide | | |

### Example 14: 5-Cyano-3-methyl-pyridine-2-carboxylic acid [3-((R)-5-amino-3-difluoromethyl-3,6-dihydro-2H-[1,4]oxazin-3-yl)-4,5-difluoro-phenyl]-amide

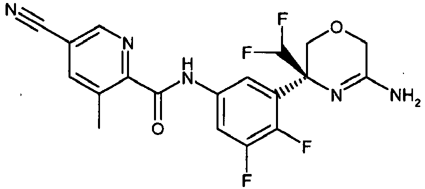

### a) 1-(2,3-Difluoro-phenyl)-2,2-difluoro-ethanone

A solution of 1,2-difluorobenzene (49.74 g, 436 mmol) in 700 ml THF was cooled to -70°C. Buli (1.6 M solution in hexanes, 272 ml, 436 mmol) was added dropwise while maintaining a reaction temperature <-60°C. After stirring for 2.5 h at -70°C, ethyl difluoroacetate (48.3 ml, 436 mmol) was added at such a rate that the reaction temperature did not exceed -45°C. After stirring for 5 min the mixture was poured onto 10% aq. NH₄Cl and TBME. The organic phase was washed with 5% aq. NaHCO₃, brine and dried with MgSO₄.H₂O. The solvents were distilled off at atmospheric pressure and the residual product was distilled at 12 mmHg. The fraction boiling at 89-90°C was collected to give 78.76 g of a colorless liquid.
¹H-NMR (CDCl₃, 400 MHz): δ 7.73 (t, 1H), 7.49 (q, 1H), 7.27 (m, 1H), 7.40 (t, J = 54 Hz, 1H).

### b) (S)-2-(2,3-Difluoro-phenyl)-1,1-difluoro-3-nitro-propan-2-ol

A solution of 1-(2,3-difluoro-phenyl)-2,2-difluoro-ethanone (21.8 g, 113 mmol) and nitromethane (61.2 ml, 1.135 mol) in 220 ml DCM was cooled to -25 °C. Catalyst 1 (3.12 g, 5.67 mmol) was added while stirring. The homogeneous solution was stored at -20 °C for 4 days. The catalyst was removed by chromatography on a small column of silica gel (DCM/(10% aq. NH₃/ EtOH) 99:1). Evaporation of the solvents gave 30.45 g crude product as a colorless oil. The product was further purified by chromatography on silica gel (hexanes/ DCM 50-100%) to give 27.9 g of the title compound as a colorless oil. α_{[D]} = +13.4° (c = 1, CHCl₃);
HPLC: Rt_{H3}= 2.055 min;
¹H-NMR (CDCl₃, 400 MHz): δ 7.52 (t, 1H), 7.33-7.20 (m, 2H), 6.00 (t, J = 54 Hz, 1H), 5.30 (d, 1H), 5.01 (d, 1H), 4.21 (s, 1H).

### c) (S)-3-Amino-2-(2,3-difluoro-phenyl)-1,1-difluoro-propan-2-ol

A solution of (S)-2-(2,3-difluoro-phenyl)-1,1-difluoro-3-nitro-propan-2-ol (27.97 g, 110 mmol) in 90 ml AcOH was added dropwise to a well-stirred suspension of Zn (72.3 g, 1.105 mol) powder in 200 ml AcOH. The reaction temperature was kept at 35-45 °C. After the addition the mixture was stirred rt 1 h, filtered over celite and washed with EtOAc. The filtrate and EtOAc washings were evaporated, the residue dissolved in EtOAc and so much 1 N aq. NaOH was added till the pH of the aq. layer had reached ca. 12. Insoluble parts were dissolved through the addition of a little sat. aq. NH₃. The organic layer was washed with brine, dried with MgSO₄.H₂O and evaporated. The residue was crystallized from TBME/hexanes to provide 22.4 g of the title compound as white crystals. HPLC: Rt_{H1}= 2.469 min [M+H]⁺ 224;
¹H-NMR (DMSO-d6, 400 MHz): δ 7.46-7.37 (m, 2H), 7.21 (q, 1H), 6.16 (t, J = 54 Hz,1H), 6.1 (br, 1 H), 3.06 (d, 1 H), 3.02 (d, 1 H), 4.21 (s, 1 H).

### d) N-[(S)-2-(2,3-Difluoro-phenyl)-3,3-difluoro-2-hydroxy-propyl]-2-nitro-benzenesulfonamide

A solution of (S)-3-amino-2-(2,3-difluoro-phenyl)-1,1-difluoro-propan-2-ol (22.4 g, 100 mmol) and pyridine (40.6 ml, 502 mmol) in 230 ml DCM was cooled at +5°C. 2-nitro-benzenesulfonyl chloride (23.36 g, 105 mmol) was added in portions (T<15°C). After the addition the mixture was stirred without ice-cooling for 1 h. The mixture was diluted with TBME and 2N HCl. The organic layer was washed with brine and dried with MgSO₄.H₂O and evaporated. The crude product was purified by chromatography on silica gel (hexanes/ DCM 15-30%, then DCM/ EtOH 0-3%) to give 39.6 g of the title compound as a yellow resin that crystallized upon standing.
HPLC: Rt_{H3}= 2.644 min [M+Na]⁺ 409 ;
¹H-NMR (CDCl₃, 400 MHz): δ 8.10 (m, 2H), 7.86 (m, 1H), 7.76 (m, 2H), 7.38 (t, 1H), 7.19-7.07 (m, 2H), 6.03 (t, J = 54 Hz, 1H), 5.67 (t, 1H), 3.88 (dd, 1H), 3.73 (dd, 1H), 3.41 (s, 1H).

### e) (R)-2-Difluoromethyl-2-(2,3-difluoro-phenyl)-1-(2-nitro-benzenesulfonyl)-aziridine

N-[(S)-2-(2,3-Difluoro-phenyl)-3,3-difluoro-2-hydroxy-propyl]-2-nitro-benzenesulfonamide (39.65 g, 97 mmol) was dissolved in 400 ml THF together with PPh₃ (30.6 g, 117 mmol), cooled to 0-5°C and treated with a 40% toluene solution of DEAD (53.4 ml, 117 mmol) in a dropwise manner. Stirring was continued for 3 h while slowly warming to rt. The solution was diluted with 400 ml toluene, concentrated to remove the THF and directly purified via chromatography on silica gel (hexanes/ DCM 50-70%) to give the title compound as a yellow resin.
HPLC: Rt_{H3}= 3.096 min [M+Na]⁺ 413 ;
¹H-NMR (CDCl₃, 400 MHz): δ 8.28-8.23 (m, 1H), 7.83-7.75 (m, 3H), 7.40 (t, 1H), 7.30-7.21 (m, 1 H), 7.19-7.12 (m, 1H), 6.17 (t, J = 54 Hz, 1H), 3.38 (s, 1H), 3.27 (s, 1H).

### f) Acetic acid (R)-2-(2,3-difluoro-phenyl)-3,3-difluoro-2-(2-nitro-benzenesulfonylamino)-propyl ester

A solution of (R)-2-difluoromethyl-2-(2,3-difluoro-phenyl)-1-(2-nitro-benzenesulfonyl)-aziridine (4.78 g, 12.25 mmol) in 50 ml DMSO was treated with KOAc (2.404 g, 24.49 mmol) and stirred for 2 h. The mixture was diluted with EtOAc, washed with water twice followed by brine and dried with MgSO₄.H₂O. The crude product was purified by chromatography on silica gel (hexanes/ EtOAc 25-35%) to give 4.6 g of the title compound as a colorless resin. HPLC: Rt_{H3}= 2.906 min [M+Na]⁺ 473;
¹H-NMR (CDCl₃, 400 MHz): δ 7.99 (d, 1H), 7.77-7.71 (m, 1H), 7.57 (m, 2H), 7.37-7.31 (m, 1H), 7.23-7.15 (m, 2H), 6.70 (s, 1H), 6.59 (t, J = 54 Hz, 1H), 4.57 (d, 1 H), 4.55 (d, 1 H), 2.10 (s, 3H).

### g) N-[(R)-1-(2,3-Difluoro-phenyl)-2,2-difluoro-1-hydroxymethyl-ethyl]-2-nitrobenzenesulfonamide

A solution of acetic acid (R)-2-(2,3-difluoro-phenyl)-3,3-difluoro-2-(2-nitrobenzenesulfonylamino)-propyl ester (4.57 g, 10.15 mmol) in 35 ml MeOH was treated with aq LiOH (4M, 12.68 ml, 50.7 mmol). The reaction was slightly exothermic. After 30 min the mixture was diluted with water brine and EtOAc. The organic layer was washed with 1 N HCl and brine, and dried with MgSO₄.H₂O. Evaporation gave the title compound as a white solid, pure enough for further transformations.
HPLC: Rt_{H3}= 2.516 min [M+Na]⁺ 431;
¹H-NMR (DMSO-d6, 400 MHz): δ 8.67 (s, 1H), 7.91 (d, 1H), 7.80 (t, 1H), 7.74-7.67 (m, 2H), 7.37 (q, 1H), 7.30-7.24 (m, 1H), 7.19-7.12 (m, 1H), 6.69 (t, J = 54 Hz, 1H), 5.44 (t, 1H), 3.98 (s, 2H), 2.10 (s, 3H).

### h) [(R)-2-(2,3-Difluoro-phenyl)-3,3-difluoro-2-(2-nitro-benzenesulfonylamino)-propoxy]-acetic acid ethyl ester

To a solution of N-[(R)-1-(2,3-difluoro-phenyl)-2,2-difluoro-1-hydroxymethyl-ethyl]-2-nitrobenzenesulfonamide (2.59 g, 6.34 mmol) and rhodium(II)acetate, dimer (0.056 g, 0.127 mmol) in 41 ml DCM was added ethyl diazoacetate (1.570 ml, 12.69 mmol) in 7.4 ml DCM over a period of 4 h using a syringe pump. The mixture was stirred for 1 h, diluted with hexanes and chromatographed on a silica gel column (hexanes/ DCM 50-100%) to give 1.78 g of the title compound as a slightly impure pale yellow resin.
HPLC: Rt_{H4}= 2.784 min [M+Na]⁺ 517;
¹H-NMR (CDCl₃, 400 MHz): δ 7.95 (d, 1 H), 7.70 (t, 1H), 7.60 (d, 1 H), 7.54 (t, 1H), 7.46 (t, 1H), 7.20-7.05 (m, 2H), 6.97 (s, 1H), 6.61 (t, J = 54 Hz, 1H), 4.34-4.08 (m, 6H), 1.37-1.27 (m, 3H).

### i) (R)-5-Difluoromethyl-5-(2,3-difluoro-phenyl)-morpholin-3-one

A solution of [(R)-2-(2,3-difluoro-phenyl)-3,3-difluoro-2-(2-nitro-benzenesulfonylamino)-propoxy]-acetic acid ethyl ester (2.46 g, 4.98 mmol) in 25 ml MeOH was treated with aq. LiOH (4M, 6.22 ml, 24.88 mmol). The reaction was slightly exothermic. After 30 min the mixture was diluted with 1 N HCl, brine and EtOAc. The organic layer was washed with brine and dried with MgSO₄.H₂O. Evaporation gave the title compound as a yellow resin, used for further transformations without purification. HPLC: Rt_{H3}= 2.575 min [M+Na]⁺ 489.
The product was dissolved in 12 ml EtOH and 6 ml THF, treated with thiophenol (1.1 g, 10 mmol) and 1 M NaOH (14.9 ml), and heated at 60 °C for 4 h. The mixture was cooled down and washed with TBME. The pH was adjusted to 6-7 with 1N HCl and evaporated to dryness. The residual product was extracted with EtOH (3x). The ethanol extracts were evaporated to give 1.69 g of a yellow foam. HPLC: Rt_{H1}= 3.478 min [M+H]⁺ 282.
This product was refluxed in 50 ml toluene containing 2.5 ml AcOH for 18 h. The mixture was evaporated and the title compound was isolated as a white solid after chromatography on silica gel (hexanes/ EtOAc 25-40%).
HPLC: Rt_{H2}= 2.673 min [M+H]⁺ 264;
¹H-NMR (CDCl₃, 400 MHz): δ 7.33-7.19 (m, 4H), 6.68 (br s, 1H), 6.34 (t, J = 54 Hz, 1H), 4.34-4.18 (m, 4H).

### j) (R)-5-Difluoromethyl-5-(2,3-difluoro-phenyl)-morpholine-3-thione

To a solution of (R)-5-difluoromethyl-5-(2,3-difluoro-phenyl)-morpholin-3-one (543 mg, 2.063 mmol) in 6 ml THF was added Lawesson's reagent (459 mg, 1.135 mmol) and the mixture was stirred at 50 °C for 45 min. The mixture was evaporated and purified by chromatography on silica gel (hexanes/EtOAc 10-15%) to give 587 mg of the title compound as a colorless resin.
HPLC: Rt_{H2}= 3.124 min [M+H]⁺ 280;
¹H-NMR (CDCl₃, 400 MHz): δ 8.43 (br s, 1H), 7.33-7.19 (m, 3H), 7.12 (t, 1H), 6.34 (t, J = 54 Hz, 1H), 4.62 (d, 1 H), 4.55 (d, 1 H), 4.27 (s, 2H).

### k) (R)-5-Difluoromethyl-5-(2,3-difluoro-phenyl)-5,6-dihydro-2H-[1,4]oxazin-3-ylamine

A solution of (R)-5-difluoromethyl-5-(2,3-difluoro-phenyl)-morpholine-3-thione in a NH₃/MeOH solution (7 mol/L, 8.5 ml) was stirred in a sealed vessel for 4 h. The mixture was evaporated and chromatographed on silica gel (DCM/ (EtOH/sat aq NH₃ 9:1) 0-5%) to yield 517 mg of the title compound as a colorless resin. HPLC: Rt_{H2}= 2.249 min [M+H]⁺ 263;
¹H-NMR (CDCl₃, 400 MHz): δ 7.51 (t, 1H), 7.24-7.12 (m, 2H), 6.34 (t, J = 54 Hz, 1H), 4.38 (d, 1H), 4.35 (d, 1H), 4.19 (d, 1H), 4.03 (d, 1H).

### l) (R)-5-Difluoromethyl-5-(2,3-difluoro-5-nitro-phenyl)-5,6-dihydro-2H-[1,4]oxazin-3-ylamine

To a stirred solution of (R)-5-difluoromethyl-5-(2,3-difluoro-phenyl)-5,6-dihydro-2H-[1,4]oxazin-3-ylamine (508 mg, 1.937 mmol) in 5 ml H₂SO₄ was added KNO₃ (255 mg, 2.52 mmol) in four portions (exothermic). The resulting solution was stirred 20 minutes at rt and then poured on ice-water. The mixture was basified by addition of solid Na₂CO₃ (careful !: foaming) and extracted with EtOAc. The organic layer was washed with brine, treated with some charcoal and MgSO₄.H₂O and filtered over celite. Evaporation of the solvent gave the title compound, containing 6% of a regioisomer. The product was used without further purification.
HPLC: Rt_{H2}= 2.313 min [M+H]⁺ 308;
¹H-NMR (CDCl₃, 400 MHz): δ 8.65 (s, 1H), 8.10 (t, 1H), 6.10 (t, J = 54 Hz,1H), 4.52-3.98 (m, 4H).

### m) [(R)-5-Difluoromethyl-5-(2,3-difluoro-5-nitro-phenyl)-5,6-dihydro-2H-[1,4]oxazin-3-yl]-carbamic acid tert-butyl ester

To a solution of (R)-5-difluoromethyl-5-(2,3-difluoro-5-nitro-phenyl)-5,6-dihydro-2H-[1,4]oxazin-3-ylamine (510 mg, 1.660 mmol) in 5 ml DCM were added DIPEA (322 mg, 2.49 mmol) and di-tert-butyldicarbonate (417 mg, 2.158 mmol). The reaction mixture was stirred overnight at 40 °C. The reaction mixture was evaporated and the title compound was isolated as white crystals (TBME/hexanes). TLC (hexanes/ EtOAc 6:1): Rf = 0.25;
HPLC: Rt_{H3}= 3.475 min; ESIMS = [M+Na]⁺ 430;
¹H-NMR (CDCl₃, 400 MHz): δ 8.55-8.51 (m, 1H), 8.14-8.08 (m, 1H), 7.43 (br s, 1H), 6.04 (t, J = 54 Hz, 1H), 4.87 (d, 1H), 4.59 (d, 1H), 4.51 (dd, 1H), 3.95 (d, 1 H), 1.52 (s, 9H).

### n) [(R)-5-(5-Amino-2,3-difluoro-phenyl)-5-difluoromethyl-5,6-dihydro-2H-[1,4]oxazin-3-yl]-carbamic acid tert-butyl ester

A solution of [(R)-5-difluoromethyl-5-(2,3-difluoro-5-nitro-phenyl)-5,6-dihydro-2H-[1,4]oxazin-3-yl]-carbamic acid tert-butyl ester (540 mg, 1.326 mmol) in 3 ml EtOH and 2 ml THF was stirred in a hydrogen atmosphere in the presence of 140 mg 5% Pd-C "degussa" E101 ND till LC-MS analysis indicated complete conversion. The mixture was flushed with nitrogen, diluted with DCM and filtered over a pad of celite. The filtrate was evaporated and further purified by chromatography on silica gel (hexanes/ EtOAc 25-50%) to give the title compound as a colorless foam. TLC (hexanes/ EtOAc 2:1): Rf = 0.26;
HPLC: Rt_{H2}= 3.057 min; ESIMS = [M+H]⁺ 378;
¹H-NMR (CDCl₃, 400 MHz, broad signals due to rotamers): δ 6.73 (m, 1H), 6.50 (m, 1H), 6.18 (t, J = 54 Hz, 1H), 4.95-3.99 (m, 4H), 1.52 (s, 9H).

### o) ((R)-5-{5-[(5-Cyano-3-methyl-pyridine-2-carbonyl)-amino]-2,3-difluoro-phenyl}-5-difluoromethyl-5,6-dihydro-2H-[1,4]oxazin-3-yl)-carbamic acid tert-butyl ester

To an ice-cold solution of [(R)-5-(5-amino-2,3-difluoro-phenyl)-5-difluoromethyl-5,6-dihydro-2H-[1,4]oxazin-3-yl]-carbamic acid tert-butyl ester (113 mg, 0.299 mmol), 5-cyano-3-methyl-pyridine-2-carboxylic acid (53.4 mg, 0.329 mmol), HOAt (65.2 mg, 0.479 mmol) in 1.2 ml DMF were added 0.07 ml (0.39 mmol) EDC (free base). The mixture was stirred overnight at rt. Water and EtOAc were added and the organic layer was washed with sat aq NaHCO₃, brine and dried with MgSO₄.H₂O. The product was purified by chromatography on silica gel (hexanes/ EtOAc 15-20%) to give 108 mg of the title compound as colorless foam. TLC (hexanes/ EtOAc 3:1): Rf = 0.31;
HPLC: Rt_{H3}= 3.374 min; ESIMS = [M+H]⁺ 522;
¹H-NMR (CDCl₃, 400 MHz): δ 10.12 (s, 1 H), 8.76 (s, 1H), 8.20 (brt, 1H), 7.99 (s, 1H), 7.40 (br s, 1 H), 6.13 (t, J = 54 Hz, 1H), 4.85 (d, 1H), 4.62 (d, 1H), 4.43 (d, 1H), 4.40 (d, 1H), 2.89 (s, 3H), 1.52 (s, 9H).

### p) 5-Cyano-3-methyl-pyridine-2-carboxylic acid [3-((R)-5-amino-3-difluoromethyl-3,6-dihydro-2H-[1,4]oxazin-3-yl)-4,5-difluoro-phenyl]-amide

To a solution of ((R)-5-{5-[(5-cyano-3-methyl-pyridine-2-carbonyl)-amino]-2,3-difluorophenyl}-5-difluoromethyl-5,6-dihydro-2H-[1,4]oxazin-3-yl)-carbamic acid tert-butyl ester (107 mg, 0.205 mmol) in 0.9 ml DCM were added dropwise 0.3 ml TFA. The mixture was stirred 1.5 h at rt. The reaction mixture was carefully poured onto ca 10% aq. soda and EtOAc. The organic phase was washed with sat aq NaHCO₃ and brine, and dried with Na₂SO₄. The product was purified by chromatography on silica gel (DCM/ (EtOH/sat. aq. NH₃ 9:1) 0-2%) to give 81 mg of the title compound as white solid.
HPLC: Rt_{H2}= 2.827 min; ESIMS [M+H]⁺= 422;
¹H-NMR (DMSO-d6, 600 MHz): 10.91 (s, 1H), 8.40 (s, 1H) 8.98-8.94 (m, 1H), 7.82 (s, 1H), 6.19 (s, 2H), 6.13 (t, J = 54 Hz, 1 H), 4.08 (d, 1 H), 4.01 (d, 1 H), 3.95 (d, 1H), 3.89 (d, 1 H), 2.53 (s, 3H).

### Examples 15 to 16: The compounds listed in Table 2 were prepared by a procedure analogous to that used in Example 14.

Hydrochloride salts were obtained from solutions of the corresponding free base by addition of hydrochloric acid in dioxane or hydrochloric acid in diethylether and evaporation of the solvents.

**Table 2**

| **Example** | **Compound** | **¹H-NMR (δ; DMSO-d₆)** | **MS [m/z; (M+1)⁺]** |
|---|---|---|---|
| **15** | 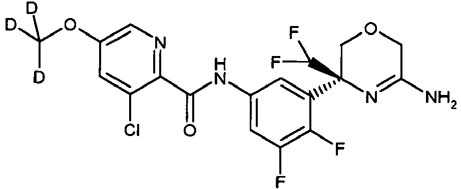 | 10.91 (s, 1H), 8.40 (s, 1H) 8.98-8.94 (m, 1H), 7.82 (s, 1H), 6.19 (s, 2H), 6.13 (t, J = 54 Hz, 1H), 4.08 (d, 1H), 4.01 (d, 1H), 3.95 (d, 1H), 3.89 (d, 1H), 2.53 (s, 3H). | 422 |
| | 3-Chloro-5-trideuteromethoxy-pyridine-2-carboxylic acid [3-((R)-5-amino-3-difluoromethyl-3,6-dihydro-2H-[1,4]oxazin-3-yl)-4,5-difluoro-phenyl]-amide | | |
| **16** | 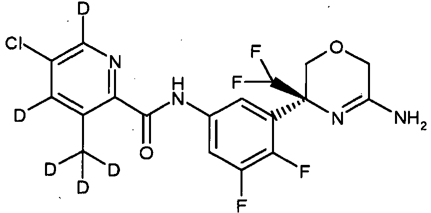 | 10.78 (s, 1H), 7.98-7.93 (m, 1H) 7.83 (s, 1H), 6.19 (s, 2H), 6.14 (t, J = 54 Hz, 1H), 4.10 (d, 1H), 4.02 (d, 1H), 3.91 (d, 1H), 3.88 (d, 1H). | 436 |
| | 4,6-Dideutero-5-chloro-3-trideuteromethyl-pyridine-2-carboxylic acid [3-((R)-5-amino-3-difluoromethyl-3,6-dihydro-2H-[1,4]oxazin-3-yl)-4,5-difluoro-phenyl]-amide | | |

### Example 17: 3-Chloro-5-cyano-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride

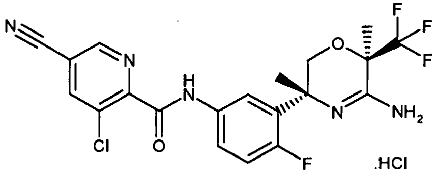

### a) 2-(5-Bromo-2-fluoro-phenyl)-propan-2-ol

To a solution of diisopropyl amine (57.3 ml, 402 mmol) in THF (500 ml) was added under argon a 1.6 M solution of nBuLi in hexane (260 ml, 416 mmol) below -50 °C. After stirring for 30 min at -75 °C, 4-bromo-1-fluoro benzene (31.1 ml, 277 mmol) was added while keeping the temperature below -70 °C. After stirring for 2 h at -75 °C, acetone (41.2 ml, 554 mmol) was added below -65 °C and the reaction mixture was stirred for 1 h at -75 °C, warmed up to -50 °C and poured onto 10% aqueous NH₄Cl solution. The mixture was extracted with TBME, organic phases were washed with aqueous KHSO₄ solution, saturated NaHCO₃ solution and brine, dried over MgSO₄, filtered and concentrated. The crude product was crystallized from hexane to provide the title compound as white crystals: TLC (hexane-EtOAc 3:1): Rf =0.45; HPLC: Rt_{H5}=1.045 min; ¹H-NMR (360 MHz, CDCl₃): δ 7.74 (dd, 1H), 7.36 (m, 1H), 6.93 (dd, 1 H), 2.04 (d, 1 H), 1.63 (s, 6H).

### b) 4-Bromo-1-fluoro-2-isopropenyl-benzene

To a solution of 2-(5-bromo-2-fluoro-phenyl)-propan-2-ol (119.7g, 498 mmol) in CH₂Cl₂ (50 ml) was added hydrochinone (2:74 g, 24.9 mmol) and 250 ml 85% H₃PO₄. The resulting reaction mixture was stirred for 3.5 h at 50 °C. The mixture was poured onto ice-water and extracted with CH₂Cl₂. The organic phases were washed with 2N aqueous NaOH and water, dried over MgSO₄, filtered and concentrated. The crude product was dissolved in hexane and filtered through a plough of silica gel to obtain after concentration at 600 mbar the title compound as a colorless oil: TLC (hexane): Rf =0.52; HPLC: Rt_{H5}=1.416 min; ¹H-NMR (360 MHz, CDCl₃): δ 7.43 (dd, 1 H), 7.37 (m, 1 H), 6.94 (dd, 1 H), 5.27 (d, 2H), 2.13 (s, 3H).

### c) (S)-2-(5-Bromo-2-fluoro-phenyl)-propane-1,2-diol

To a suspension of K₃Fe(CN)₆ (186 g, 561 mmol), K₂CO₃ (78 g, 561 mmol), (DHQ)₂-PHAL (1.311 g, 1.674 mmol) and K₂OsO₂(OH)₄ (0.378 g, 1 mmol) in t-BuOH-H₂O 1:1 (1600 ml) was added 4-bromo-1-fluoro-2-isopropenyl-benzene (36 g, 167 mmol) at 0 °C and the reaction mixture was stirred for 14 h at 0 °C. After careful addition of Na₂S₂O₅ (100 g) at 0-5 °C the reaction mixture was stirred for 1 h before extraction with EtOAc. Combined extracts were washed with 5% NaS₃O₃ solution and brine, dried over MgSO4, filtered and concentrated to give the title compound as a white solid: TLC (hexane-EtOAc 1:1): Rf =0.46; HPLC: Rt_{H5}=0.767 min; ¹H-NMR (360 MHz, CDCl₃): δ 7.71 (dd, 1H), 7.27 (m, 1H), 6.83 (dd, 1H), 3.85 (d, 1 H), 3.62 (d, 1 H), 2.94 (s, 3H), 2.01 (s, 1 H), 1.43 (s, 3H); ESI MS: 266, 268 [(M+NH₄)⁺].

### d) (S)-2-(5-Bromo-2-fluoro-phenyl)-2-methyl-oxirane

To a solution of (S)-2-(5-bromo-2-fluoro-phenyl)-propane-1,2-diol (37.35 g, 150 mmol) in CH₂Cl₂ (400 ml) was added under argon NEt₃ (41.8 ml, 300 mmol) and dropwise mesyl chloride (12.8 ml, 165 mmol) at 0-5 °C. After stirring for 0.5 h at 0-5 °C the reaction mixture was added to cold 1 N HCl and extracted with CH₂Cl₂. Combined extracts were washed with 1 N HCl, H₂O and saturated NaHCO₃ solution, dried over MgSO₄, filtered and concentrated. The crude mesylate was dissolved in TBME (500 ml) and 200 ml 2N aqueous NaOH and after stirring for 2 h at 25 °C the mixture was extracted with TBME. Combined extracts were washed with NaH₂PO₄ solution and brine, dried over MgSO₄, filtered and concentrated to provide the (S)-enantiomer as a colorless oil: 78% ee (Chiralpak AS-H 1218, hexane-EtOH 97:3, 0.4 mL/min); TLC (hexane-EtOAc 3:1): Rf=0.69; HPLC: Rt_{H5}= 1.186 min; ¹H-NMR (360 MHz, CDCl₃): δ 7.46 (dd, 1 H), 7.30 (m, 1 H), 6.83 (dd, 1 H), 2.88 (d, 1 H), 2.72 (d, 1 H), 1.59 (s, 3H).

### e) (S)-1-Azido-2-(5-bromo-2-fluoro-phenyl)-propan-2-ol

To a solution of (S)-2-(5-bromo-2-fluoro-phenyl)-2-methyl-oxirane (51.85 g, 224 mmol) in EtOH (800 ml) was added NaN₃ (36.8 g, 531 mmol), NH₄Cl (60.6 g, 1122 mmol) and 18-crown-6 (59.8 g, 224 mmol) and the reaction mixture was heated at reflux for 6 h. The reaction mixture was filtered and concentrated to half of its volume. The residual oil was extracted with EtOAc. Combined extracts were washed with saturated NaHCO₃ solution and brine, dried over MgSO₄, filtered and concentrated to provide the title compound as a light yellow oil: TLC (hexane-EtOAc 1:1): Rf=0.70; HPLC: Rt_{H3}= 1.115 min; ¹H-NMR (360 MHz, CDCl₃): δ 7.72 (dd, 1 H), 7.32 (m, 1 H), 6.85 (dd, 1H), 3.73 (d, 1H), 3.51 (d, 1 H), 2.44 (s, 1H), 1.50 (s, 3H).

### f) (S)-1-Amino-2-(5-bromo-2-fluoro-phenyl)-propan-2-ol

To a suspension of LiAlH₄ (4.65 g, 122 mmol) in THF (250 ml) was added under argon at 0-5 °C a solution of (S)-1-azido-2-(5-bromo-2-fluoro-phenyl)-propan-2-ol (33.4 g, 122 mmol) dissolved in THF (150 ml) over a period of 30 min. After stirring for 1 h at 0-5 °C, the reaction was quenched by careful addition of water (4.7 ml), 4 N NaOH (4.7 ml) and water (14.1 ml) and stirred again for 3 h at 25 °C. The white suspension was dried with MgSO₄, filtered and concentrated. The solidified product was re-crystallized from TBME-hexane to provide the title compound as beige crystals: 98% ee (Chiralpak AD-H hexane-EtOH 75-25 + 0.05% NEt₃); TLC (CH₂Cl₂-MeOH 10:1) Rf =0.10; HPLC: Rt_{H5}= 0.558 min; ESIMS: 248, 250 [(M+H)⁺]; ¹H-NMR (360 MHz, CDCl₃): δ 7.76 (dd, 1H), 7.25 (m, 1H), 6.82 (dd, 1H), 4.16 (br s, 1 H), 3.19 (d, 1H), 2.72 (d, 1 H), 1.44 (s, 3H), 0.95 (br s, 2H).

### g) N-[(S)-2-(5-Bromo-2-fluoro-phenyl)-2-hydroxy-propyl]-2-nitro-benzenesulfonamide

To a solution of (S)-1-amino-2-(5-bromo-2-fluoro-phenyl)-propan-2-ol (34.7 g, 140 mmol) in THF (400 ml) was added 2-nitro-benzenesulfonyl chloride (34.9 g, 154 mmol) at 0-5 °C and afterwards 1 N aqueous NaOH over a period of 0.5 h. The reaction mixture was stirred for 2 h at 20 °C. The reaction mixture was diluted with TBME and washed with water and NaH₂PO₄ solution and brine, dried over MgSO₄, filtered and concentrated to provide the title compound after crystallization from TBME-hexane as beige crystals: TLC (toluene-EtOAc 3:1): Rf =0.51; HPLC: Rt_{H5}= 1.118 min; ESIMS: 450, 452 [(M+NH₄)⁺]; ¹H-NMR (360 MHz, CDCl₃): δ 7.98 (m, 1 H), 7.81 (m, 1H), 7.65 (m, 2H), 7.59 (dd, 1H), 7.24 (m, 1H), 6.79 (dd, 1H), 5.60 (t, 1H), 4.16 (br s, 1 H), 3.55 (dd, 1 H), 3.44 (dd, 1 H), 2.51 (s, 1 H), 1.51 (s, 3H).

### h) (R)-2-(5-Bromo-2-fluoro-phenyl)-2-methyl-1-(2-nitro-benzenesulfonyl)-aziridine

To a solution of N-[(S)-2-(5-bromo-2-fluoro-phenyl)-2-hydroxy-propyl]-2-nitro-benzenesulfonamide (20.8 g, 48 mmol) in CH₂Cl₂ (400 ml) was added PPh₃ (19.2 g, 72.4 mmol) at 0-5 °C and diethyl azodicarboxylate (11.6 ml, 72.4 mmol). The reaction mixture was stirred for 24 h at 25 °C and concentrated. The title compound was obtained after chromatographic purification over silica gel (hexane-EtOAc 20:1 to 2:1) as yellow crystals: TLC (toluene-EtOAc 3:1): Rf=0.69; HPLC: Rt_{H5}= 1.308 min; ¹H-NMR (360 MHz, CDCl₃): δ 8.31 (m, 1H), 7.28 (m, 3H), 7.60 (dd, 1 H), 7.42 (m, 1 H), 6.91 (dd, 1 H), 3.24 (s, 1 H), 2.81 (s, 1 H), 2.06 (s, 3H).

### i) (R)-2-[(R)-2-(5-Bromo-2-fluoro-phenyl)-2-(2-nitro-benzenesulfonylamino)-propoxy]-3,3,3-trifluoro-2-methyl-propionic acid ethyl ester

To a suspension of NaH (2.53 g 60% in mineral oil, 63 mmol) in DMF (160 ml) was added drop-wise under argon (R)-3,3,3-trifluoro-2-hydroxy-2-methyl-propionic acid ethyl ester (11.99 g, 63 mmol) and after stirring for 0.5 h at 20 °C (R)-2-(5-bromo-2-fluoro-phenyl)-2-methyl-1-(2-nitro-benzenesulfonyl)-aziridine (21.85 g, 52.6 mmol). The reaction was kept at 25 °C for 16 h. The mixture was added to cold aqueous 2N HCl and the product extracted with TBME. Combined organic layers were washed with saturated NaHCO₃ solution and brine, dried over MgSO₄, filtered and concentrated. The residual solid was re-crystallized from TBME-hexane to provide the title compound as yellow crystals: TLC (hexane-EtOAc 1:1): Rf=0.59; HPLC: Rt_{H5}= 1.444 min; ESIMS: 618, 620 [(M+NH₄)⁺]; ¹H-NMR (360 MHz, CDCl₃): δ 7.83 (dd, 1 H), 7.61 (m, 3H), 7.48 (dd, 1 H), 7.27 (m, 1 H), 6.73 (s, 1 H), 6.60 (dd, 1 H), 4.33 (m, 2H), 3.84 (s, 2H), 1.84 (s, 3H), 1.57 (s, 3H), 1.33 (t, 3H).

### j) (R)-2-[(R)-2-(5-Bromo-2-fluoro-pheyl)-2-(2-nitro-benzenesulfonylamino)-propoxy]-3,3,3-trifluoro-2-methyl-propionamide

A solution of (R)-2-[(R)-2-(5-bromo-2-fluoro-phenyl)-2-(2-nitro-benzenesulfonylamino)-propoxy]-3,3,3-trifluoro-2-methyl-propionic acid ethyl ester (26.6 g, 44.2 mmol) in 7N NH₃ in MeOH (75 ml) was stirred for 16 h at 50 °C. The solvent was removed under reduced pressure and the residual solid re-crystallized from Et₂O to give the title compound as yellow crystals: TLC (hexane-EtOAc 1:1): Rf=0.35; HPLC: Rt_{H5}= 1.184 min; ESIMS: 589, 591 [(M+NH₄)⁺]; ¹H-NMR (360 MHz, CDCl₃): δ 7.85 (d, 1H), 7.64 (m, 3H), 7.44 (d, 1H), 7.41 (dd, 1H), 7.26 (m, 1H), 6.68 (br s, 1H), 6.57 (dd, 1H), 6.19 (s, 1H), 5.54 (br s, 1H), 4.24 (d, 1 H), 3.93 (d, 1H), 1.79 (s, 3H), 1.67 (s, 3H).

### k) N-[(R)-1-(5-Bromo-2-fluoro-phenyl)-2-((R)-1-cyano-2,2,2-trifluoro-1-methyl-ethoxy)-1-methyl-ethyl]-2-nitro-benzenesulfonamide

To a solution of (R)-2-[(R)-2-(5-bromo-2-fluoro-pheyl)-2-(2-nitro-benzenesulfonylamino)-propoxy]-3,3,3-trifluoro-2-methyl-propionamide (20.83 g, 35.6 mmol) in CH₂Cl₂ (300 ml) was added under argon NEt₃ (12.5 ml, 89 mmol) and at 0-5 °C trifluoroacetic anhydride (6.15 ml, 42.7 mmol). After stirring for 4 h at 25 °C the reaction mixture was added to a cold NaHCO₃ solution and the product was extracted with CH₂Cl₂. Combined extracts were washed with cold 0.1 N aqueous HCl, water and saturated NaHCO₃ solution, dried over MgSO₄, filtered and concentrated to provide the title compound as a yellow oil, which was used as such for the next step: TLC (hexane-EtOAc 1:1): Rf =0.73; HPLC: Rt_{H5}= 1.364 min; ESIMS: 571, 573 [(M+NH₄)⁺]; ¹H-NMR (360 MHz, CDCl₃): δ 7.89 (d, 1H), 7.62 (ddd, 1H), 7.57 (ddd, 1H), 7.52 (m, 2H), 7.29 (m, 1H), 6.58 (dd, 1H), 6.19 (s, 1H), 4.17 (s, 2H), 1.81 (s, 3H), 1.72 (s, 3H).

### l) (2R,5R)-5-(5-Bromo-2-fluoro-phenyl)-2,5-dimethyl-2-trifluoromethyl-5,6-dihydro-2H-[1,4]oxazin-3-ylamine

To a solution of N-[(R)-1-(5-bromo-2-fluoro-phenyl)-2-((R)-1-cyano-2,2,2-trifluoro-1-methyl-ethoxy)-1-methyl-ethyl]-2-nitro-benzenesulfonamide (6.54 g, 11.8 mmol) and N-acetylcysteine (2.4 g, 26.0 mmol) in MeOH (80 ml) was added K₂CO₃ (3.62 g, 26.0 mmol) and the reaction mixture was heated at 80 °C for 16 h. After removal of the solvent the residue was dissolved in water and extracted with EtOAc. Combined extracts were washed with saturated NaHCO₃ solution and brine, dried over MgSO₄, filtered and concentrated to provide the title compound after after chromatographic purification over silica gel (hexane-EtOAc 10:1 to 1:2 containing 0.03% NEt₃) as a yellow oil: TLC (hexane-EtOAc 1:1): Rf=0.58; HPLC: Rt_{H5}= 0.843 min; ESIMS: 369, 371 [(M+H)⁺]; ¹H-NMR (360 MHz, CDCl₃): δ 7.66 (dd, 1H), 7.35 (m, 1 H), 6.91 (dd, 1 H), 3.97 (m, 2H), 1.53 (s, 3H), 1.49 (s, 3H).

### m) (2R,5R)-5-(2-Fluoro-phenyl)-2,5-dimethyl-2-trifluoromethyl-5,6-dihydro-2H-[1,4]oxazin-3-ylamine

A solution of (2R,5R)-5-(5-bromo-2-fluoro-phenyl)-2,5-dimethyl-2-trifluoromethyl-5,6-dihydro-2H-[1,4]oxazin-3-ylamine (1.66 g, 4.5 mmol) and sodium acetate (0.369 g,4.5 mmol) in MeOH (50 ml) was hydrogenated over 10% Pd-C for 6 h at 50 °C. The catalyst was filtered off over Celite and the filtrate was concentrated. The residue was dissolved in saturated NaHCO₃ solution and extracted with EtOAc. Combined extracts were washed with brine, dried over MgSO₄, filtered and concentrated to provide the title compound as a colorless oil: TLC (hexane-EtOAc 1:1): Rf =0.19; HPLC: Rt_{H5}= 0.777 min; ESIMS: 291 [(M+H)⁺]; ¹H-NMR (360 MHz, CDCl₃): δ 7.41 (dt, 1 H), 7.26 (m, 1H), 7.11 (t, 1 H), 7.05 (dd, 1H), 4.11 (dd, 1H), 3.94 (dd, 1 H), 1.54 (s, 3H), 1.49 (s, 3H).

### n) (2R,5R)-5-(2-Fluoro-5-nitro-phenyl)-2,5-dimethyl-2-trifluoromethyl-5,6-dihydro-2H-[1,4]oxazin-3-ylamine

To a solution of (2R,5R)-5-(2-fluoro-phenyl)-2,5-dimethyl-2-trifluoromethyl-5,6-dihydro-2H-[1,4]oxazin-3-ylamine (1.035 g, 3.57 mmol) in H₂SO₄ (6 ml) was added in portions KNO₃ (0.379 g, 3.74 mmol) under ice-water cooling. The reaction mixture was stirred for 2 h at 25 °C, diluted with water and basified with K₂CO₃ under cooling. The product was extracted with EtOAc. Combined extracts were washed with saturated NaHCO₃ solution and brine, dried over MgSO₄, filtered and concentrated. Purification via chromatography on silica gel (hexane-EtOAc 4:1 to 1:1 containing 0.05% NEt₃) gave the title compound as a light yellow oil: TLC (hexane-EtOAc 1:1): Rf =0.50; HPLC: Rt_{H5}= 0.749 min; ESIMS: 336 [(M+H)⁺]; ¹H-NMR (360 MHz, CDCl₃): δ 8.48 (dd, 1H), 8.14 (m, 1H), 7.15 (dd, 1 H), 4.20 (br s, 2H), 4.04 (dd, 1 H), 3.91 (dd, 1 H), 1.54 (s, 3H), 1.49 (s, 3H).

### o) [(2R,5R)-5-(2-Fluoro-5-nitro-phenyl)-2,5-dimethyl-2-trifluoromethyl-5,6-dihydro-2H-[1,4]oxazin-3-yl]-carbamic acid tert-butyl ester

To a solution of (2R,5R)-5-(2-fluoro-5-nitro-phenyl)-2,5-dimethyl-2-trifluoromethyl-5,6-dihydro-2H-[1,4]oxazin-3-ylamine (1.14 g, 3.4 mmol) in ACN (20 ml) was added Boc₂O (0.891 g, 4.08 mmol) and NEt₃ (0.72 ml, 5.1 mmol) and the mixture was stirred for 16 h at 25 °C. The reaction mixture was evaporated and the residual oil purified by chromatography on silica gel (hexane-EtOAc 20:1 to 7:3) to give the title compound after crystallization from Et₂O-hexane as beige crystals: TLC (hexane-EtOAc 3:1): Rf =0.37; HPLC: Rt_{H5}= 1.355 min; ESIMS: 436 [(M+H)⁺]; ¹H-NMR (360 MHz, CDCl₃): δ 11.04 (br s, 1H), 8.24 (m, 2H), 7.30 (dd, 1H), 4.41 (dd, 1H), 4.11 (dd, 1H), 1.68 (s, 3H), 1.51 (s, 9H), 1.49 (s, 3H).

### p) [(2R,5R)-5-(5-Amino-2-fluoro-phenyl)-2,5-dimethyl-2-trifluoromethyl-5,6-dihydro-2H-[1,4]oxazin-3-yl]-carbamic acid tert-butyl ester

A solution of [(2R,5R)-5-(2-fluoro-5-nitro-phenyl)-2,5-dimethyl-2-trifluoromethyl-5,6-dihydro-2H-[1,4]oxazin-3-yl]-carbamic acid tert-butyl ester (0.98 g, 2.25 mmol) in isopropanol-THF 2:1 (24 ml) was hydrogenated over 5% Pd-C for 4 h at 50 °C. The catalyst was filtered off over Celite and the filtrate was concentrated to provide the title compound after crystallization from TBME-hexane as beige crystals: TLC (hexane-EtOAc 1:1): Rf =0.42; HPLC: Rt_{H5}= 0.955 min; ESIMS: 406 [(M+H)⁺]; ¹H-NMR (360 MHz, CDCl₃): δ 6.82 (dd, 1H), 6.52 (m, 2H), 4.30 (dd, 1H), 3.97 (dd, 1H), 3.06 (br s, 2H), 1.58 (s, 3H), 1.48 (s, 3H), 1.46 (s, 9H).

### q) ((2R,5R)-5-{5-[(3-Chloro-5-cyano-pyridine-2-carbonyl)-amino]-2-fluoro-phenyl}-2,5-dimethyl-2-trifluoromethyl-5,6-dihydro-2H-[1,4]oxazin-3-yl)-carbamic acid tert-butyl ester

To a solution of [(2R,5R)-5-(5-amino-2-fluoro-phenyl)-2,5-dimethyl-2-trifluoromethyl-5,6-dihydro-2H-[1,4]oxazin-3-yl]-carbamic acid tert-butyl ester (82 mg, 0.20 mmol) in DMF (2 ml) was added 3-chloro-5-cyano-pyridine-2-carboxylic acid (47 mg, 0.26 mmol), EDC-HCl (57 mg, 0.30 mmol), HOAt (41 mg, 0.30 mmol) and DIPEA (0.14 ml, 0.79 mmol) and the reaction mixture was kept at 25 °C for 16 h. The reaction mixture was concentrated under reduced pressure, the residue dissolved in EtOAc and washed with saturated NaHCO₃ solution and brine, dried over MgSO₄, filtered and concentrated. The title compound was obtained after purification by flash column chromatography on silica gel (hexane-EtOAc 20:1 to 1:1) as a light yellow foam. TLC (hexane-EtOAc 2:1): Rf =0.29; HPLC: Rt_{H5}= 1.398 min; ESIMS: 570, 572 [(M+H)⁺]; ¹H-NMR (360 MHz, CDCl₃): δ 11.05 (br s, 1H), 9.74 (br s, 1H), 8.79 (s, 1H), 8.19 (s, 1 H), 7.87 (m, 1H), 7.55 (dd, 1H), 7.16 (dd, 1H), 4.43 (d, 1H), 4.09 (d, 1H), 1.71 (s, 3H), 1.57 (s, 3H), 1.56 (m, 9H); ¹⁹F-NMR (360 MHz, CDCl₃): δ 74.3, 116.2.

### r) 3-Chloro-5-cyano-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride

To a solution of ((2R,5R)-5-{5-[(3-Chloro-5-cyano-pyridine-2-carbonyl)-amino]-2-fluorophenyl}-2,5-dimethyl-2-trifluoromethyl-5,6-dihydro-2H-[1,4]oxazin-3-yl)-carbamic acid tert-butyl ester (105 mg, 0.166 mmol) in CH₂Cl₂ (1 ml) was added TFA (0.3 ml) and the reaction mixture was kept at 25 °C for 2 h. The reaction was added to cold 10% aq. K₂CO₃ solution and the product was extracted with EtOAc. Combined organic extracts were washed with brine, dried over MgSO₄, filtered and concentrated to provide 3-chloro-5-cyano-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide as a colorless foam. The title compound was converted into its hydrochloride salt by dissolving the free base in CH₂Cl₂, adding 1.05 equivalent of 2N HCl in Et₂O, evaporation to dryness, followed by crystallization from CH₂Cl₂-Et₂O to provide the title compound as a white solid: TLC (CH₂Cl₂-MeOH 9:1): Rf =0.51; HPLC: Rt_{H5}= 0.939 min; ESIMS: 470, 472 [(M+H)⁺]; ¹H-NMR (600 MHz, DMSO-d₆): δ 11.59 (s, 1H), 11.15 (s, 1H), 9.60 (d, 2H), 9.13 (s, 1H), 8.84 (s, 1H), 7.83 (m, 1H), 7.78 (dd, 1H), 7.36 (dd, 1H), 4.32 (d, 1H), 4.09 (d, 1H), 1.73 (s, 3H), 1.72 (s, 3H); ¹⁹F-NMR (360 MHz, CDCl₃): δ 76.4, 116.4.

### Examples 18 to 36: The compounds listed in Table 3 were prepared by a procedure analogous to that used in Example 17.

**Table 3**

| **Example** | **Compound** | **¹H-NMR (δ; DMSO-d₆)** | **MS [m/z;** (**M**+**1**)**⁺**] |
|---|---|---|---|
| **18** | 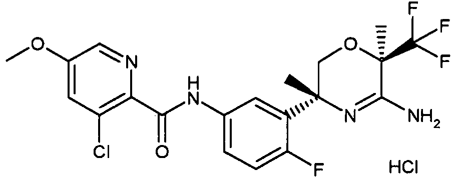 | 11.75 (s, 1H), 10.80 (s, 1H), 9.65 (d, 2H), 8.36 (d, 1H), 7.90 (m, 1H), 7.82 (dd, 1H), 7.75 (d, 1H), 7.32 (dd, 1H), 4.32 (d, 1H), 4.07 (d, 1H), 3.94 (s, 3H), 1.74 (s, 3H), 1.72 (s, 3H). | 475,477 |
| | 3-Chloro-5-methoxy-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride | | |
| **19** | 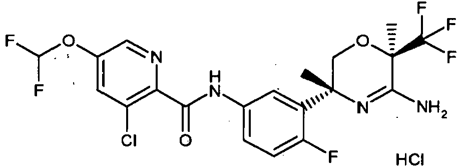 | 11.59 (s, 1H), 10.95 (s, 1H), 9.59 (d, 2H), 8.60 (d, 1H), 8.15 (d, 1H), 7.85 (m, 1H), 7.80 (d, 1H), 7.51, (t, 1H), 7.35 (dd, 1H), 4.32 (d, 1H), 4.08 (d, 1H), 1.74 (s, 3H), 1.72 (s, 3H). | 511,513 |
| | 3-Chloro-5-difluoromethoxy-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride | | |
| **20** | 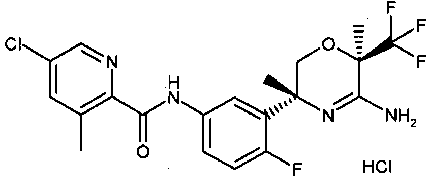 | 11.55 (s, 1H), 10.84 (s, 1H), 9.55 (d, 2H), 8.61 (d, 1H), 8.06 (d, 1H), 7.96 (m, 1H), 7.87 (d, 1H), 7.33 (dd, 1H), 4.32 (d, 1H), 4.08 (d, 1H), 2.57 (s, 3H), 1.75 (s, 3H), 1.72 (s, 3H). | 459, 461 |
| | 5-Chloro-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride | | |
| **21** | 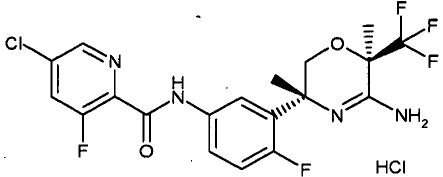 | 11.62 (s, 1H), 10.98 (s, 1H), 9.59 (d, 2H), 8.69 (s, 1H), 8.36 (d, 1H), 7.93 (m, 1H), 7.88 (d, 1H), 7.35 (dd, 1H), 4.33 (d, 1H), 4.08 (d, 1H), 1.75 (s, 3H), 1.72 (s, 3H). | 463,465 |
| | 5-Chloro-3-fluoro-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride | | |
| **22** | 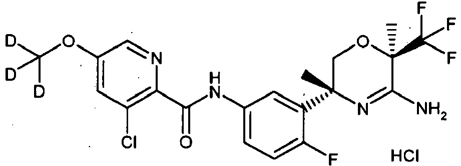 | 11.61 (s, 1H), 10.80 (s, 1H), 9.59 (d, 2H), 8.37 (d, 1H), 7.88 (m, 1H), 7.83 (m, 1H), 7.76 (d, 1H), 7.33 (dd, 1H), 4.32 (d, 1H), 4.08 (d, 1H), 1.74 (s, 3H), 1.72 (s, 3H). | 478,480 |
| | 3-Chloro-5-trideutero-methoxy-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride | | |
| **23** | 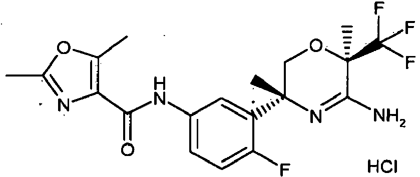 | 11.59 (s, 1H), 10.31 (s, 1H), 9.55 (d, 2H), 7.96 (m, 1H), 7.86 (dd, 1H), 7.27 (dd, 1H), 4.30 (d, 1H), 4.05 (d, 1H), 2.58 (s, 3H), 2.46 (s, 3H), 1.75 (s, 3H), 1.70 (s, 3H). | 429 |
| | 2,5-Dimethyl-oxazole-4-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride | | |
| **24** | 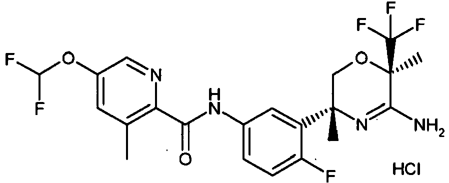 | 11.53 (br s, 1H), 10.78 (s, 1H), 9.54 (br s, 2H), 8.45 (d, 1H), 8.00-7.94 (m, 1H), 7.88 (dd, 1H), 7.75 (d, 1H), 7.45 (t, 1H), 7.32 (dd, 1H), 4.32 (d, 1H), 4.08 (d, 1H), 2.60 (s, 3H), 1.75 (s, 3H), 1.72 (s, 3H). | 491 |
| | 5-Difluoromethoxy-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride | | |
| **25** | 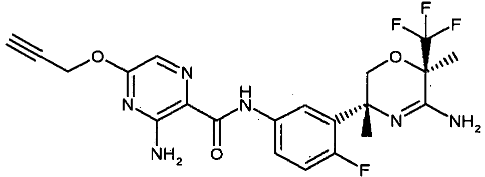 | 10.20 (s, 1H), 8.05 (br. s, 1H), 7.85 (dd, 1H), 7.70-7.61 (m, 1H), 7.57 (s, 1H), 7.52 (br. s, 1H), 7.11 (dd, 1H), 6.07 (br. s, 2H), 5.01 (d, 2H), 3.94-3.84 (m, 1H), 3.80 (d, 1H), 3.63 (s, 1H), 1.48 (s, 3H), 1.40 (s, 3H). | 481 |
| | 3-Amino-5-prop-2-ynyloxy-pyrazine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide | | |
| **26** | 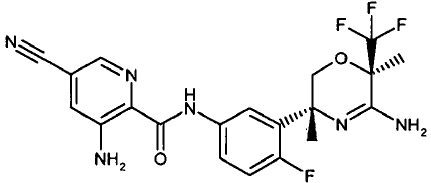 | 10.54 (s, 1H), 8.21 (d, 1H), 7.85 (dd, 1H), 7.70 (d, 1H), 7.64 (d, 1H), 7.25 (br. s, 2H), 7.13 (dd, 1H), 6.06 (br. s, 2H), 3.87 (d, 1H), 3.82 (d, 1H), 1.48 (s, 3H), 1.40 (s, 3H). | 451 |
| | 3-Amino-5-cyano-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide | | |
| **27** | 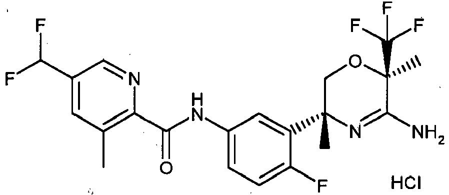 | 11.57 (br s, 1H), 10.88 (s, 1H), 9.56 (br s, 2H), 8.74 (s, 1H), 8.07 (s, 1H), 8.00-7.93 (m, 1H), 7.88 (dd, 1H), 7.40-7.08 (m, 2H), 4.32 (d, 1H), 4.08 (d, 1H), 2.60 (s, 3H), 1.75 (s, | 475 |
| | 5-Difluoromethyl-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride | | |
| **28** | 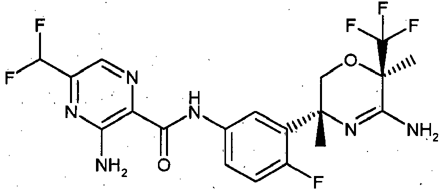 | 10.68 (s, 1H), 8.13 (s, 1H), 7.98-7.81 (m, 3H), 7.67 (d, 1H), 7.14 (dd, 1H), 6.95 (t, 1H, CHF2), 6.07 (br. s, 2H), 3.89 (d, 1H), 3.80 (d, 1H), 1.48 (s, 3H), 1.40 (s, 3H). | 477 |
| | 3-Amino-5-difluoromethyl-pyrazine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide | | |
| **29** | 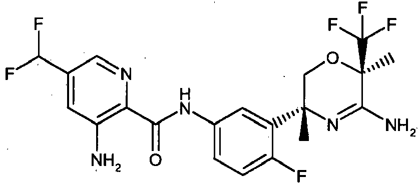 | 10.48 (s, 1H), 8.03 (s, 1H), 7.86 (dd, 1H), 7.71 (d, 1 H), 7.40 (s, 1H), 7.19-7.11 (m, 3 H), 7.12 (t, 1H, CHF2), 6.06 (br. s, 2H), 3.93-3.74 (m, 2H), 1.49 (s, 3H), 1.41 (s, 3H). | 476 |
| | 3-Amino-5-difluoromethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide | | |
| **30** | 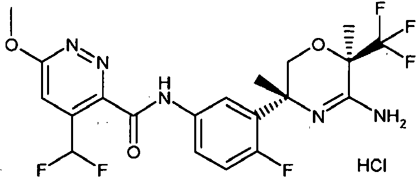 | 11.53 (s, 1H), 11.31 (s, 1H), 9.55 (s, 2H), 8.03 (d, 1H), 7.91 (dd, 1H), 7.66 (s, 1H), 7.61 (t, 1H), 7.36 (dd, 1H), 4.32 (d, 1H), 4.20 (s, 3H), 4.08 (d, 1H), 1.75 (s, 3H), 1.7 (s, 3H). | 492 |
| | 4-Difluoromethyl-6-methoxy-pyridazine-3-carboxylic acid [3-((3R,6R)-5-amino-3,6-dimethyl-6-trifluoromethyl-3,6-dihydro-2H-[1,4]oxazin-3-yl)-4-fluoro-phenyl]-amide hydrochloride | | |
| **31** | 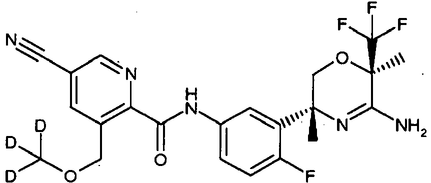 | 10.8 (s, 1H, NH), 9.08 (s, 1H), 8.47 (s, 1 H), 7.81 (m, 1H), 7.73 (s broad, 1H), 7.16 (triplettoid, 1H), 6.08 (s broad, 2H, NH₂ amidine), 4.81 (s, 2H), 3.90 (d, 1H, AB), 3.80 (d, 1H, AB), 1.45 (s, 3H), 1.40 (s, 3H). | 483 |
| | 5-Cyano-3-trideuteromethoxymethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide | | |
| **32** | 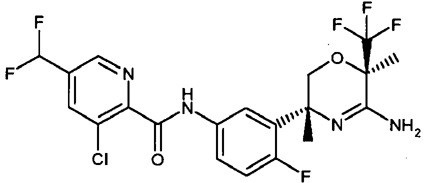 | 10.83 (s, 1H), 8.86 (s, 1 H), 8.40 (s, 1H), 7.74 - 7.70 (m, 2H), 7.25 (t, 1H, CHF2), 7.20 - 7.16 (m, 1H), 6.11 (br. s, 2H), 3.95 - 3.93 (m, 1H), 3.80-3.78 (m, 1H), 1.47 (s, 3 H), 1.43 (s, 3H). | 495 |
| | 3-Chloro-5-difluormethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide | | |
| **33** | 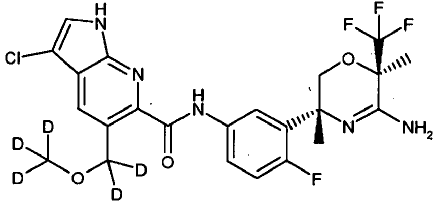 | 12.3 (s, 1H, NH), 11.65 (s, 1H, ⁺N-H, amidine), 10.72 (s, 1H, NH, amide), 9.82 (s, NH, amidine), 9.48 (s, 1H, NH, amidine), 8.12 (s, 1H), 7.90 (m, 3H), 7.31 (dd, 1H), 4.32 (d, 1H, AB), 4.08 (d, 1H, AB), 1.75 (s, 3H), 1.72 (s, 3H). | 533 |
| | 3-Chloro-5-trideuteromethoxy-dideuteromethyl-1 H-pyrrolo[2,3-b]pyridine-6-carboxylic acid [3-((3R,6R)-5-amino-3,6-dimethyl-6-trifluoromethyl-3,6-dihydro-2H-[1,4]oxazin-3-yl)-4-fluoro-phenyl]-amide | | |
| **34** | 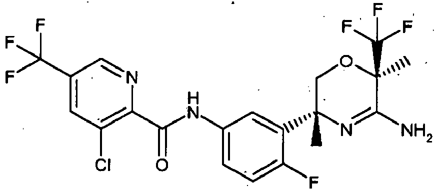 | 10.88 (s, 1H), 9.08 (s, 1H), 8.72 (s, 1H), 7.71 (dd, 2H), 7.21 - 7.17 (m, 1H), 6.11 (br. s, 2H), 3.94 (d, 1H), 3.79 (d, 1H), 1.46 (s, 3H), 1.42 (s, 3H). | 513 |
| | 3-Chloro-5-trifluoromethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide | | |
| **35** | 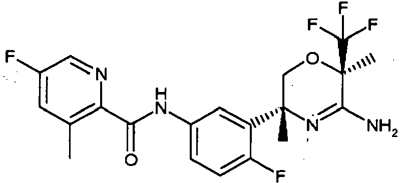 | 10.50 (br s, 1H), 8.53 (d, 1H), 7.84 - 7.78 (m, 2H), 7.73 (br s, 1H), 7.14 (dd, 1H), 6.04 (br. s, 2H), 3.91 (d, 1H), 3.80 (d, 1H), 2.57 (s, 3H), 1.48 (br s, 3H), 1.42 (br s, 3H) | 443 |
| | 5-Fluoro-3-methyl-pyridine-2-carboxylic acid [3-((3R,6R)-5-amino-3,6-dimethyl-6-trifluoro-methyl-3,6-dihydro-2H-[1,4]oxazin-3-yl)-4-fluoro-phenyl]-amide | | |
| **36** | 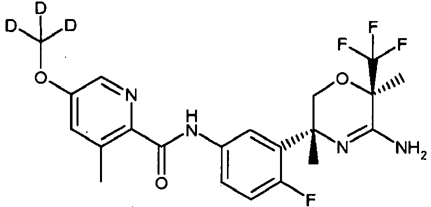 | 10.37 (br s, 1H), 8.22 (d, 1H), 7.81 (dd, 1H), 7.78-7.71 (m, 1H), 7.40 (d, 1H), 7.12 (dd, 1H), 6.04 (br. s, 2H), 3.89 (d, 1H), 3.82 (d, 1H), 2.61 (s, 3H), 1.49 (br s, 3H), 1.42 (br s, 3H) | 458 |
| | 5-Trideuteromethoxy-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide | | |

### Example 37: 5-Cyano-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide

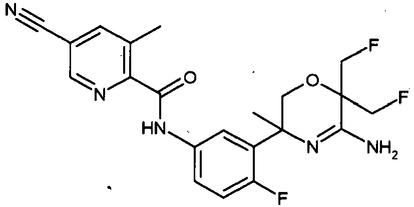

### a) 3-Fluoro-2-fluoromethyl-2-trimethylsilanyloxy-propionitrile

To 1,3-Difluoro-propan-2-one (8.5 g, 90 mmol) was added drop wise over 30 min TMS-Cyanide (8.97 g, 90 mmol). The reaction mixture was stirred for 16 h at ambient temperature. ¹H-NMR (400 MHz, CDCl₃): δ 4.55 (d, 2 H), 4.44 (d, 2 H), 0.28 (s, 9H);
¹⁹F-NMR (376 MHz, CDCl₃): δ - 226 (t).

### b) 3-Fluoro-2-fluoromethyl-2-hydroxy-propionic acid

3-Fluoro-2-fluoromethyl-2-trimethylsilanyloxy-propionitrile (17.4 g, 90 mmol) was treated with 37% HCl (300 ml) and heated to gentle reflux for 3 h. The reaction mixture was cooled to ambient temperature and concentrated in vacuo. The solid thus obtained was redisolved in 300 ml Ethanol and concentrated in vacuo and dried in high vaccum.
The solid thus obtained (17 g) contained significant amount of Ammonium-Chloride and was used without further purification.
¹H-NMR (400 MHz, DMSO-D₆): δ 7.3 - 7.0 (m, 4H), 6.5 - 5.6 (s, 1 H), 4.58 - 4.43 (m, 4 H). ¹³C-NMR (150 MHz, DMSO-D₆): δ 171 (t), 85 (d), 83 (d), 75 (t).

### c) 3-Fluoro-2-fluoromethyl-2-hydroxy-propionic acid ethyl ester

Crude 3-Fluoro-2-fluoromethyl-2-hydroxy-propionic acid (17 g) was dissolved in Ethanol (400 ml) and H₂SO₄ (98%, 30 g) was added. The reaction mixture was refluxed for 16 h.
The reaction mixture was cooled to ambient temperature and filtered. The solution was carefully treated with 30 g solid Na₂CO₃ and the resulting mixture was stirred for 30 min at room temperature. 400 ml DCM were added and the mixture was filtered. The solution was concentrated (50°C, 150 mbar) and further purified by distillation (82°C, 20 mbar) to give a colorless liquid.
¹H-NMR (400 MHz, DMSO-D₆): δ 4.65 - 4.43 (m, 4H), 4.30 (q, 2H), 3.88 - 3.63 (s,1H), 1.30 (t, 3H).

### d) 2-[2-(5-Bromo-2-fluoro-phenyl)-2-(2-nitro-benzenesulfonylamino)-propoxy]-3-fluoro-2-fluoromethyl-propionic acid ethyl ester

To a suspension of NaH (1.62 g, 60%, 40.5 mmol) in 75 ml DMF was added 3-Fluoro-2-fluoromethyl-2-hydroxy-propionic acid ethyl ester (6.8 g, 40.5 mmol). The reaction mixture was stirred at ambient temperature for 30 min and then rac. 2-(5-Bromo-2-fluoro-phenyl)-2-methyl-1-(2-nitro-benzenesulfonyl)-aziridine (14 g, 33.7 mmol, analogous to example 17 step a-h)) was added. The reaction mixture was stirred at ambient temperature for 2 days.
The reaction mixture was added to a cold solution of 2N aq. HCl (250 ml) and the product was extracted with 2 x 250 ml EtOAc., washed with NaHCO₃ solution (250 ml) and brine (250 ml). The organic layer was dried over MgSO₄ and concentrated under reduced pressure to obtain an off-white solid which was titruated with cold Methanol.
HPLC: Rt_{H3}= 1.26 min; ESIMS [M+H₃O]⁺= 600, 602;
¹H-NMR (400 MHz, DMSO): δ 8.40 (s, 1 H), 7.89 (d, 1 H), 7.85 - 7.60 (m, 3H), 7.45 (d, 1 H), 6.91 (dd, 1 H), 4.85 - 4.45 (m, 4H), 4.20 (q, 2H), 4.00 (d, 1 H), 3.81 (d, 1 H), 1.61 (s, 3H), 1.20 (t, 3H).

### e) 2-[2-(5-Bromo-2-fluoro-phenyl)-2-(2-nitro-benzenesulfonylamino)-propoxy]-3-fluoro-2-fluoromethyl-propionamide

2-[2-(5-Bromo-2-fluoro-phenyl)-2-(2-nitro-benzenesulfonylamino)-propoxy]-3-fluoro-2-fluoromethyl-propionic acid ethyl ester (10 g, 17.14 mmol) was dissolved in 7N NH₃ in MeOH (40 ml) and the yellow reaction mixture was stirred at 50 - 55 °C for 16 h in a sealed vial. The reaction mixture was concentrated under reduced pressure to obtain a pale yellow solid. HPLC: Rt_{H3}= 1.05 min; ESIMS [M+H₃O]⁺= 571, 573;
¹H-NMR (400 MHz, DMSO): δ 8.85 - 8.65 (s, 1H), 7.95 - 7.40 (m, 6H), 6.95 (m, 1H), 4.63 (d, 4H), 3.88 (m, 2H), 1.56 (s, 3H).

### f) N-[1-(5-Bromo-2-fluoro-phenyl)-2-(cyano-bis-fluoromethyl-methoxy)-1-methyl-ethyl]-2-nitro-benzenesulfonamide

2-[2-(5-Bromo-2-fluoro-phenyl)-2-(2-nitro-benzenesulfonylamino)-propoxy]-3-fluoro-2-fluoromethyl-propionamide (5 g, 9 mmol) was suspended in 150 ml dry DCM. N-Methyl-morpholine (2.5 ml) was added. TFAA (2.3 g, 10.8 mmol) was added in 20 ml DCM dropwise over 5 min. The reaction mixture was stirred at ambient temperature for 40 min. N-Methyl-morpholine (2.5 ml) was added. TFAA (2.3 g, 10.8 mmol) was added in 20 ml DCM dropwise over 5 min.
The reaction mixture was added to a cold saturated aqueous solution of NaHCO₃ (400 ml) and the mixture was stirred for 5 min at RT. The phases were separated and the aqueous was extracted 2x with DCM (100 ml). The Combined organic phases were washed with cold 0.1 N HCl (100 ml), water (100 ml) and sat. NaHCO₃ solution (100 ml), dried over MgSO4, filtered and concentrated.
HPLC: Rt_{H3}= 1.17 min; ESIMS [M+H₃O]⁺= 553, 555;
¹H-NMR (400 MHz, CDCl₃): δ 7.89 (d, 1 H), 7.70 - 7.47 (m, 4H), 7.31 (d, 1H), 6.59 (dd, 1H), 6.21 (s, 1 H), 4.67 (m, 2H), 4.56 (m, 2H), 4.25 (d, 1 H), 4.17 (d, 1 H), 1.83 (s, 3H).

### g) 5-(5-Bromo-2-fluoro-phenyl)-2,2-bis-fluoromethyl-5-methyl-5,6-dihydro-2H-[1,4]oxazin-3-ylamine

A suspension of N₋[1-(5-Bromo-2-fluoro-phenyl)-2-(cyano-bis-fluoromethyl-methoxy)-1-methyl-ethyl]-2-nitro-benzenesulfonamide (5 g, 9.32 mmol), K2CO3 (2.83 g, 20.51 mmol) and 2-Acetylamino-3-mercapto-propionic acid (3.8 g, 23.31 mmol) in EtOH (100 ml) was refluxed for 16 h. The reaction mixture was cooled to RT and filtered. The solution was concentrated to obtain a yellow solid foam.
The solid foam was suspended in 10% Na₂CO₃ solution (50 ml) and was extracted with EtOAc (3x 200 ml). The combined organic layers were washed with 10% aq. Na2CO3 solution (50 ml), 1 M NaOH (50 ml) and brine (50 ml). The solution was dried over MgSO4, filtered and evaporated.
HPLC: Rt_{H3}= 1.17 min; ESIMS [M+H]⁺= 351, 353;
¹H-NMR (400 MHz, CDCl₃): δ 7.68 (dd, 1H), 7.33 (m, 1H), 6.89 (dd, 1H), 4.75 - 4.39 (m, 4H), 3.96 (d, 1H), 3.87 (d, 1H), 1.51 (s, 3H).

### h) [5-(5-Bromo-2-fluoro-phenyl)-2,2-bis-fluoromethyl-5-methyl-5,6-dihydro-2H-[1,4]oxazin-3-yl]-carbamic acid tert-butyl ester

5-(5-Bromo-2-fluoro-phenyl)-2,2-bis-fluoromethyl-5-methyl-5,6-dihydro-2H-[1,4]oxazin-3-ylamine (3.3 g, 9.4 mmol) was dissolved in 100 ml DCM. Boc-Anhydride (2.46 g, 11.48 mmol) was added and the reaction mixture was stirred at ambient temperature for 16h. The reaction mixture was treated with 10% aqueous Citric acid (50 ml) solution and stirred for 5 min at RT. The phases were separated and the organic layers were washed with NaHCO₃ solution (25 ml) and brine (25 ml). The solution was dried over MgSO4, filtered and evaporated. The crude product was purified via silica-gel chromatography to provide the title compound as a white crystalline solid. TLC (Hexane / EtOAc 9:1): Rf=0.27;
HPLC: Rt_{H3}= 1.27 min; ESIMS [M+H]⁺= 451, 453;
¹H-NMR (400 MHz, CDCl₃): δ 10.98 - 10.94 (s, 1H), 7.43 (m, 2H), 6.98 (m, 1 H), 5.04 - 4.88 (dd, 1 H), 4.77 - 4.70 (m, 1 H), 4.63 - 4.43 (m, 3H), 4.06 (d, 1 H), 1.67 (s, 3H), 1.53 (s, 9H).

### i) [5-(5-Amino-2-fluoro-phenyl)-2,2-bis-fluoromethyl-5-methyl-5,6-dihydro-2H-[1,4]oxazin-3-yl]-carbamic acid tert-butyl ester

[5-(5-Bromo-2-fluoro-phenyl)-2,2-bis-fluoromethyl-5-methyl-5,6-dihydro-2H-[1,4]oxazin-3-yl]-carbamic acid tert-butyl ester (2.27 g, 5.03 mmol), rac-trans-N,N-Dimethylcyclohexane-1,2-diamine (715 mg, 5.03 mmol), Sodium ascorbate (400 mg, 2mmol), NaN₃ (2.62, 40.2 mmol) were suspended in in EtOH (100 ml) and H₂O (43 ml). The reaction mixture was degassed and CuI (383 mg, 2mmol) were added under N₂. The reaction mixture was stirred at 70 °C for 45 min. The reaction mixture was cooled to RT and water (100 ml) and EtOAc (200 ml) were added. The phases were separated and the aqueous phase was extracted with EtOAc (200 ml). The combined organic phases were washed with water (250 ml), 5% aqueous Ammonia (250 ml) and brine (250 ml). The organic layer was dried over anhydrous Na₂SO₄ and the organic layer was concentrated under reduced pressure. The solid obtained was dissolved in Ethanol (50 ml) and Pd/C 5% (350 mg, E101 N/D Degussa) was added. The reaction mixture was degassed and hydrogenated at 1.1 bar for 1 h at ambient temperature. The reaction mixture was filtered and concentrated. The crude product was purified via silica-gel chromatography (gradient: Hexane/EtOAc 6% → Hexane/EtOAc 48%) to provide the title compound as a white crystalline solid: TLC (Hexane / EtOAc 2:1): Rf=0.66;
HPLC: Rt_{H3}= 1.07 min; ESIMS [M+H]⁺= 388;
¹H-NMR (400 MHz, DMSO): δ 10.75 - 10.72 (s, 1 H), 6.90 (m, 1 H), 6.50 (m, 2H), 5.10 - 5.03 (s, 1 H), 5.04 - 4.40 (m, 4H), 4.32 - 4.05 (dd, 2H), 1.60 (s, 3H), 1.42 (s, 9H).

### j) (5-{5-[(5-Cyano-3-methyl-pyridine-2-carbonyl)-amino]-2-fluoro-phenyl}-2,2-bis-fluoromethyl-5-methyl-5,6-dihydro-2H-[1,4]oxazin-3-yl)-carbamic acid tert-butyl ester

[5-(5-Amino-2-fluoro-phenyl)-2,2-bis-fluoromethyl-5-methyl-5,6-dihydro-2H-[1,4]oxazin-3-yl]-carbamic acid tert-butyl ester (400 mg, 1.03 mmol), 5-cyano-3-methyl-pyridine-2-carboxylic acid (201 mg, 1.24 mmol), HOAT (215, 1.55 mmol) and N-Methyl-morpholine (209 mg, 2.65 mmol) were dissolved in dry DMF (10 ml). EDC*HCl (297 mg, 1.55 mmol) were added and the reaction mixture was stirred at ambient temperature for 3 h.
The reaction mixture was treated with water (30 ml) and EtOAc (50 ml and stirred for 5 min at RT. The phases were separated and the organic layers were washed with NaHCO₃ solution (25 ml) and brine (25 ml). The solution was dried over Na₂SO₄, filtered and evaporated. The crude product was purified via silica-gel chromatography to provide the title compound as a white crystalline solid. TLC (Hexane / EtOAc 7:3): Rf=0.39;
HPLC: Rt_{H3}= 1.24 min; ESIMS [M+H]⁺= 532;
¹H-NMR (400 MHz, CDCl₃): δ 11.09-11.01 (s, 1 H), 10.01 (s, 1 H), 8.71 (s, 1 H), 7.93 (s, 1 H), 7.80 (m, 1 H), 7.58 (m, 1H), 7.12 (m, 1 H), 5.06 - 4.90 (dd, 1H),), 4.76 (d, 1 H), 4.67 - 4.45 (m, 3H), 4.10 (m, 1H), 2.83 (s, 3H), 1.72 (s, 3H), 1.54 (s, 9H).

### k) 5-Cyano-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide

(5-{5-[(5-Cyano-3-methyl-pyridine-2-carbonyl)-amino]-2-fluoro-phenyl}-2,2-bis-fluoromethyl-5-methyl-5,6-dihydro-2H-[1,4]oxazin-3-yl)-carbamic acid tert-butyl ester (450 mg, 0.847 mmol) was dissolved in DCM (8 ml). TFA (965, 8.47 mmol) was added dropwise. The reaction mixture was then stirred 2 h at ambient temperature. The reaction mixture was added to a cold aqueous Na₂CO₃ solution (50 ml). DCM (30 ml) was added and the reaction mixture was stirred for 10 min. The phases were separated and the organic layers were washed with NaHCO₃ solution (25 ml) and brine (25 ml). The solution was dried over Na₂SO₄, filtered and evaporated to provide the title compound as a white solid.
HPLC: Rt_{H3}= 0.73 min; ESIMS [M+H]⁺= 432;
¹H-NMR (400 MHz, DMSO): δ 10.70- 10.63 (br. s, 1H), 8.96 (s, 1H), 8.37 (s, 1H), 7.75 (m, 2H), 7.11 (m, 1H), 6.10 - 6.00 (s, 2H), 4.90 (dd, 1H),), 4.73 - 4.47 (m, 3H), 3.85 (dd, 2H), 2.51 (s, 3H), 1.40 (s, 3H).

### Example 38: 5-Cyano-3-methyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide

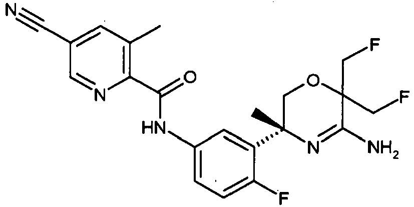

The racemic product 5-Cyano-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide (350 mg) was separated via prep-HPLC on Chiralpak AD-H 320 x 7.65 mm column using n-Heptane / iPrOH 70 : 30 (+ 0.05% Diethyl Amine) as eluent.

The desired compound was the slower eluting (R)-enantiomer (146 mg, white solid, ee = 100 % (Detection at 210 nm)).

### Examples 39 to 41: The compounds listed in Table 4 were prepared by procedures analogous to those used in Example 37 and Example 38.

**Table 4**

| **Example** | **Compound** | **¹H-NMR (δ; DMSO-d₆)** | **MS [m/z; (M+1)⁺]** |
|---|---|---|---|
| **39** | 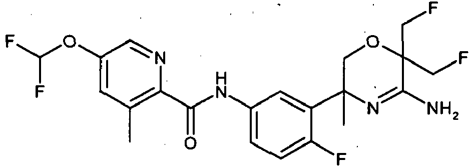 | 10.43 - 10.47 (s, 1H), 8.41 (d, 1H), 7.68-7.82 (m, 3H), 7.23-7.60 (t, 1H), 7.10 (m, 1H), 6.00 - 6.10 (s, 2H), 4.45 - 5.00 (m, 4H), 3.87 (m, 2H), 2.57 (s, 3H), 1.42 (s, 3H). | 473 |
| | 5-Difluoromethoxy-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide | | |
| **40** | 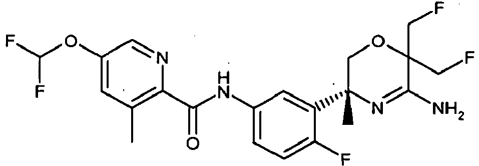 | 10.43 - 10.47 (s, 1H), 8.41 (d, 1H), 7.68-7.82 (m, 3H), 7.23-7.60 (t, 1H), 7.10 (m, 1H), 6.00 - 6.10 (s, 2H), 4.45 - 5.00 (m, 4H), 3.87 (m, 2H), 2.57 (s, 3H), 1.42 (s, 3H). | 473 |
| | 5-Difluoromethoxy-3-methyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide | | |
| **41** | 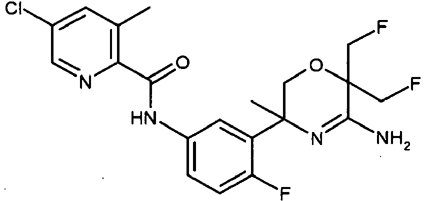 | 10.47 - 10.52 (s, 1H), 8.57 (s, 1H), 8.02 (s, 1H), 7.81 (m, 1H), 7.73 (m, 1H), 7.11 (m, 1H), 6.03 - 6.10 (s, 2H), 4.87 - 5.00 (d, 1H), 4.50 - 4.73 (m, 3H), 3.87 (dd, 2H), 2.54 (s, 3H), 1.41 (s, 3H). | 441 |
| | 5-Chloro-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide | | |

### Example 42: 5-Cyano-3-methyl-pyridine-2-carboxylic acid [3-((R)-5-amino-3,6,6-tris-fluoromethyl-3,6-dihydro-2H-[1,4]oxazin-3-yl)-4-fluoro-phenyl]-amide

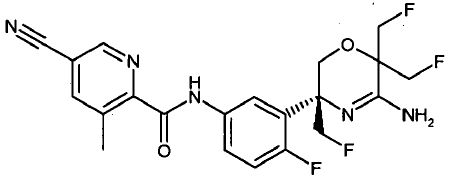

### a) (5-Bromo-2-fluoro-phenyl)-oxo-acetic acid ethyl ester

A solution of 22.80 ml (160 mmol) diisopropyl amine in 400 ml THF was cooled to -78 °C. A 1.6 M solution of BuLi in hexanes (100 ml, 160 mmol) was added dropwise. After 15 minutes 25.45 g of 4-bromo-1-fluoro benzene (145 mmol) was added dropwise while keeping the temperature below -60 °C. After stirring for 2.5 h at -70 °C 21.7 ml diethyl oxalate (160 mmol) were added. The mixture was warmed to -50 °C. After 15 min the temperature cooled to -70 °C again, then the mixture was by poured onto 350 ml 1M HCl. The mixture was extracted with ligroin, dried with MgSO₄.H₂O, concentrated and distilled at ca 6 mbar (b.p. 112-115 °C) to give 31.58 g of the desired product as a yellow liquid. ¹H-NMR (CDCl₃, 400 MHz): δ 8.07 (dd, 1H), 7.77 (ddd, 1H), 7.12 (t, 1 H), 4.47 (q, 2H), 1.44 (t, 3H).

### b) (R)-2-(5-Bromo-2-fluoro-phenyl)-2-hydroxy-3-nitro-propionic acid ethyl ester

To an at -25 °C cooled solution of 35.86 g (130 mmol) (5-bromo-2-fluoro-phenyl)-oxo-acetic acid ethyl ester and 3.59 g (6.52 mmol) Catalyst 1 (CHX135): 3,5-bis-trifluoromethyl-benzoic acid (R)-(6-hydroxy-quinolin-4-yl)-(5-vinyl-1-aza-bicyclo[2.2.2]oct-2-yl)-methyl ester (CAS registry: 1079392-85-0) in 360 ml DCM were added 70.3 ml (1.3 mol) nitromethane. The mixture was kept for 3 days at -20 °C till TLC analysis showed complete conversion. The catalyst was removed by passing the reaction mixture over a small pad of silica gel (DCM/ (EtOH/ sat aq NH₃ 9:1) 99:1). The crude product was purified by chromatography on silica gel (hexanes/ EtOAc 5-15%) to give 39.88 g of the title compound as a colorless oil. E.e. 96%; HPLC: Rt_{H3}= 2.705 min; ESIMS [M+Na]⁺= 358/360(1 Br); ¹H-NMR (CDCl₃, 400 MHz): 6 7.84 (dd, 1 H), 7.51 (ddd, 1 H), 7.01 (dd, 1 H), 5.57 (d, 1 H), 4.86 (d, 1 H), 4.46-4.28 (m, 2H), 1.33 (t, 3H).

### c) (R)-3-Amino-2-(5-bromo-2-fluoro-phenyl)-2-hydroxy-propionic acid ethyl ester

Zn dust (78 g, 1.187 mol) was suspended in 240 ml AcOH using a mechanical stirrer. A solution of (R)-2-(5-bromo-2-fluoro-phenyl)-2-hydroxy-3-nitro-propionic acid ethyl ester (39.88 g, 119 mmol) in 160 ml AcOH was added dropwise to this suspension while keeping the temperature between 30-40 °C with the use of a water bath. After 15 min the mixture was filtered over celite and washed with EtOAc. The filtrate was concentrated, taken up in EtOAc and was washed with 10% soda solution. Any insoluble parts were dissolved by adding some aq. NH₃. The organic layer was washed with sat aq NaHCO₃ and brine, and dried with Na₂SO₄. Evaporation gave the 34 g of the title compound as a white solid, pure enough for further synthesis. HPLC: Rt_{H2}= 2.397 min; ESIMS [M+H]⁺= 306/308(1Br); ¹H-NMR (DMSO-d6, 400 MHz): δ 7.74 (dd, 1H), 7.54 (ddd, 1 H), 7.14 (dd, 1 H), 4.17-4.03 (m, 2H), 3.21 (d, 1H), 2.87 (d, 1H), 1.13 (t, 3H).

### d) (R)-3-Amino-2-(5-bromo-2-fluoro-phenyl)-propane-1,2-diol, hydrochloride

Under nitrogen atmosphere is added dropwise 1.415 ml BH₃.SMe₂ (neat, 14.9 mmol) to a solution of (R)-3-amino-2-(5-bromo-2-fluoro-phenyl)-2-hydroxy-propionic acid ethyl ester (1.52 g, 4.97 mmol) in 15 ml THF. The reaction is exothermic under evolution of gas. The mixture is heated to reflux for 3 h. The excess borane is quenched by the careful addition of 3 ml MeOH. More MeOH is added followed by 3 ml 2M aq. HCl. The mixture is evaporated, dissolved in 20 ml MeOH and evaporated (2x). The residue is crystallized from EtOH(EtOAc to give 907 mg of the title compound as white crystals. HPLC: Rt_{H1}= 2.451 min; ESIMS [M+H]⁺= 264/266(1 Br); ¹H-NMR (DMSO-d6, 400 MHz): δ 7.80 (br, 2H), 7.76 (dd, 1 H), 7.58 (ddd, 1 H), 7.19 (dd, 1 H), 6.30 (s, 1 H), 5.28 (br s, 1 H), 3.72 (d, 1 H), 3.63 (d, 1 H), 3.26 (d, 1 H), 3.14 (d, 1H).

### e) N-[(R)-2-(5-Bromo-2-fluoro-phenyl)-2,3-dihydroxy-propyl]-2-nitro-benzenesulfonamide

A suspension of (R)-3-amino-2-(5-bromo-2-fluoro-phenyl)-propane-1,2-diol, hydrochloride (790 mg, 2.63 mmol), 2-nitro-benzenesulfonyl chloride (583 mg, 2.63 mmol), K₂CO₃ (363 mg, 2.63 mmol) and KHCO₃ (562 mg, 5.26 mmol) in 8 ml ACN was stirred for 2 h. The mixture was partitioned between EtOAc and brine. The organic layer was washed with brine, dried with MgSO₄.H₂O and evaporated. Chromatography on silica gel (hexanes/ EtOAc 25-50%) gave 1.42 g of the title compound as a colorless foam. HPLC: Rt_{H2}= 3.136 min; ESIMS [M+Na]⁺= 371/373(1Br); ¹H-NMR (DMSO-d6, 400 MHz): δ 7.96-7.90 (m, 2H), 7.87-7.79 (m, 2H), 7.65 (dd, 1 H), 7.58 (br, 1 H), 7.44 (ddd, 1 H), 7.03 (dd, 1 H), 5.60 (s, 1 H), 4.88 (t, 1 H), 3.67-3.57 (m, 2H), 3.41 (d, 1 H), 3.31 (d, 1H).

### f) (S)-2-(5-Bromo-2-fluoro-phenyl)-1-(2-nitro-benzenesulfonyl)-2-(tetrahydro-pyran-2-yloxymethyl)-aziridine

To an ice-cold solution of N-[(R)-2-(5-bromo-2-fluoro-phenyl)-2,3-dihydroxy-propyl]-2-nitro-benzenesulfonamide (1.40 g, 3.12 mmol) and dihydropyrane (0.299 ml, 3.27 mmol) in 14 ml DCM was added CSA (36 mg, 0.156 mmol). After warming to rt the mixture was stirred 2 h. EtOAc and sat. aq. NaHCO₃ were added and the organic phase was washed with brine, dried with MgSO₄.H₂O and evaporated. Chromatography on silica gel (hexanes/ EtOAc 25-35%) gave 1.52 g of the title compound as a colorless resin. TLC (hexanes/ EtOAc 2:1): Rf = 0.28; HPLC: Rt_{H3}= 3.348 min; ESIMS [M+Na]⁺= 555/557(1Br). This product was dissolved in 14 ml THF together with PPh3 (838 mg, 3.19 mmol), cooled to 0-5 °C and treated with a 40% toluene solution of DEAD (1.46 ml, 3.19 mmol) in a dropwise manner. Stirring was continued for 2.5 h while slowly warming to rt. The solution was diluted with 20 ml toluene, concentrated and directly purified via chromatography on silica gel (hexanes/ EtOAc 5-15%) to give the title compound as a colorless resin (1:1 mixture of diastereomers). HPLC: Rt_{H4}= 3.361 min; ESIMS [M+Na]⁺= 537/539(1Br); ¹H-NMR (CDCl₃, 400 MHz, 1:1 mixture of diastereomers): δ 8.32-8.27 (m, 1 H), 7.81-7.76 (m, 3H), 7.71-7.65 (m, 1H), 7.46-7.42 (m, 1H), 6.95 (t, 1 H), 5.74 and 5.62 (t, 1H), 4.36 and 4.34 (d, 1H), 4.12 and 4.10 (d, 1H), 3.74-3.57 (m, 1H), 3.52-3.44 (m, 1H), 3.52 and 3.35 (s, 1H), 2.99 and 2.94 (s, 1 H).

### g) N-[(R)-1-(5-Bromo-2-fluoro-phenyl)-1-fluoromethyl-2-hydroxy-ethyl]-2-nitro-benzenesulfonamide

A mixture of (S)-2-(5-bromo-2-fluoro-phenyl)-1-(2-nitro-benzenesulfonyl)-2-(tetrahydro-pyran-2-yloxymethyl)-aziridine (1.08 g, 2.096 mmol) and TBAF.3H₂O (860 mg, 2.72 mmol) in 11 ml DMF was stirred overnight. The mixture was partitioned between brine and TBME. The organic layer was washed with diluted brine (3x), dried with MgSO₄.H₂O and evaporated to give 1.12 g of the mono fluoro THP ether as a yellow resin (1:1 mixture of diastereomers). TLC (hexanes/ EtOAc 4:1): Rf = 0.26; HPLC: Rt_{H4}= 3.328 and 3.429 min; ESIMS [M+Na]⁺= 557/559(1 Br). The product was taken up in 16 ml MeOH and 6 ml THF containing 49 mg (0.209 mmol) CSA and stirred. After 6 h the reaction was complete and the homogeneous mixture was partitioned between EtOAc and sat aq NaHCO3. The organic phase was washed with sat. aq. NaHCO₃, dried with MgSO₄.H₂O and evaporated. The title compound was obtained as white crystals (741 mg, TBME/hexanes). HPLC: Rt_{H3}= 2.733 min; ESIMS [M+Na]⁺= 473/475(1Br); ¹H-NMR (DMSO-d6, 400 MHz): δ 8.40 (s, 1H), 7.88 (d, 1H), 7.77 (dt, 1 H), 7.68-7.62 (m, 2H), 7.47-7.40 (m, 2H), 6.89 (dd, 1H), 5.38 (t, 1 H), 5.07 (q, 1H), 4.94 (q, 1H), 3.89-3.73 (m, 2H).

### h) (R)-2-(5-Bromo-2-fluoro-phenyl)-2-fluoromethyl-1-(2-nitro-benzenesulfonyl)-aziridine

N-[(R)-1-(5-Bromo-2-fluoro-phenyl)-1-fluoromethyl-2-hydroxy-ethyl]-2-nitro-benzenesulfonamide (662 mg, 1.467 mmol) was dissolved in 7 ml THF together with PPh3 (462 mg, 1.76 mmol), cooled to 0-5 °C and treated with a 40% toluene solution of DEAD (0.807 ml, 1.76 mmol) in a dropwise manner. Stirring was continued for 2.5 h while slowly warming to rt. The solution was diluted with 20 ml toluene, concentrated and directly purified via chromatography on silica gel (hexanes/ EtOAc 5-15%) to give the title compound as a colorless resin. HPLC: Rt_{H3}= 3.274 min; ESIMS [M+Na]⁺= 455/457(1Br); ¹H-NMR (CDCl3, 400 MHz): δ 8.34-8.30 (m, 1H), 7.84-7.80 (m, 3H), 7.68 (dd, 1 H), 7.49 (ddd, 1 H), 7.00 (t, 1H), 5.04 (d, 2H), 3.40 (s, 1H), 3.03 (d, 1H).

### i) 2-[(R)-2-(5-Bromo-2-fluoro-phenyl)-3-fluoro-2-(2-nitro-benzenesulfonylamino)-propoxy]-3-fluoro-2-fluoromethyl-propionic acid ethyl ester

To a suspension of NaH (78 mg, 60% in mineral oil, 1.94 mmol) in DMF (160 ml) was added drop-wise under argon 3-fluoro-2-fluoromethyl-2-hydroxy-propionic acid ethyl ester (327 mg, 1.94 mmol) and after stirring for 0.5 h at 20 °C (R)-2-(5-bromo-2-fluoro-phenyl)-2-fluoromethyl-1-(2-nitro-benzenesulfonyl)-aziridine (526 mg, 1.214 mmol). The reaction was kept at 25 °C for 16 h. The mixture was added to cold aq. 2N HCl and the product extracted with TBME. Combined organic layers were washed with saturated NaHCO₃ solution and brine, dried over MgSO₄.H₂O, filtered and concentrated. The residual compound was purified via chromatography on silica gel (hexanes/ EtOAc 10-20%) to give the title compound as a white solid. TLC (hexanes/ EtOAc 1:1): Rf = 0.59; HPLC Rt_{H4}= 3.230 min; ESIMS [M+Na]⁺= 623, 625(1Br); ¹H NMR (400 MHz, CDCl₃): δ 7.93 (dd, 1 H), 7.75 (dt, 1H), 7.66 (dt, 1H), 7.44 (dt, 1 H), 7.39 (dd, 1 H), 7.35 (ddd, 1 H), 6.94 (s, 1 H), 6.53 (dd, 1 H), 5.33-4.62 (m, 6H), 4.39 (q, 2H), 4.19 (d, 1H), 4.14 (d, 1H), 1.37 (t, 3H).

### j) (R)-5-(5-Bromo-2-fluoro-phenyl)-2,2,5-tris-fluoromethyl-4-(2-nitro-benzenesulfonyl)-morpholin-3-one

To a solution of 2-[(R)-2-(5-bromo-2-fluoro-phenyl)-3-fluoro-2-(2-nitro-benzenesulfonylamino)-propoxy]-3-fluoro-2-fluoromethyl-propionic acid ethyl ester (462 mg, 0.768 mmol) in 3 ml MeOH and 2 ml THF were added 0.96 ml (3.84 mmol) of 4M aq. LiOH. The mixture was stirred at rt for 30 min. The reaction mixture was taken up in 1 N HCl and EtOAc. The organic phase was washed with brine, dried with MgSO₄.H₂O and evaporated to give 445 mg of the acid as a white solid. HPLC Rt_{H4}= 3.230 min; ESIMS [M+Na]⁺= 595, 597(1 Br). The acid was suspended in DCM and N-methyl morpholine (263 mg, 2.60 mmol) was added, followed by ethyl chloroformate (141 mg, 1.300 mmol) in a drop-wise manner. The resulting yellow solution was stirred at rt for 1 h. The reaction mixture was partitioned between 1 N HCl and EtOAc. The organic layer was washed with brine and 10% aq NaHCO₃, dried with MgSO₄.H₂O and evaporated. Crystallization from TBME/hexanes provided the title compound. TLC (hexanes/ EtOAc 3:1): Rf = 0.20; HPLC Rt_{H4}= 3.062 min; ESIMS [M+Na]⁺= 577/579(1 Br); ¹H NMR (400 MHz, CDCl₃): δ 8.30 (d, 1 H), 7.83-7.74 (m, 4H), 7.57 (ddd, 1H), 7.08 (dd, 1H), 5.68 (dd, 1H), 5.47 (dd, 1H), 4.48 (ddd, 2H), 4.66 (dd, 1 H), 4.60 (d, 1H), 4.51 (d, 1H), 4.39 (d, 1H).

### k) (R)-5-(5-Bromo-2-fluoro-phenyl)-2,2,5-tris-fluoromethyl-morpholin-3-one

A mixture of (R)-5-(5-bromo-2-fluoro-phenyl)-2,2,5-tris-fluoromethyl-4-(2-nitro-benzenesulfonyl)-morpholin-3-one (365 mg, 0.657 mmol), K₂CO₃ (363 mg, 2.63 mmol) and thioglycolic acid (121 mg, 1.315 mmol) in 3.5 ml DMF was stirred at 60 °C for 3 h.The mixture was diluted with EtOAc and brine. The org layer was washed with sat aq NaHCO₃ and brine, dried with MgSO₄.H₂O and evaporated. The residual compound was purified via chromatography on silica gel (hexanes/ EtOAc 10-25%) to give the title compound as a white solid. TLC (hexanes/ EtOAc 3:1): Rf = 0.31; HPLC Rt_{H2}= 3.202 min; ESIMS [M+H]⁺= 370/372 (1 x Br); ¹H NMR (400 MHz, CDCl₃): δ 7.56-7.51 (m, 2H), 7.06 (dd, 1 H), 6.85 (br, 1H), 4.98-4.30 (m, 8H).

### l) (R)-5-(5-Bromo-2-fluoro-phenyl)-2,2,5-tris-fluoromethyl-morpholine-3-thione

To a solution of (R)-5-(5-bromo-2-fluoro-phenyl)-2,2,5-tris-fluoromethyl-morpholin-3-one (141 mg, 0.381 mmol) and hexamethyldisiloxane (111 mg, 0.686 mmol) in toluene was added phosphorous pentasulfide (102 mg, 0.457 mmol). The reaction mixture was heated to 100 °C and stirred 4 h. After the reaction mixture had been cooled to room temperature, 1 ml acetone and 1.42 ml aq K₂CO₃ solution (10% w/w) were added. This mixture was stirred for 90 minutes and then partitioned between water and EtOAc. The layers were separated, washed with 0.1 N NaOH, brine and EtOAc. The organic layers were combined, dried over MgSO₄.H₂O and evaporated. The crude product was purified via chromatography on silica gel (hexanes/ EtOAc 10-15%) to give the title compound as a white solid: TLC (hexanes/ EtOAc 6:1): Rf = 0.38; HPLC Rt_{H2}= 3.553 min; ESIMS [M+H]⁺= 386/388 (1 x Br); ¹H NMR (400 MHz, CDCl₃): δ 8.62 (br, 1H), 7.56 (ddd, 1 H), 7.47 (dd, 1H), 7.08 (dd, 1H), 5.12-4.70 (m, 6H), 4.95 (d, 1H), 4.33 (d, 1H).

### m) (R)-5-(5-Bromo-2-fluoro-phenyl)-2,2,5-tris-fluoromethyl-5,6-dihydro-2H-[1,4]oxazin-3-ylamine

(R)-5-(5-Bromo-2-fluoro-phenyl)-2,2,5-tris-fluoromethyl-morpholine-3-thione (134 mg, 0.347 mmol) was dissolved in NH₃ solution 7 mol/l in methanol (3 ml). The sealed reaction vessel was heated to 80 °C for 3 days. The reaction mixture was evaporated and purified on a silica gel column by eluting with (hexanes/ EtOAc 15-35%) to give the title compound as a colorless resin. TLC (hexanes/ EtOAc 3:1): Rf = 0.13; HPLC: Rt_{H2}= 2.684 min; ESIMS [M+H]⁺= 369/371(1Br); ¹H-NMR (CDCl3, 400 MHz): δ 11.91 (s, 1H), 7.72 (dd, 1H), 7.54-7.45 (m, 2H), 7.08-6.96 (m, 2H), 5.20-4.25 (m, 8H).

### n) [(R)-5-(5-Bromo-2-fluoro-phenyl)-2,2,5-tris-fluoromethyl-5,6-dihydro-2H-[1,4]oxazin-3-yl]-carbamic acid tert-butyl ester

To a solution of (R)-5-(5-bromo-2-fluoro-phenyl)-2,2,5-tris-fluoromethyl-5,6-dihydro-2H-[1,4]oxazin-3-ylamine (113 mg, 0.306 mmol) in 1 ml DCM were added DIPEA (60 mg, 0.46 mmol) and di-tert-butyldicarbonate (87 mg, 0.4 mmol). The reaction mixture was stirred overnight at 40 °C. The reaction mixture was evaporated and purified on a silica gel column by eluting with hexanes/ TBME 5-20% to give 132 mg of the title compound as a colorless foam. TLC (hexanes/ EtOAc 9:1): Rf = 0.16; HPLC: Rt_{H4}= 3.123 min; ESIMS = [M+H]⁺ 469/471(1 Br); ¹H-NMR (CDCl₃, 400 MHz): δ 11.22 (br s, 1 H), 7.54-7.45 (m, 2H), 7.05 (dd, 1H), 5.06-4.34 (m, 8H), 1.53 (s, 9H).

### o) [(R)-5-(5-Amino-2-fluoro-phenyl)-2,2,5-tris-fluoromethyl-5,6-dihydro-2H-[1,4]oxazin-3-yl]-carbamic acid tert-butyl ester

To a solution [(R)-5-(5-bromo-2-fluoro-phenyl)-2,2,5-tris-fluoromethyl-5,6-dihydro-2H-[1,4]oxazin-3-yl]-carbamic acid tert-butyl ester (132 mg, 0.283 mmol) and 40.2 mg (0.283 mmol) trans-N,N'-dimethylcyclohexanes-1,2-diamine in 4 ml EtOH was added a solution of 147 mg (2.26 mmol) sodium azide and 22.4 mg (0.113 mmol) sodium-ascorbate in 1.6 ml water. The mixture was degassed and brought under nitrogen atmosphere. CuI (21.5 mg, 0.113 mmol) was added and the mixture was heated at 70 °C. The initially formed suspension turned into a homogeneous blue solution. The mixture was cooled to rt, diluted with TBME and washed with diluted aq. NH₄OH and brine. The organic phase was dried with MgSO₄.H₂O and evaporated to give 128 mg of a yellow resin, consisting of a mixture of an azide intermediate and the title compound. The product was dissolved in 1.3 ml EtOH and 0.2 ml THF, treated with 68 mg 5% Pd-C "Degussa" E101 ND and stirred under an atmosphere of hydrogen until the starting material had been consumed. The mixture was diluted with DCM and filtered over Celite. The product was purified by chromatography on silica gel (hexanes/ EtOAc 25-50%) to give 71 mg of the title compound as colorless foam. HPLC: Rt_{H2}= 2.963 min; ESIMS = [M+H]⁺ 406; ¹H-NMR (CDCl₃, 400 MHz): δ 6.93 (dd, 1H), 6.72-6.67 (m, 2H), 5.09-4.33 (m, 8H), 1.53 (s, 9H).

### p) ((R)-5-{5-[(5-Cyano-3-methyl-pyridine-2-carbonyl)-amino]-2-fluoro-phenyl}-2,2,5-tris-fluoromethyl-5,6-dihydro-2H-[1,4]oxazin-3-yl)-carbamicacid tert-butyl ester

To an ice-cold solution of [(R)-5-(5-amino-2-fluoro-phenyl)-2,2,5-tris-fluoromethyl-5,6-dihydro-2H-[1,4]oxazin-3-yl]-carbamic acid tert-butyl ester (71 mg, 0.176 mmol), 5-cyano-3-methyl-pyridine-2-carboxylic acid (31.5 mg, 0.194 mmol), HOAt (38.4 mg, 0.282 mmol) in 0.72 ml DMF were added 0.04 ml (0.23 mmol) EDC (free base). The mixture was stirred at 0-5 °C for 1 h and 2 h at rt. EtOAc and water were added and the organic layer was washed with sat. aq. NaHCO₃, brine and dried with MgSO₄.H₂O. The product was purified by chromatography on silica gel (hexanes/ EtOAc 15-50%) to give 94 mg of the title compound as colorless foam. TLC ((hexane/ EtOAc 3:1): Rf = 0.18; HPLC: Rt_{H3}= 3.452 min; ESIMS = [M+H]⁺ 550; ¹H-NMR (CDCl₃, 400 MHz): δ 11.28 (s, 1H), 10.12 (s, 1H), 8.76 (s, 1H), 7.99 (s, 1H), 7.92 (ddd, 1 H), 7.71 (dd, 1 H), 7.22 (dd, 1H), 5.05-4.44 (m, 8H), 2.89 (s, 3H), 1.59 (s, 9H).

### q) 5-Cyano-3-methyl-pyridine-2-carboxylic acid [3-((R)-5-amino-3,6,6-tris-fluoromethyl-3,6-dihydro-2H-[1,4]oxazin-3-yl)-4-fluoro-phenyl]-amide

To a solution of ((R)-5-{5-[(5-cyano-3-methyl-pyridine-2-carbonyl)-amino]-2-fluoro-phenyl}-2,2,5-tris-fluoromethyl-5,6-dihydro-2H-[1,4]oxazin-3-yl)-carbamicacid tert-butyl ester (94 mg, 0.172 mmol) in 0.75 ml DCM were added 0.25 ml TFA. The mixture was stirred for 1 h, poured onto 10% aq. Na2CO3 and extracted with EtOAc. The org layer was washed with brine and dried with Na₂SO₄. The product was purified by chromatography on silica gel (DCM/ (EtOH/aq NH₃ 9:1) 0.5-1.5%) to give 59 mg of the title compound as colorless foam. HPLC: Rt_{H2}= 2.850 min; ESIMS = [M+H]⁺ 450;
¹H-NMR (DMSO-d6, 600 MHz): δ 10.69 (s, 1H), 8.98 (s, 1 H), 8.39 (s, 1H), 7.92 (m, 1H), 7.77 (m, 1H), 7.15 (dd, 1 H), 6.33 (br s, 2H), 4.98-4.40 (m, 6H), 4.16 8d, 1H), 4.00 (d, 1 H), 2.52 (s, 3H).

### Examples 43 to 45: Example 43 in Table 5 was made using a procedure analogous to that used to prepare Example 42, whereas Examples 44 and 45 were made using a procedure analogous to that used to prepare Example 17.

**Table 5**

| **Example** | **Compound** | **¹H-NMR (δ; DMSO-d₆)** | **MS** |
|---|---|---|---|
| **43** | 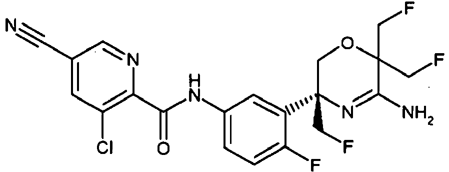 | 10.90 (s, 1H), 9.11 (s, UPLC: 1H), 8.82 (s, 1H), 7.85 (m, 1H), 7.76 (m, 1H), 7.18 (dd, 1H), 6.37 (br s, 2H), 5.01-3.97 (m, 8H), 4.15 (m, 1H), 1.67 (br s, 3H), 1.47 (s, 9H) | UPLC: Rt_{H10} = 0.74 min; ESI+: 470 [(M+H)⁺] |
| | 3-Chloro-5-cyano-pyridine-2-carboxylic acid [3-((R)-5-amino-3,6,6-tris-fluoromethyl-3,6-dihydro-2H-[1,4]oxazin-3-yl)-4-fluoro-phenyl]-amide | | |
| **44** | 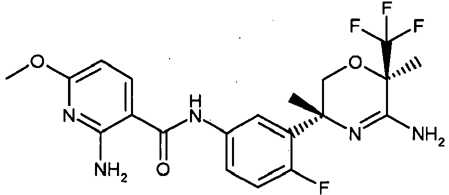 | 11.55 (s, 1H), 10.14 (s, 1H), 9.58 (d, 1H), 8.14 (d, 1H), 7.78 (dd, 1H), 7.72 (m, 1H), 7.39 (br s, 1H), 7.32 (dd, 1H), 6.11 (d, 1H), 4.33 (d, 1H), 4.11 (d, 1H), 3.84 (s, 3H), 1.77 (s, 3H), 1.73 (s, 3H) | UPLC: Rt_{H12} = 0.790 min; MS [m/z; (M+1)⁺] 456 |
| | 2-Amino-N-[3-((3R,6R)-5-amino-3,6-dimethyl-6-trifluoromethyl-3,6-dihydro-2H-[1,4]oxazin-3-yl)-4-fluoro-phenyl]-6-methoxy-nicotinamide | | |
| **45** | 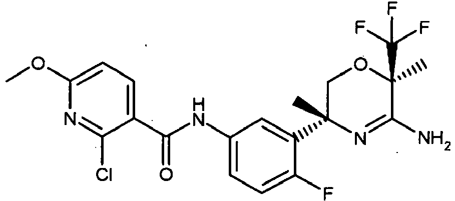 | 11.62 (s, 1H), 10.80 (s, 1H), 9.62 (m, 2H), 8.00 (d, 1H), 7.78 (m, 2H), 7.35 (dd, 1H), 6.99 (d, 1H), 4.32 (d, 1H), 4.09 (d, 1H), 3.92 (s, 3H), 1.75 (s, 3H), 1.73 (s, 3H) | UPLC: Rt_{H12} = 0.891 min; MS [m/z; (M+1)⁺] 475, 477 |
| | N-[3-((3R,6R)-5-Amino-3,6-dimethyl-6-trifluoromethyl-3,6-dihydro-2H-[1,4]oxazin-3-yl)-4-fluoro-phenyl]-2-chloro-6-methoxy-nicotinamide | | |

The compounds in Table 6 below may also be made using the procedures described hereinbefore or procedures analogous thereto.

**Table 6**

| | |
|---|---|
| 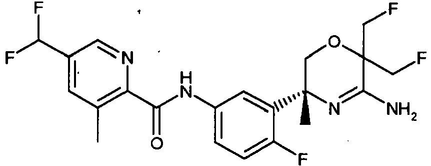 | 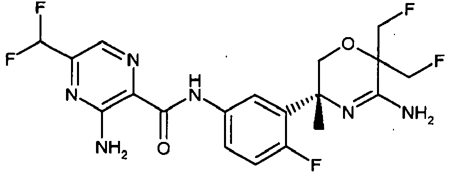 |
| 5-Difluoromethyl-3-methyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide | 3-Amino-5-difluoromethyl-pyrazine-2-carboxylic acid [3-((R)-5-amino-6,6-bis-fluoromethyl-3-methyl-3,6-dihydro-2H-[1,4]oxazin-3-yl)-4-fluoro-phenyl]-amide |
| 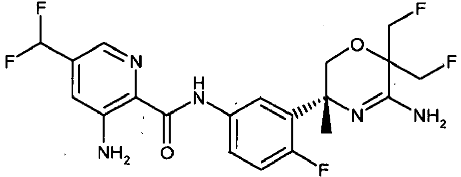 | 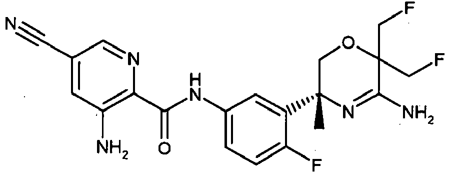 |
| 3-Amino-5-difluoromethyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide | 3-Amino-5-cyano-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide |
| 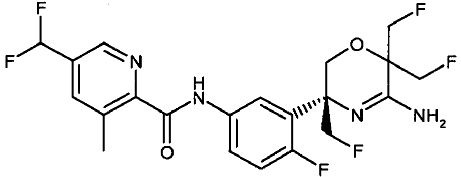 | 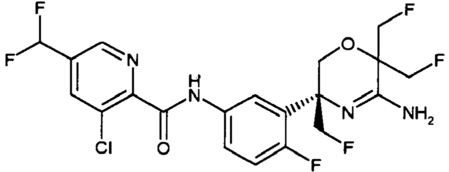 |
| 5-Difluoromethyl-3-methyl-pyridine-2-carboxylic acid [3-((R)-5-amino-3,6,6-tris-fluoromethyl-3,6-dihydro-2H-[1,4]oxazin-3-yl)-4-fluoro-phenyl]-amide | 3-Chloro-5-difluoromethyl-pyridine-2-carboxylic acid [3-((R)-5-amino-3,6,6-tris-fluoromethyl-3,6-dihydro-2H-[1,4]oxazin-3-yl)-4-fluoro-phenyl]-amide |
| 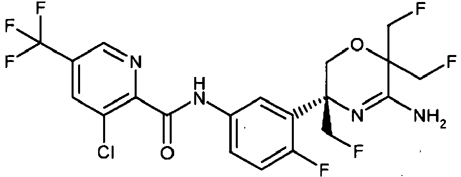 | 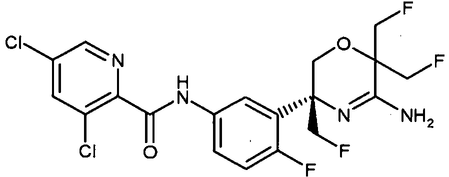 |
| 3-Chloro-5-trifluoromethyl-pyridine-2-carboxylic acid [3-((R)-5-amino-3,6,6-tris-fluoromethyl-3,6-dihydro-2H-[1,4]oxazin-3-yl)-4-fluoro-phenyl]-am ide | 3,5-Dichloro-pyridine-2-carboxylic acid [3-((R)-5-amino-3,6,6-tris-fluoromethyl-3,6-dihydro-2H-[1,4]oxazin-3-yl)-4-fluoro-phenyl]-amide |
| 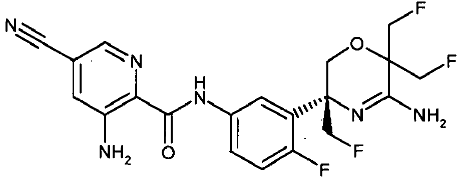 | 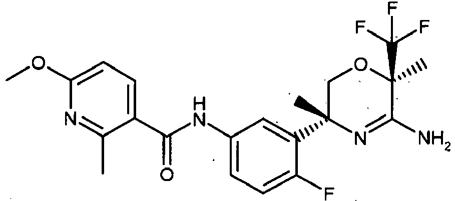 |
| 3-Amino-5-cyano-pyridine-2-carboxylic acid [3-((R)-5-amino-3,6,6-tris-fluoromethyl-3,6-dihydro-2H-[1,4]oxazin-3-yl)-4-fluoro-phenyl]-amide | N-[3-((3R,6R)-5-Amino-3,6-dimethyl-6-trifluoromethyl-3,6-dihydro-2H-[1,4]oxazin-3-yl)-4-fluoro-phenyl]-6-methoxy-2-methyl-nicotinamide |
| 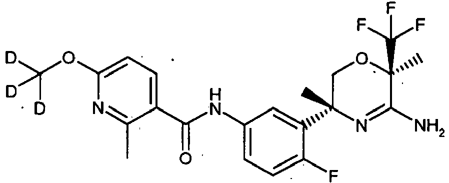 | 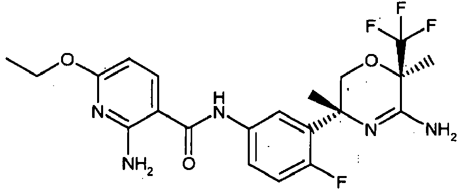 |
| N-[3-((3R,6R)-5-Amino-3,6-dimethyl-6-trifluoromethyl-3,6-dihydro-2H-[1,4]oxazin-3-yl)-4-fluoro-phenyl]-6-trideuteromethoxy-2-methyl-nicotinamide | 2-Amino-N-[3-((3R,6R)-5-amino-3,6-dimethyl-6-trifluoromethyl-3,6-dihydro-2H-[1,4]oxazin-3-yl)-4-fluoro-phenyl]-6-ethoxy-nicotinamide |
| 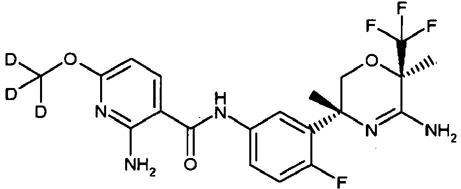 | 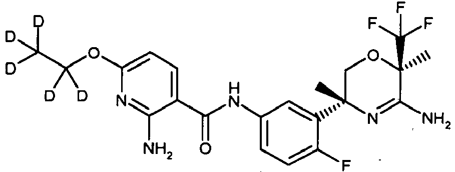 |
| 2-Amino-N-[3-((3R,6R)-5-amino-3,6-dimethyl-6-trifluoromethyl-3,6-dihydro-2H-[1,4]oxazin-3-yl)-4-fluoro-phenyl]-6-trideuteromethoxy-nicotinamide | 2-Amino-N-[3-((3R,6R)-5-amino-3,6-dimethyl-6-trifluoromethyl-3,6-dihydro-2H-[1,4]oxazin-3-yl)-4-fluoro-phenyl]-6-pentadeuteroethoxy-nicotinamide |
| 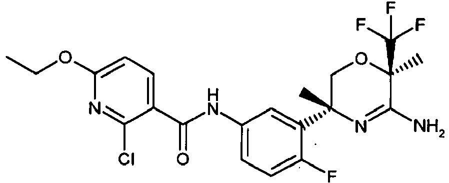 | 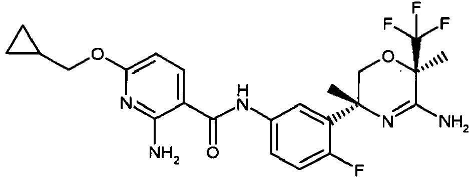 |
| N-[3-((3R,6R)-5-Amino-3,6-dimethyl-6-trifluoromethyl-3,6-dihydro-2H-[1,4]oxazin-3-yl)-4-fluoro-phenyl]-2-chloro-6-ethoxy-nicotinamide | 2-Amino-N-[3-((3R,6R)-5-amino-3,6-dimethyl-6-trifluoromethyl-3,6-dihydro-2H-[1,4]oxazin-3-yl)-4-fluoro-phenyl]-6-cyclopropylmethoxy-nicotinamide |
| 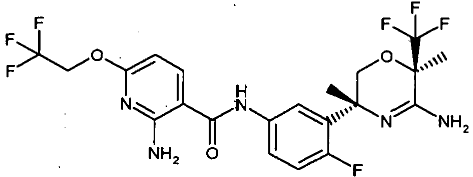 | |
| 2-Amino-N-[3-((3R,6R)-5-amino-3,6-dimethyl-6-trifluoromethyl-3,6-dihydro-2H-[1,4]oxazin-3-yl)-4-fluoro-phenyl]-6-(2,2,2-trifluoro-ethoxy)-nicotinamide | |

### Preparation of Intermediates

The substituted acid building blocks were either commercially available or can be prepared as described in the literature or in an analogous manner, e.g. DE19725802A1, Tetrahedron: Asymmetry 1999, 10(4), 679-687, WO 2005063738, WO 2009091016, WO 2010047372, Bioorg. Med. Chem. 2001, 9, 2061-2071, or can be prepared as described hereafter or in an analogous manner.

### Acid-1: 5-Chloro-3-methoxymethyl-pyridine-2-carboxylic acid

### a) (2,5-Dichloro-pyridin-3-yl)-methanol

A 100 ml round bottomed flask was charged with 2,5-dichloropyridine-3-carbaldehyde (Matrix Sci., 3.4 g, 19.32 mmol) followed by addition of ethanol (50 ml). Sodium borohydride was added at room temperature in small portions. After 1 h the starting material was consumed and the reaction was quenched carefully with addition of diluted aq. acetic acid. The reaction mixture was diluted with ethyl acetate, washed with saturated bicarbonate solution and brine, dried over sodium sulfate, filtered and evaporated, to provide the title compound as white solid.
TLC: Rf=0.43 (2:1 cyclohexane:ethyl acetate);
¹H-NMR (400 MHz, CDCl₃): δ 8.29 (d, 1 H), 7.94 (d, 1 H), 4.80 (d, 2H), 2.23 (broad unresolved triplett, 1H, OH).

### b) 2,5-Dichloro-3-methoxymethyl-pyridine

To a solution of (2,5-dichloro-pyridin-3-yl)-methanol (1000 mg, 5.62 mmol) in dry DMF (25 ml) was added sodium hydride (245 mg, 5.62 mmol, 55% in oil) at 0 °C. After 15 minutes methyliodide (0.457 ml, 7.30 mmol) was added and stirring was continued at room temperature over night. The reaction mixture was quenched with water and diluted with ethyl acetate. The organic phase was washed with saturated bicarbonate solution and brine, dried over sodium sulfate, filtered and evaporated. The crude yellow oil was chromatographed over silica gel gel (cyclohexane:ethyl acetate 83:17) to provide the title compound as a clear oil.
TLC: Rf=0.57 (5:1 cyclohexane:ethyl acetate);
¹H-NMR (360 MHz, CDCl₃): δ 8.25 (d, 1H), 7.82 (d, 1H), 4.48 (d, 2H), 3.51 (s, 3H).

### c) 5-Chloro-3-methoxymethyl-pyridine-2-carbonitrile

To a mixture of 2,5-dichloro-3-methoxymethyl-pyridine (1150 mg, 5.99 mmol) zinc cyanide (492 mg, 4.19 mmol) and zinc dust (39.2 mg, 0.599 mmol) in dry DMF (18 ml) was added (dppf)PdCl₂ CH₂Cl₂ adduct catalyst (245 mg, 0.299 mmol) under nitrogen. The mixture was heated at 150 °C for 2 hours. After 2 h the starting material was consumed and the reaction mixture was diluted with ethyl acetate and washed with saturated bicarbonate solution and brine, dried over sodium sulfate, filtered and evaporated. The crude dark residue (960 mg) was chromatographed over silica gel (cyclohexane:ethyl acetate 80:20) to provide the title compound as a yellow solid.
TLC: Rf=0.41 (3:1 cyclohexane:ethyl acetate);
LC-MS: Rt_{H9}= 0.83 min. (100 % pure, ESI+ 183, 185);
¹H-NMR (360 MHz, CDCl₃): δ 8.56 (d, 1H, H6), 7.95 (d, 1H, H4), 4.66 (s, 2H), 3.51 (s, 3H).

### d) 5-Chloro-3-methoxymethyl-pyridine-2-carboxylic acid

A suspension of 5-chloro-3-methoxymethyl-pyridine-2-carbonitrile (100 mg, 0.548 mmol) in 2N NaOH (2 ml) was stirred at 100° C for 4 hours. The reaction mixture was washed with diethyl ether and then set acidic (pH 5-6) with 2M HCl. The aqueous layer was extracted with ethyl acetate and the organic phase washed with brine, dried over sodium sulfate, filtered and evaporated to provide the title compound as white solid.
MS: ESI- 200; LC-MS: RtH₉= 0.58 min. (100 % pure, ESI+ 202);
¹H-NMR (360 MHz, CDCl₃): δ 11.1 (s, broad, 1H, COOH), 8.45 (d, 1H, H6), 8.25 (d, 1H, H4), 4.99 (s, 2H), 3.55 (s, 3H).

### Acid-2: 3-Amino-5-methoxy-pyrazine-2-carboxylic acid

### a) 3-Amino-5-tri-deutero-methoxy-pyrazine-2-carboxylic acid tri-deutero methyl ester

To a solution of 0.217 ml (5.33 mmol) tetra-deutero methanol in 7 ml THF was added at 0 °C 94 mg (2.346 mmol) 60% sodium hydride in oil and the mixture was stirred at room temperature for 1 h. After re-cooling to 0 °C 400 mg (2.132 mmol) 3-amino-5-chloro-pyrazine-2-carboxylic acid methyl ester (GB 1248146) was added and the mixture was allowed to warm to room temperature and stirred for four days.
Saturated aq. NH₄Cl was added and the mixture was extracted with EtOAc, the combined organic layers were washed with saturated aq. sodium chloride, dried with Na₂SO₄ and evaporated. The residue was purified by chromatography on silica gel (cyclohexane to cyclohexane/EtOAc 1:3) to provide the title compound as colorless solid.
HPLC: Rt_{H9}= 0.61 min; ESIMS [M+H]⁺= 190.2;
¹H-NMR (400 MHz, DMSO-*d*₆): δ 7.51 (s, 1H), 7.48 (br s, 2H).

### b) 3-Amino-5-tri-deutero-methoxy-pyrazine-2-carboxylic acid

To a solution of 49 mg (0.259 mmol) 3-amino-5-tri-deutero-methoxy-pyrazine-2-carboxylic acid tri-deutero methyl ester in 2 ml THF was added 0.388 ml (0.388 mmol) 1N sodium hydroxide and the mixture was stirred at room temperature for 60 h. To the mixture were added 0.363 ml (0.363 mmol) 1N HCl after stirring for 5 min toluene was added and the solvents were evaporated to provide the title compound together with sodium chloride as colorless solid. The mixture was used for coupling reactions without further purification. HPLC: Rt_{H9}= 0.50 min; ESIMS [M+H]⁺= 173.1;
¹H NMR (400 MHz, DMSO-*d*₆): δ 7.22 (s, 1H).

### Acid-3: 3-Amino-5-prop-2-ynyloxy-pyrazine-2-carboxylic acid

The title compound was prepared by an analogous procedure to Acid-2 using prop-2-yn-1-ol instead of tetra-deutero methanol [Acid-2 step a)].
HPLC: Rt_{H9}= 0.59 min; ESIMS [M+H]⁺= 194.1;
¹H NMR (400 MHz, DMSO-*d*₆): δ 7.58 (br. s, 2H), 7.48 (s, 1H), 4.96 (d, 2H), 3.58 (s, 1H).

### Acid-4: 3-Chloro-1H-pyrrolo[2,3-b]pyridine-6-carboxylic acid

### a) 1H-Pyrrolo[2,3-b]pyridine-6-carbonitrile

To a mixture of 6-bromo-1H-pyrrolo[2,3-b]pyridine (Synthesis, 1992, 661, example 3b) (788 mg, 4 mmol), zinccyanide (329 mg, 2.80 mmol) and zinc dust (26.2 mg, 0.4 mmol) in dry DMF (12 ml) was added (dppf)PdCl₂xCH₂Cl₂ adduct catalyst (163 mg, 0.2 mmol) under nitrogen. The mixture was heated at 140 °C for 4 h. The reaction mixture was diluted with ethyl acetate and washed with aq. Saturated bicarbonate solution and brine, dried over sodium sulfate, filtered and evaporated. 1.04 g dark yellow oil. The crude product was chromatographed over silica gel (cyclohexane/ethyl acetate 3:1) to provide the title compound as a white solid.
TLC Rf=0.35 (2:1 cyclohexane:ethyl acetate);
LC-MS: Rt_{H11}= 0.81 min. (100% purity, ESI+ 144), API-ES+ 144;
¹H-NMR (400 MHz, CDCl₃): δ 11.05 (s, 1 H, NH), 8.08 (d, 1 H), 7.71 (dd, 1 H), 7.52 (d, 1 H), 6.66 (m, 1H).

### b) 1H-Pyrrolo[2,3-b]pyridine-6-carboxylic acid

A suspension of 1H-pyrrolo[2,3-b]pyridine-6-carbonitrile (690 mg, 4.82 mmol) in NaOH 2M (12 ml) was stirred at 100 °C for 6 h. The reaction mixture was washed with diethyl ether and the aq. phase was set slightly acidic (pH 6-7) with conc. HCl. The solid formed was filtered off and dried to provide the title compound.
LC-MS: Rt_{H8}= 0.51 min. (100% purity, ESI+ 163);
¹H-NMR (400 MHz, DMSO-D₆): δ 12.78 (s, 1H), 12.01 (s, 1 H), 8.08 (d, 1 H), 7.80 (m, 1H), 7.73 (s, broad, unresolved, 1H), 6.56 (s, broad, 1H).

### c) 3-Chloro-1H-pyrrolo[2,3-b]pyridine-6-carboxylic acid

A solution of 1H-pyrrolo[2,3-b]pyridine-6-carboxylic acid (300 mg, 1.85 mmol) and NCS (247 mg, 1.85 mmol) in dry DMF (12 ml) was stirred under argon at room temerature for 20 h. The reaction mixture was diluted with ethyl acetate and washed with brine. The precipitate formed was filtered off, washed with ethyl acetate and dried to provide the title compound as light brown solid.
LC-MS: Rt_{H8}= 0.72 min. (100% purity, ESI+ 197/199);
¹H-NMR (400 MHz, DMSO-D₆): δ 13.03 (s, 1H), 12.43 (s, 1H), 8.07 (d, 1H), 7.96 (d, 1H), 7.90 (d, 1 H).

### Acid-5: 3-(di-tert-Butoxycarbonyl-amino)-5-difluoromethyl-pyrazine-2-carboxylic acid

### a) 3-Amino-5-vinyl-pyrazine-2-carboxylic acid methyl ester

To a mixture of 161 mg (0.86 mmol) 3-amino-5-chloro-pyrazine-2-carboxylic acid methyl ester (GB 1248146), 0.352 ml (1.204 mmol) tributyl(vinyl)tin and 102 mg (2.498 mmol) lithium chloride in DMF was added 30.2 mg (0.043 mmol) PdCl₂(PPh₃)₂ and the mixture was heated to 85 °C for 2.5 h. After cooling to room temperature,water was added and the mixture was extracted with EtOAc, the combined organic layers were washed with water and half saturated aq. NaCl, dried with Na₂SO₄ and evaporated. The residue was purified by chromatography on silica gel (cyclohexane to cyclohexane/EtOAc 1:9) to provide the title compound as yellow solid.
HPLC: Rt_{H11}= 0.71 min; ESIMS [M+H]⁺= 179.9;
¹H-NMR (600 MHz, DMSO*-d*₆): δ 8.04 (s, 1H), 7.35 (br. s, 1H), 6.75 (dd, 1H), 6.38 (d, 1H), 5.70 (d, 1H), 3.84 (s, 3H).

### b) 3-(di-tert-Butoxycarbonyl-amino)-5-vinyl-pyrazine-2-carboxylic acid methyl ester

To an ice cooled solution of 1.28 g (7.14 mmol) 3-amino-5-vinyl-pyrazine-2-carboxylic acid methyl ester in 45 ml DCM was added 8.58 g (39.3 mmol) Boc₂O and the mixture was stirred at room temperature for 30 min, then the mixture was heated to 40 °C for 4 h. After cooling to room temperature water was added and the mixture was extracted with DCM. The combined organic layers were washed with 0.5 N HCl and saturated aq. NaCl, dried with Na₂SO₄ and evaporated. The residue was purified by chromatography on silica gel (cyclohexane+5% NEt₃ to EtOAc+5% NEt₃) to provide the title compound as yellow solid.
HPLC: Rt_{H9}= 1.15 min; ESIMS [M-Boc]⁺= 280.3;
¹H NMR (400 MHz, DMSO-*d*₆): δ 8.93 (s, 1H), 7.00 (dd, 1H), 6.51 (dd, 1H), 5.86 (dd, 1H), 3.88 (s, 3H), 1.34 (s, 18 H).

### c) 3-(di-tert-Butoxycarbonyl-amino)-5-formyl-pyrazine-2-carboxylic acid methyl ester

A mixture of 1 g (2.64 mmol) 3-(di-tert-butoxycarbonyl-amino)-5-vinyl-pyrazine-2-carboxylic acid methyl ester and 0.332 g (3.95 mmol) sodium bicarbonate in 45 ml DCM and 15 ml MeOH was cooled to -78 °C and purged with oxygen for 5 min. The reaction mixture was treated with ozone for 40 min until the mixture turned blue. The reaction mixture was purged with oxygen for 10 min and with nitrogen for 10 min, then 0.487 ml (6.59 mmol) dimethyl sulfide was added at -78 °C and the mixture was allowed to warm to room temperature. The mixture was diluted with DCM and washed with 10% aq. sodium thiosulfate. The aq. layer was extracted with DCM and the combined organic layers were dried with Na₂SO₄ and evaporated to provide the title compound as yellow oil. The compound was used for the next step without further purification.
¹H-NMR (400 MHz, DMSO-*d*₆): δ 10.07 (s, 1H), 9.24 (s, 1H), 3.94 (s, 3H), 1.36 (s, 18H).

### d) 3-(di-tert-Butoxycarbonyl-amino)-5-difluoromethyl-pyrazine-2-carboxylic acid methyl ester

To an ice cooled solution of 550 mg (1.44 mmol) 3-(di-tert-butoxycarbonyl-amino)-5-formyl-pyrazine-2-carboxylic acid methyl ester in 20 ml DCM was added dropwise within 1 h 0.798 ml (4.33 mmol) Deoxofluor (50% in THF). Stirring was continued at 0 °C for 2.5 h then the reaction mixture was allowed to room temperature over night. Saturated aq. sodium bicarbonate was added and the mixture extracted with EtOAc, the combined organic layers were washed with sat. aq. sodium chloride, dried with Na₂SO₄ and evaporated. The residue was purified by chromatography on silica gel (cyclohexane+5% NEt₃ to cyclohexane+5% NEt₃ / EtOAc+5% NEt₃ 1:1) to provide the title compound as colorless solid.
HPLC: Rt_{H9}= 1.14 min; ESIMS [2M+Na]⁺= 829.6;
¹H-NMR (600 MHz, DMSO-*d*₆): δ 9.14 (s, 1H), 7.26 (t, 1H, CHF2), 3.92 (s, 3H), 1.33 (s, 18H).

### e) 3-(di-tert-Butoxycarbonyl-amino)-5-difluoromethyl-pyrazine-2-carboxylic acid

To a solution of 75 mg (0.186 mmol) 3-(di-tert-butoxycarbonyl-amino)-5-difluoromethyl-pyrazine-2-carboxylic acid methyl ester in 2 ml THF was added dropwise 0.205 ml (0.205 mmol) 1 N NaOH and the reaction mixture was stirred for 1.5 h. To the mixture were added 0.186 ml (0.186 mmol) 1 N HCl after stirring for 5 min toluene was added and the solvents were evaporated to provide the title compound together with sodium chloride as colorless solid. The mixture was used for coupling reactions without further purification.
HPLC: Rt_{H11}= 0.89 min; ESIMS [M-Boc]⁺= 290.0;
¹H-NMR (400 MHz, DMSO-*d*₆): δ 14.30 (br. s, 1 H), 9.10 (s, 1H), 7.25 (t, 1H, CHF2), 1.33 (s, 18 H).

### Acid-6: 5-Methoxy-3-methyl-pyridine-2-carboxylic acid

### a) 5-Methoxy-3-methyl-pyridine-2-carbonitrile

To a solution of 5-hydroxy-3-methyl-pyridine-2-carbonitrile (CAS registry 228867-86-5) (1.5 g, 11.18 mmol) and methanol (0.499 ml, 0.394 g, 12.30 mmol) in THF (100 ml) was added at 0 °C triphenylphosphine (4.44 g, 16.77 mmol) and the reaction mixture was stirred for 10 min at 0 °C. Then a solution of DIAD (3.25 ml, 3.39 g, 16.77 mmol) in THF (50 ml) was added. The reaction mixture was stirred for 18 h at rt, diluted with EtOAc and washed with water and brine. The combined aq. layers were reextracted with EtOAc, the combined organic layers dried over Na₂SO₄, filtered and the filtrate was concentrated. The title compound was obtained after flash chromatography on silica gel (cyclohexane / EtOAc gradient 0-5 min 95:5, 5-50 min 95:5 to 60:40).
HPLC: Rt_{H10}= 0.75 min; ESIMS: 149 [(M+H)⁺];
¹H NMR (400 MHz, CDCl₃): δ 8.22 (d, 1 H), 7.08 (d, 1 H), 3.92 (s, 3H), 2.55 (s, 3H).

### b) 5-Methoxy-3-methyl-pyridine-2-carboxylic acid

A solution of 5-methoxy-3-methyl-pyridine-2-carbonitrile (3.41 g, 10.20 mmol) in conc. aq. HCl soln. (10 ml) was stirred for 3.5 h at 120 °C. The reaction mixture was cooled to rt, diluted with TBME and extracted twice with water. The combined aq. layers were washed with TBME and lyophilized. The residue was dissolved in water, 1M aq. NaOH soln. was added to adjust the pH to 3 and the solution was extracted 3x with DCM. The combined organic layers were dried over Na₂SO₄, filtrated and the filtrate was concentrated to yield the title compound as a white solid which was used for the next step without further purification.
HPLC: Rt_{H10} = 0.40 min; ESIMS: 168 [(M+H)⁺];
¹H NMR (400 MHz, MeOD): δ 8.14 (d, 1H), 7.36 (d, 1H), 3.94 (s, 3H), 2.65 (s, 3H).

### Acid-7: 5-Difluoromethyl-3-methyl-pyridine-2-carboxylic acid

### a) 2-Chloro-5-difluoromethyl-3-methyl-pyridine

To a precooled solution of 6-chloro-5-methyl-pyridine-3-carbaldehyde (CAS registry 176433-43-5) (500 mg, 3.21 mmol) in DCM (15 ml) was added at -78 °C DAST (0.632 ml, 0.777 g, 4.82 mmol). The reaction mixture was stirred for 18 h at -78 °C to rt, then quenched at 0 °C with sat. aq. NaHCO₃ soln., diluted with H₂O and extracted with DCM. The organic layer was washed with H₂O, dried over Na₂SO₄, filtrated and the filtrate was concentrated. The title compound was obtained as a yellow oil after flash chromatography on silica gel (cyclohexane / EtOAc gradient 0-5 min 100:0, 5-40 min 100:0 to 80:20).
HPLC: Rt_{H10} = 0.94 min; ESIMS: 178 [(M+H)⁺];
¹H NMR (400 MHz, CDCl₃): 8.38 (d, 1H), 7.72 (d, 1H), 6.69 (t, 1H), 2.46 (s, 3H).

### b) 5-Difluoromethyl-3-methyl-pyridine-2-carbonitrile

A solution of 2-chloro-5-difluoromethyl-3-methyl-pyridine (337 mg, 1.898 mmol), Zn(CN)₂ (159 mg, 1.328 mmol) and Pd(PPh₃)₄ (132 mg, 0.114 mmol) in DMF (10 ml) was stirred for 10 min at 120 °C in a microwave, filtrated over hyflo and washed with water and brine. The combined aq. layers were reextracted with TBME, the combined org. layers were dried over Na₂SO₄, filtrated and the filtrate was concentrated. The title compound was obtained as a yellow oil after flash chromatography on silica gel (cyclohexane / EtOAc gradient 0-3 min 100:0, 3-35 min 100:0 to 80:20).
HPLC: Rt_{H10}= 0.83 min; ESIMS: 169 [(M+H)⁺];
¹H NMR (400 MHz, CDCl₃): δ 8.68 (s, 1H), 7.84 (s, 1H), 6.75 (t, 1H), 2.65 (s, 3H).

### c) 5-Difluoromethyl-3-methyl-pyridine-2-carboxylic acid

A solution of 5-difluoromethyl-3-methyl-pyridine-2-carbonitrile (209 mg, 0.787 mmol) in conc. aq. HCl soln. (2 ml) was stirred for 2h at 120 °C in a sealed tube. The reaction mixture was cooled to rt, diluted with TBME and extracted twice with water. The combined aq. layers were washed with TBME and lyophilized. The residue was dissolved in water, 1M aq. NaOH soln. was added to adjust the pH to 2 and the solution was extracted 3x with DCM. The combined organic layers were dried over Na₂SO₄, filtrated and the filtrate was concentrated to yield the title compound as a white solid which was used for the next step without further purification.
HPLC: Rt_{H10} = 0.49 min; ESIMS: 188 [(M+H)⁺];
¹H NMR (400 MHz, MeOD): δ 8.62 (s, 1H), 7.98 (s, 1H), 6.95 (t, 1H), 2.65 (s, 3H).

### Acid-8: 5-Fluoro-3-trideuteromethoxymethyl-pyridine-2-carboxylic acid

### a) 2-Chloro-5-fluoro-3-trideuteromethoxymethyl-pyridine

To a solution of (2-chloro-5-fluoropyridin-3-yl)-methanol (CAS: 870063-2-8; 950 mg, 5.88 mmol) in dry DMF (25 ml) was added sodium hydride (235 mg, 5.88 mmol, 60% in oil) at 0 °C. After 15 minutes iodomethane-D3 (1.11 g, 7.64 mmol) was added and stirring was continued at room temperature for 4 h. The reaction mixture was quenched with water and diluted with ethyl acetate. The organic phase was washed with saturated bicarbonate solution and brine, dried over sodium sulfate, filtered and evaporated. The crude brown oil was chromatographed over silica gel gel (cyclohexane:ethyl acetate) to provide the title compound.
LC-MS: Rt_{H8}= 0.87 min. (100 % purity, ESI+ 179, 181);
¹H-NMR (360 MHz, CDCl₃): 8.18 (d, 1H), 7.64 (m, 1H), 4.52 (s, 2H).

### b) 5-Fluoro-3-trideuteromethoxymethyl-pyridine-2-carbonitrile

2-Chloro-5-fluoro-3-trideuteromethoxymethyl-pyridine (700 mg, 3.92 mmol) was reacted with zinc dust, zinccyanide and (dppf)PdCl₂ catalyst in an analogous manner as in example A1 c) to afford the title compound after silica gel chromatography (cyclohexane/ethyl acetate) to provide the title compound.
TLC Rf=0.42 (3:1 cyclohexane:ethyl acetate);
LC-MS: Rt_{H8}= 0.74min. (100 % purity); API ES+ 170;
¹H-NMR (360 MHz, CDCl₃): δ 8.49 (d, 1H), 7.72 (m, 1H), 4.71 (s, 2H).

### c) 5-Fluoro-3-trideuteromethoxymethyl-pyridine-2-carboxylic acid

5-Fluoro-3-trideuteromethoxymethyl-pyridine-2-carbonitrile (150 mg, 0.887 mmol) was hydrolised in 2N NaOH in an analogous manner as in Acid 1 step d) to afford the crude title compound.
LC-MS: Rt_{H8}= 0.58 min; (100 % purity, ESI+ 189); API ES+ 189;
¹H-NMR (360 MHz, CDCl₃): δ 11.3 (broad, 1H), 8.36 (d, 1H), 8.01 (m, 1H), 5.03 (s, 2H).

### Acid-9: 5-Trideuteromethoxy-3-trideuteromethoxymethyl-pyridine-2-carboxylic acid

### a) 5-Trideuteromethoxy-3-trideuteromethoxymethyl-pyridine-2-carbonitrile

To a solution of CD30D (48 mg, 1.33 mmol) in DMSO (2 ml) was added sodium hydride (53.2 mg, 1.33 mmol, 60% in oil) followed 10 minutes later by 5-Fluoro-3-trideuteromethoxymethyl-pyridine-2-carbonitrile (150 mg, 0.887 mmol, Acid-8 b)). The reaction mixture was heated at 90 °C for 1 h. The reaction mixture was diluted with ethyl acetate and washed with water and brine. The organic layer was dried over sodium sulfate, filtered and evaporated in vacuo. The crude product was chromatographed over silica gel (cyclohexane/ethyl acetate) to provide the title compound.
TLC: Rf=0.21 (3:1 cyclohexane:ethyl acetate);
LC-MS: Rt_{H8}= 0.73 min, (93 % purity, ESI+ 185); API-ES+ 185;
¹H-NMR (400 MHz, CDCl₃): δ 8.29 (d, 1H), 7.39 (d, 1H), 4.68 (s, 2H).

### b) 5-Trideuteromethoxy-3-trideuteromethoxymethyl-pyridine-2-carboxylic acid

5-Trideuteromethoxy-3-trideuteromethoxymethyl-pyridine-2-carbonitrile (80 mg, 0.434 mmol) was hydrolised in NaOH 2N (2 ml) in an analogous manner as in Acid 1 step d) to afford the crude title compound.
LC-MS: Rt_{H8}= 0.49 min; (100 % purity, ESI+ 204); API ES+ 204;
¹H-NMR (360 MHz, CDCl₃): δ 8.15 (d, 1H), 7.70 (d, 1H), 5.03 (s, 2H).

### Acid-10: 3-Amino-5-cyano-pyridine-2-carboxylic acid

### a) 5-Bromo-3-nitro-pyridine-2-carboxylic acid tert-butyl ester

To an ice cooled solution of 4.84 g (19.59 mmol) 5-bromo-3-nitro-pyridine-2-carboxylic acid (CAS 954240-89-2) in 59 ml THF was added 239 mg (1.96 mmol) DMAP and 5.56 g (25.5 mmol) Boc₂O and the reaction mixture was heated to 60 °C for 3 h. After cooling to 0 °C half saturated aq. sodium bicarbonate was added and the mixture extracted with EtOAc. The combined organic layers were washed with water and half saturated aq. NaCl, dried with Na₂SO₄ and evaporated. The residue was purified by chromatography on silica gel (cyclohexane to cyclohexane/EtOAc 3:2) to provide the title compound as pale beige solid. HPLC: Rt_{H8}= 1.17 min; ESIMS [M+H]⁺= 304.1;
¹H-NMR (600 MHz, DMSO-*d*₆): δ 9.11 (s, 1H), 8.92 (s, 1H), 1.53 (s, 9H).

### b) 5-Cyano-3-nitro-pyridine-2-carboxylic acid tert-butyl ester

To a solution of 888 mg (2.93 mmol) 5-bromo-3-nitro-pyridine-2-carboxylic acid tert-butyl ester in 8.8 ml DMF was added 206 mg (1.76 mmol) zinc cyanide and 2 mg (0.03 mmol) zinc dust. The mixture was purged with nitrogen (3 times) 150 mg (0.293 mmol) bis(tri-tert-butylphosphine)palladium(0) were added and the mixture was heated to 80 °C for 4 h. After cooling to 0 °C water was added and the mixture extracted with EtOAc, the combined organic layers were washed with half saturated aq. NaCl, dried with Na₂SO₄ and evaporated. The residue was purified by chromatography on silica gel (cyclohexane to cyclohexane/EtOAc 1:4) to provide the title compound as beige solid.
HPLC: Rt_{H8}= 1.04 min; ESIMS [M+H]⁺= 248.0;
¹H-NMR (600 MHz, DMSO-*d*₆): δ 9.39 (s, 1H), 9.29 (s, 1H), 1.55 (s, 9H).

### c) 3-Amino-5-cyano-pyridine-2-carboxylic acid tert-butyl ester

To a mixture of 130 mg (0.522 mmol) 5-cyano-3-nitro-pyridine-2-carboxylic acid tert-butyl ester in 3 ml water was added 0.149 ml (2.61 mmol) acetic acid, the mixture was stirred at room temperature for 20 min, 454 mg (2.61 mmol) sodium dithionite were added and stirring was continued for 23 h. Additional 182 mg (1.043 mmol) sodium dithionite were added and the reaction mixture stirred for an other 48 h. The mixture was extracted with DCM, the combined organic layers were washed with water and saturated aq. NaCl, dried with Na₂SO₄ and evaporated to provide the title compound as yellow solid. The product was used for the next step without further purification.
HPLC: Rt_{H9}= 0.86 min; ESIMS [M+H]⁺= 220.2;
¹H-NMR (400 MHz, DMSO-*d*₆): δ 8.15 (d, 1 H), 7.61 (d, 1 H), 6.95 (br. s, 2H), 1.55 (s, 9H).

### d) 3-Amino-5-cyano-pyridine-2-carboxylic acid

To a mixture of 60 mg (0.274 mmol) 3-amino-5-cyano-pyridine-2-carboxylic acid tert-butyl ester and 0.358 ml (2.74 mmol) 1,3-dimethoxybenzene were added dropwise within 10 min 0.59 ml (7.66 mmol) TFA and the reaction mixture was stirred for 6 h. Toluene was added and the solvents were evaporated to provide the title compound as yellow solid. The product was used for the next step without further purification.
HPLC: Rt_{H9}= 0.38 min; ESIMS [M+H]⁺= 164.1;
¹H-NMR (400 MHz, DMSO-*d*₆): δ 13.05 (br. s, 1H), 8.16 (d, 1H), 7.64 (d, 1H), 7.08 (br. s, 2H).

### Acid-11: 3-Amino-5-difluoromethyl-pyridine-2-carboxylic acid

### a) 5-Difluoromethyl-3-nitro-pyridine-2-carboxylic acid tert-butyl ester

The title compound was prepared by an analogous reaction sequence to Acid-5 using 5-bromo-3-nitro-pyridine-2-carboxylic acid instead of 3-amino-5-chloro-pyrazine-2-carboxylic acid methyl ester in step a) and omitting step b).
HPLC: Rt_{H9}= 1.07 min; ESIMS [M+H]⁺= 275.3;
¹H NMR (600MHz, DMSO-d₆): δ 9.18 (s, 1H), 8.82 (s, 1H), 7.31 (t, 1H, CHF2), 1.55 (s, 9H).

### b) 5-Difluoromethyl-3-nitro-pyridine-2-carboxylic acid

In a mixture of 5 ml DCM and 2.5 ml TFA was dissolved 345 mg (1.26 mmol) 5-difluoromethyl-3-nitro-pyridine-2-carboxylic acid tert-butyl ester and the reaction mixture was stirred for 4 h. Toluene was added and the solvents were evaporated to provide the title compound as colorless solid.
HPLC: Rt_{H9}= 0.31 min; ESIMS [2M-H]⁻= 435.3;
¹H-NMR (600 MHz, DMSO-*d*₆): δ 14.59 (br. s, 1H), 9.16 (s, 1H), 8.80 (s, 1H), 7.31 (t, 1H, CHF2).

### c) 3-Amino-5-difluoromethyl-pyridine-2-carboxylic acid

To a solution of 265 mg (1.22 mmol) 5-difluoromethyl-3-nitro-pyridine-2-carboxylic acid in EtOH was added 50 mg Raney-Nickel (Degussa B113W) and the reaction mixture was kept shaking under a hydrogen atmosphere for 16 h. The catalyst was filtered off (Celite) and washed with EtOH and the filtrate was evaporated to provide the title compound as off-white solid.
HPLC: Rt_{H9}= 0.34 min; ESIMS [M+H]⁺= 189.2;
¹H-NMR (600 MHz, DMSO-*d*₆): δ 7.98 (s, 1H), 7.39 (s, 1H), 7.09 (t, 1H, CHF2), 7.02 (br. s, 2H).

### Acid-12: 3-Chloro-5-difluoromethoxy-pyridine-2-carboxylic acid

### a) 3-Chloro-5-difluoromethoxy-pyridine-2-carbonitrile

To a solution of 3-chloro-5-hydroxy-pyridine-2-carbonitrile (330 mg, 2.03 mmol) in DMF (10 ml) was added K₂CO₃ (1.68 g, 12.2 mmol) and sodium chlorodifluoroacetate (1.29 g, 8.1 mmol) and the reaction mixture was heated at 100 °C for 10 min. The cold reaction mixture was diluted with TBME and washed with water and brine, dried over MgSO₄, filtered and concentrated. The title compound was obtained after flash column chromatography on silica gel (hexane to hexane-EtOAc 1:1) as a yellow oil: TLC (hexane-EtOAc 2:1): Rf =0.54; HPLC: Rt_{H5}= 0.966 min; ESIMS: 203, 205 [(M-H)⁻];
¹H NMR (360 MHz, CDCl₃): δ 8.41 (d, 1 H), 7.61 (d, 1 H), 6.60 (t, 1 H).

### b) 3-Chloro-5-difluoromethoxy-pyridine-2-carboxylic acid

To a solution of 3-chloro-5-difluoromethoxy-pyridine-2-carbonitrile (470 mg, 2.29 mmol) in dioxane (18 ml) was added 1 N NaOH (8.0 ml, 8 mmol) and the reaction mixture was stirred overnight at 70 °C. The cold reaction mixture was acidified with 4N HCl and evaporated to dryness. The residue was suspended in CH₂Cl₂-MeOH 8:1, filtered and concentrated to provide the title compound as a yellow oil.
HPLC: Rt_{H5}= 0.664 min; ESIMS: 222, 224 [(M-H)⁻];
¹H NMR (360 MHz, CDCl₃): δ 8.38 (br s, 1H), 7.82 (d, 1H), 7.06 (t, 1H).

### Acid 13: 5-cyano-3-methyl-pyridine-2-carboxylic acid

### a) 5-Bromo-3-methyl-pyridine-2-carboxylic acid tert-butyl ester

To a solution of 10.20 g (47.2 mmol) 5-bromo-3-methyl-pyridine-2-carboxylic acid and 20.61 g (94 mmol) di-tert-butyldicarbonate in 100 ml THF were added 0.577 g DMAP. Evolution of CO₂ started immediately and the mixture was stirred for 2 h at RT. TBME and sat aq NaHCO3 were added. The layers were separated and the organic layer washed with sat aq NaHCO3 and brine, and dried with MgSO₄.H₂O. Chromatography on silica gel (hexanes/ EtOAc 1-7%) provided the title compound as a yellow liquid.
HPLC: Rt_{H3}= 3.018 min; ESIMS [M+H]⁺= 272, 274 (1 Br); ¹H-NMR (360 MHz, CDCl₃): δ 8.59 s, 1H), 7.77 (s, 1H), 2.52 (s, 3H), 1.65 (s, 9H).

### b) 5-Bromo-3-methyl-pyridine-2-carboxylic acid tert-butyl ester

A mixture of 6.0 g (22.05 mmol) 5-bromo-3-methyl-pyridine-2-carboxylic acid tert-butyl ester, 1.813 g (15.43 mmol) Zn(CN)₂, 0.144 g Zn powder (2.205 mmol) and 0.571 g (0.551 mmol) Pd₂(dba)₃.CHCl₃ were suspended in 10 ml DMF under nitrogen atmosphere. tBu₃P (0.321 ml, 1.323 mmol) was added and the mixture was stirred for 5 h at 60 °C. After being cooled down the mixture was diluted with TBME, filtered over celite and washed with brine three times. The crude product was purified by column chromatography on silica gel (hexanes/ EtOAc 5-15%) to give the title compound as an off white solid. TLC (hexanes/ EtOAc 3:1): Rf = 0.31; HPLC: Rt_{H3}= 2.431 min; ESIMS [M+Na]⁺= 241; ¹H-NMR (360 MHz, CDCl₃): δ 8.78 (s, 1 H), 7.88 (s, 1 H), 2.56 (s, 3H), 1.67 (s, 9H); Ft-IR: 2231 cm⁻¹ (CN).

### c) 5-cyano-3-methyl-pyridine-2-carboxylic acid

To a solution of 8.50 g (38.9 mmol) 5-cyano-3-methyl-pyridine-2-carboxylic acid tert-butyl ester in 51 ml (389 mmol) 1,3-dimethoxybenzene were added 85 ml TFA and stirred for 6.5 h. The reaction mixture was diluted with toluene and evaporated. The residue was taken up in toluene and evaporated (2x). The product was crystallized from TBME/hexanes to give the title compound as a white powder. HPLC: Rt_{H1}= 2.314 min; ESIMS [M+Na]⁺= 163; ¹H-NMR (360 MHz, CDCl₃): δ 8.77 (s, 1 H), 8.07 (s, 1 H), 2.87 (s, 3H).

### Acid-14: 3-Chloro-5-trideutero-methoxy-pyridine-2-carboxylic acid

**a) 3-Chloro-5-hydroxy-pyridine-2-carbonitrile**

To an argon degased solution of acetic acid 5,6-dichloro-pyridin-3-yl ester (4.87 g, 23.66 mmol) in DMF (50 ml) was added Zn(CN)₂ (1.278 g, 10.88 mmol), zinc-dust (0.07 g, 1.06 mmol) and DPPF PdCl₂ (0.996 g, 1.18 mmol) and the resulting reaction mixture was heated at 150 °C for 18 h. The reaction mixture was diluted with TBME and water, filtered over Celite and the product was extracted with TBME. Combined extracts were washed with brine, dried over MgSO₄, filtered and concentrated. The title compound was obtained after recrystallization from EtOAc-hexane as a beige solid: TLC (CH₂Cl₂-MeOH 19:1): Rf =0.22; HPLC: Rt_{H5}= 0.677 min; ESIMS: 153,155 [(M-H)⁻];
¹H NMR (360 MHz, CD₃OD): δ 8.19 (d, 1 H), 7.41 (d, 1H).

### b) 3-Chloro-5-trideutero-methoxy-pyridine-2-carbonitrile

To a solution of 3-chloro-5-hydroxy-pyridine-2-carbonitrile (0.855 g, 5.5 mmol) in THF (50 ml) was added at 0 °C CD₃OD (0.292 ml, 7.19 mmol) und PPh₃ (2.176 g, 8.30 mmol) and afterwards dropwise DIAD (1.613 ml, 8.30 mmol). After stirring for 1 h at 0-5 °C the reaction mixture was concentrated. The title compound was obtained after flash column chromatography on silica gel (toluene-EtOAc 3:1) as a colorless solid: TLC (toluene-EtOAc 1:1): Rf=0.57;
HPLC: Rt_{H5}= 0.866 min; ESIMS: 172, 174 [(M+H)⁺]; ¹H NMR (360 MHz, CDCl₃): δ 8.30 (d, 1 H), 7.31 (d, 1H).

### c) 3-Chloro-5-trideutero-methoxy-pyridine-2-carboxylic acid

To a solution of 3-chloro-5-trideutero-methoxy-pyridine-2-carbonitrile (760 mg, 4.43 mmol) in dioxane (10 ml) was added 4N NaOH (11.07 ml, 44.3 mmol) and the reaction mixture was stirred overnight at 85 °C. The cold reaction mixture was acidified with 4N HCl and extracted with EtOAc. Combined extracts were washed with brine, dried over MgSO₄, filtered and concentrated. The title compound was obtained after crystallization from EtOAc-diisopropylether as a colorless solid.
HPLC: Rt_{H5}= 0.538 min; ESIMS: 191,193 [(M+H)⁺];
¹H NMR (360 MHz, CDCl₃): δ 8.21 (d, 1 H), 7.38 (d, 1H).

### Acid-15: 5-Difluoromethoxy-3-methyl-pyridine-2-carboxylic acid

### a) 5-Difluoromethoxy-3-methyl-pyridine-2-carbonitrile

A solution of 5-hydroxy-3-methyl-pyridine-2-carbonitrile (CAS registry 228867-86-5) (228 mg, 1.70 mmol), sodium chlorodifluoroacetate (CAS registry 1895-39-2) (518 mg, 3.40 mmol) and K₂CO₃ (705 mg, 5.10 mmol) in DMF (7 ml) was stirred for 0.5 h at 100 °C. The reaction mixture was diluted with EtOAc and washed with sat. aq. NH₄Cl soln. and brine. The aq. layers were reextracted with EtOAc, the combined organic layers dried over Na₂SO₄, filtrated and the filtrate was concentrated. The title compound was obtained as a colourless oil after flash chromatography on silica gel (cyclohexane / EtOAc gradient 0-3 min 95:5, 3-35 min 95:5 to 60:40).
HPLC Rt_{H10} = 0.87 min; ESIMS: 185 [(M+H)⁺];
¹H NMR (400 MHz, CDCl₃): 8.40 (d, 1H), 7.45 (d, 1H), 6.64 (t, 1H), 2.61 (s, 3H).

### b) 5-Difluoromethoxy-3-methyl-pyridine-2-carboxylic acid

To a solution of 5-difluoromethoxy-3-methyl-pyridine-2-carbonitrile (145 mg, 0.787 mmol) in EtOH (5 ml) was added 1M aq. NaOH soln. (2.5 ml). The reaction mixture was stirred for 7h at 70 °C, then for 9h at room temperature. It was diluted with Et₂O and twice extracted with water. The combined aq. layers were reextracted with Et₂O, acidified to pH 2 with 1M aq. HCl and twice extracted with TBME. The combined organic layers were dried over Na₂SO₄, filtrated and the filtrate was concentrated to yield the title compound as a white solid which was used for the next step without further purification.
HPLC Rt_{H10}= 0.61 min; ESIMS: 204 [(M+H)⁺];
¹H NMR (400 MHz, MeOD): 8.32 (d, 1H), 7.61 (d, 1H), 7.06 (t, 1H), 2.64 (s, 3H).

### Acid-16: 5-Chloro-4,6-dideutero-3-trideuteromethyl-pyridine-2-carboxylic acid

A suspension of 500 mg (2.91 mmol) 5-chloro-3-methyl-pyridine-2-carboxylic acid (CAS Nr.: 886365-46-4) in 9 ml of D₂O (99,96% D) was treated with 1 ml of a 40% solution of NaOD in D₂O. The homogeneous solution was heated in a 100 ml Teflon vessel with a Synthos 3000 Microwave apparatus. The mixture was heated at 160 °C for 5 h and cooled down. 1H-NMR and MS analyses of the product showed that deuteration had progressed to a high degree. Only minor amounts of tetradeutero derivatives were present. The reaction mixture was acidified to pH3 with 2N HCl and extracted with EtOAc. The org. phase was dried with MgSO₄.H₂O and evaporated to give the title compound as a white solid, pure enough for further transformations.
HPLC: Rt_{H1}= 2.820 min; ESIMS [M+H]⁺= 177 (5D);
¹H-NMR (360 MHz, D₂O): δ non deuterated impurities.

### Acid-17: Sodium; 4-difluoromethyl-6-methoxy-pyridazine-3-carboxylate

### a) 2-Diazo-4,4-difluoro-3-oxo-butyric acid ethyl ester

To a solution of 4,4-difluoro-3-oxo-butyric acid ethyl ester (5.0 g, 29 mmol) and 4-acetylamino-benzenesulfonyl azide (7.95 g, 32 mmol) in ACN (50 mL) was added at 0 °C NEt₃ (6.1 mL, 43,8 mmol) within 30 min. The reaction mixture was stirred for 2 h at 0-5 °C and overnight at 25 °C, than diluted with TBME and filtered. The filtrate was washed with 10% aq. NaH₂PO₄ and brine, dried over MgSO₄, filtered and concentrated. The title compound was obtained after flash column chromatography on silica gel (hexane to hexane-TBME 1:1) as a yellow oil.
TLC (hexane-TBME 1:1): Rf =0.46;
¹H NMR (360 MHz, CDCl₃): δ 6.62 (t, 1H), 4.38 and 4.24 (q, 2H),1.38 and 1.31 (t, 3H).

### b) (E)-4-Diazo-3-difluoromethyl-pent-2-enedioic acid 5-ethyl ester 1-methyl ester

To a solution of 2-diazo-4,4-difluoro-3-oxo-butyric acid ethyl ester 0.5 g, 2.6 mmol) in Et₂O (10mL) was added methoxycarbonylmethylen-triphenylphosphoran (1.3 g, 3.9 mmol) and the reaction mixture was stirred for 3 days at 25 °C. The reaction mixture was filtered through a plug of silica gel and concentrated to provide the title compound after purification by flash column chromatography on silica gel (hexane to hexane-TBME 1:1) as a yellow oil.
TLC (hexane-TBME 1:1): Rf =0.60;
¹H NMR (360 MHz, CDCl₃): δ 6.82 (t, 1H), 6.32 (s, 1H), 4.29 (q, 2H), 3.79 (s, 3H), 1.34 (t, 3H).

### c) 4-Difluoromethyl-6-methoxy-pyridazine-3-carboxylic acid ethyl ester

To a solution of (E)-4-diazo-3-difluoromethyl-pent-2-enedioic acid 5-ethyl ester 1-methyl ester (0.18 g, 0.78 mmol) in Et₂O (10 ml) was added PPh₃ (0.31 g, 1.18 mmol) and the reaction mixture was stirred for 3 days at 25 °C. The reaction mixture was concentrated and purified by flash column chromatography on silica gel (hexane to hexane-TBME 1:1) to obtain the title compound as a yellow oil.
TLC (hexane-TBME 1:1): Rf=0.31;
HPLC: Rt_{H5}= 0.877 min;
¹H NMR (360 MHz, CDCl₃): δ 7.41 (t, 1H), 7.13 (s, 1H), 4.52 (q, 2H), 4.25 (s, 3H), 1.45 (t, 3H).

### d) Sodium; 4-difluoromethyl-6-methoxy-pyridazine-3-carboxylate

To a solution of 4-difluoromethyl-6-methoxy-pyridazine-3-carboxylic acid ethyl ester (0.13 g, 0.56 mmol) in dioxane (2 ml) was added 4N NaOH (0.7 ml, 2.8 mmol) and the reaction mixture was stirred for 0.5 h at 25 °C. After addition of 4N HCl (0.56 mL, 2.24 mmol) the reaction mixture was evaporated to dryness. The crude product was re-dissolved in DMF and concentrated again to provide the title compound as a light yellow solid, which was used as such in the next step.
HPLC: Rt_{H5}= 0.420 min; ESIMS: 203 [(M-H)⁻].

### Acid-18: 5-Cyano-3-trideuteromethoxymethyl-pyridine-2-carboxylic acid

### a) 5-Chloro-3-trideuteromethoxymethyl-pyridine-2-carboxylic acid

5-Chloro-3-trideuteromethoxymethyl-pyridine-2-carboxylic acid was prepared from (2,5-dichloro-pyridin-3-yl)-methanol in analogous manner to the sequence of Acid-1 step a) to d) using trideuteromethyliodide instead of methyliodide in the alkylation step b).
LC-MS: Rt_{H8}= 0.77 min. (100 % purity, ES+ 205, 207), API ES- 203, 205;
¹H-NMR (400 MHz, CDCl₃): δ 8.47 (d, 1 H), 8.27 (m, 1 H), 4.87 (s, 2H).

### b) 5-Chloro-3-trideuteromethoxymethyl-pyridine-2-carboxylic acid benzyl ester

A mixture of 5-Chloro-3-trideuteromethoxymethyl-pyridine-2-carboxylic acid (100 mg, 0.489 mmol) and 2-benzyl-1,3-dicyclohexyl-isourea (169 mg, 0.538 mmol) in toluene (2 ml) was stirred at 90 °C for 3 . The reaction mixture was filtered and evaporated in vacuo. Chromatography over silica gel (cyclohexane/ethyl acetate) afforded the title compound. LC-MS: Rt_{H8}= 1.15 min. (100 % purity, ES+ 295, 297);
¹H-NMR (400 MHz, CDCl₃): δ 8.85 (d, 1H), 8.10 (d, 1H), 7.50 (m, 2H), 7.40 (m, 3H), 5.46 (s, 2H), 4.82 (s, 2H).

### c) 5-Cyano-3-trideuteromethoxymethyl-pyridine-2-carboxylic acid benzyl ester

5-Chloro-3-trideuteromethoxymethyl-pyridine-2-carboxylic acid benzyl ester (120 mg, 0.407 mmol) was reacted with zinc dust (2.66 mg, 0.04 mmol), zinc cyanide (28.7 mg, 0.244 mmol) and bis(tri-t-butylphosphine)palladium(0) catalyst (20.81 mg, 0.041 mmol) in an analogous manner at 80 °C for 3 h as in Acid 1 step c) to afford the title compound after silica gel chromatography (cyclohexane/ethyl acetate). TLC Rₜ=0.40 (3:1 cyclohexane:ethyl acetate); LC-MS: Rt_{H8}= 1.04. (100 %, ES+ 286);
¹H-NMR (400 MHz, CDCl₃): δ 8.87 (d, 1H), 8.39 (d, 1H), 7.50 (m, 2H), 7.40 (m, 3H), 5.47 (s, 2H), 4.82 (s, 2H).

### d) 5-Cyano-3-trideuteromethoxymethyl-pyridine-2-carboxylic acid

A solution of 5-Cyano-3-trideuteromethoxymethyl-pyridine-2-carboxylic acid benzyl ester (50 mg, 0.175 mmol) in ethanol (1.8 ml) was hydrogenated for 18 hours over Pd/C (10%, 18.65 mg) at room temperature and atmospheric pressure. The reaction mixture was filtered and evaporated in vacuo. The residue was partitioned between diethyl ether and 2N NaOH solution. The aqueous phase was set acidic with 2N HCl solution and was extracted with ethyl acetate. The organic phase was washed with brine, dried over sodium sulfate, filtered and concentrated in vacuo to provide the title compound as glassy solid.
LC-MS: Rt_{H8}= 0.48 min. (100 % purity, ES- 194);
¹H-NMR (400 MHz, CDCl₃): δ 8.81 (d, 1 H), 8.60 (m, 1H), 5.05 (s, 2H).

### Acid-19: 3-Chloro-5-difluoromethyl-pyridine-2-carboxylic acid

### a) 5-Bromo-3-chloro-pyridine-2-carboxylic acid tert-butyl ester

To an ice cooled solution of 11.82 g (50 mmol) 5-bromo-3-chloro-pyridine-2-carboxylic acid (CAS 1189513-51-6) in 150 ml THF was added 611 mg (5 mmol) DMAP and 14.19 g (65 mmol) Boc₂O and the reaction mixture was heated to 60 °C for 3 h. After cooling to 0 °C half saturated aq. sodium bicarbonate was added and the mixture extracted with EtOAc. The combined organic layers were washed with half saturated aq. NaCl, dried with Na₂SO₄ and evaporated. The residue was purified by chromatography on silica gel (cyclohexane to cyclohexane/EtOAc 1:1) to provide the title compound as colorless oil.
HPLC: Rt_{H8}= 1.22 min; ESIMS [M-tBu]⁺= 237.8;
¹H-NMR (600 MHz, DMSO-*d*₆): δ 8.73 (d, 1H), 8.52 (d, 1H), 1.55 (s, 9H).

### b) 3-Chloro-5-vinyl-pyridine-2-carboxylic acid tert-butyl ester

A mixture of 1.755g (6 mmol) 5-bromo-3-chloro-pyridine-2-carboxylic acid tert-butyl ester and 884 mg (6.6 mmol) potassium trifluoro(vinyl)borate in 18 ml dioxane was purged with nitrogen, 1.67 ml (12 mmol) triethylamine and 153 mg (0.3 mmol) bis(tri-tert-butylphosphine)palladium(0) were added and the mixture was heated to 80 °C for 0.5 h. After cooling to room temperature and addition of EtOAc the mixture was filtered through Hyflo and the filtrate was evaporated. The residue was purified by chromatography on silica gel (cyclohexane to cyclohexane/EtOAc 7:3) to provide the title compound as pale yellow oil. HPLC: Rt_{H8}= 1.13 min; ESIMS [M-tBu]⁺= 184.0;
¹H-NMR (600 MHz, DMSO-*d*₆): δ 8.64 (s, 1 H), 8.24 (s, 1 H), 6.79 (dd, 1 H), 6.18 (d, 1 H), 5.56 (d, 1 H), 1.55 (s, 9H).

### c) 3-Chloro-5-difluoromethyl-pyridine-2-carboxylic acid

The title compound was prepared by an analogous reaction sequence to Acid-5 steps c) and d) using 3-chloro-5-vinyl-pyridine-2-carboxylic acid tert-butyl ester instead of 3-(di-tert-butoxycarbonyl-amino)-5-vinyl-pyrazine-2-carboxylic acid methyl ester [Acid-5 step c)], followed by cleavage of the tert.-butyl ester in a manner analogous to the procedure of Acid-11 step b).
HPLC: Rt_{H9}= 0.41 min; ESIMS [M+H]⁺= 207.8;
¹H NMR (600 MHz, DMSO-d₆): 14.30 (br. s, 1H), 8.78 (s, 1H), 8.34 (s, 1H), 7.20 (t, 1H, CHF2).

### Acid-+20: 3-Chloro-5-trideuteromethoxy-dideuteromethyl-pyrrolo[2,3-b]pyridin-6-carboxylic acid

### a) 1-Triisopropylsilanyl-1H-pyrrolo[2,3-b]pyridine-5-deuterocarbaldehyde

To a solution of 1-triisopropylsilanyl-1H-pyrrolo[2,3]pyridine-5-bromide (18.8 g, 53.2 mmol, CAS: 858116-66-2) was added dropwise butyllithium (23.4 ml, 58.5 mmol, 2.5 molar in hexane) at -78 °C under a nitrogen atmosphere. After stirring for 45 minutes at this temperature deutero-D1-DMF (6.26 ml, 80 mmol, 98% from Armar) in THF (5 ml) was added slowly and the cooling bath was removed 15 minutes after the addition was complete. The reaction was quenched at 0 °C by adding aq. 1 N acetic acid (5 ml) and was diluted with ethyl acetate. The organic phase was washed with saturated sodium bicarbonate solution and brine, dried over sodium sulfate, filtered and evaporated. 17.1 g (94 % yield). TLC Rf = 0.55 (5:1 cyclohexane:ethyl acetate). LC-MS Rt_{H9}= 1.54 min. (89 % purity, ES+ 304), API MS ES+ 304. ¹H-NMR (400 MHz, CDCl₃): 8.79 (d, 1 H), 8.40 (d, 1 H), 7.42 (d, 1H), 6.72 (d, 1 H), 1.89 (septett, 3H), 1.15 (d, 18H). Used crude in the next step.

### b) (1-Triisopropylsilanyl-1H-pyrrolo[2,3-b]pyridine-5-yl)-dideuteromethanol

To a solution of 1-triisopropylsilanyl-1H-pyrrolo[2,3]pyridine-5-deuterocarbaldehyde (17.1 g, 50.1 mmol) in ethanol (250 ml) was added sodium borodeuteride (2.6 g, 62:2 mmol) at room temperature. Stirring was continued for 2 h and the the reaction was carefully quenched with 1 N acetic acid. The reaction mixture was diluted with ethyl acetate and washed with sat. sodium bicarbonate solution and brine, dried over sodium sulfate, filtered and evaporated. 18 g oil. Silica gel chromatography (89:11 cychlohexane:ethyl acetate) afforded the title compound as a white solid. 12.55 g (82 % yield). TLC Rf= 0.46 (2:1 cyclohexane:ethyl acetate). LC-MS Rt_{H9}= 1.40 min. (100% purity, ES+ 307). API MS ES+ 307. ¹H-NMR (400 MHz, CDCl₃): 8.29 (d, 1H), 7.91 (d, 1H), 7.35 (d, 1 H), 6.57 (d, 1H), 1.88 (septett, 3H), 1.15 (d, 18H).

### c) 5-Trideuteromethoxy-dideuteromethyl-1-triisopropylsilanyl-1H-pyrrolo[2,3-b]pyridine

To a solution of (1-Triisopropylsilanyl-1H-pyrrolo[2,3-b]pyridine-5-yl)-dideuteromethanol (5 g, 16.31 mmol) in dry DMF (125 ml) was added sodium hydride (718 mg, 17.94 mmol, 60% in oil) at 0° C. After 15 minutes D3-methyl iodide (1.354 ml, 21.21 mmol) was added and stirring was continued at room temperature for 3 h. The reaction mixture was quenched with water and diluted with ethyl acetate. The organic phase was washed with sat. sodium bicarbonate solution and brine, dried over sodium sulfate, filtered and evaporated. 4 g yellow oil. Silica gel chromatography (85:15 cychlohexane:ethyl acetate) afforded the title compound. 3.235 g (61.3 % yield). TLC Rf = 0.55 (2:1 cyclohexane:ethyl acetate). LC-MS Rt_{H8}= 1.68 min. (96 % purity, ES+ 324). ¹H-NMR (400 MHz, CDCl₃): 8.26 (d, 1H), 7.88 (d, 1H), 7.33 (d, 1H), 6.56 (d, 1H), 1.88 (septett, 3H), 1.15 (d, 18H).

### d) 5-Trideuteromethoxy-dideuteromethyl-1H-pyrrolo[2,3-b]pyridine

To a solution of 5-trideuteromethoxy-dideuteromethyl-1-triisopropylsilanyl-1H-pyrrolo[2,3-b]pyridine (3.253 g, 10.05 mmol) in dry THF (20 ml) was added TBAF 1M in THF (10.56 ml, 10.56 mmol). The reaction was stirred for 18 h at room temperature. The reaction mixture was poured into water and extracted with ethyl acetate. The organic phase was washed with brine, dried over sodium sulfate, filtered and evaporated. 4.31 g. Silica gel chromatography ( cyclohexane/ethyl acetate) afforded the title compound. 791 mg (47.1 % yield). TLC Rf = 0.13 (1:2 cyclohexane:ethyl acetate). LC-MS Rt_{H8}= 0.54 min. (100 % purity, ES+ 168). API MS ESI+ 168. ¹H-NMR (400 MHz, CDCl₃): 10.50 (broad s, 1H), 8.36 (d, 1H), 7.98 (d, 1 H), 7.40 (m, 1H), 6.53 (m, 1H).

### e) 5-Trideuteromethoxy-dideuteromethyl-1H-pyrrolo[2,3-b]pyridine 7-oxide

To a solution of 5-Trideuteromethoxy-dideuteromethyl-1H-pyrrolo[2,3-b]pyridine (780 mg, 4.66 mmol) in DME (20 ml) was added m-CPBA (1127 mg, 6.53 mmol) at 0 °C. Stirring was continued at room temperature over night. The solvent was removed in vacuo and the crude product was suspended in water and the pH was adjusted to 9 with sat. potassium carbonate solution. Stirring was continued and the aqueous solution was saturated with sodium chloride and extracted with ethyl acetate. The organic phase was dried over sodium sulfate, filtered and evaporated. 988 mg brown oil which was used without purification in the next step. LC-MS Rt_{H8}= 0.52 min. (73 % purity, ES+ 184). API MS ESI+ 184. ¹H-NMR (400 MHz, CDCl₃): 12.81 (broad s, 1 H), 8.26 (d, 1H), 7.68 (d, 1H), 7.41 (m, 1H), 6.55 (m, 1H).

### f) (6-Bromo-5-trideuteromethoxy-dideuteromethyl-pyrrolo[2,3-b]pyridin-1-yl)-phenyl-methanone

Solutions of benzoyl bromide (1.319 ml, 11.19 mmol) in dichloromethane (2 ml) and HMDS (0.938 ml, 4.48 mmol) in dichloromethane (1 ml) were simultaneously added dropwise to a solution of 5-trideuteromethoxy-dideuteromethyl-1H-pyrrolo[2,3-b]pyridine 7-oxide (988 mg, 4.48 mmol) in dichloromethane (3 ml) under argon atmosphere at rt over 30 minutes. Stirring was continued at room temperature over night. White precipitation. After 18 h stirring the mixture was washed with sat. sodium bicarbonate solution and brine, dried over magnesium sulfate, filtered and evaporated. 652 mg brown oil. Silica gel chromatography (cyclohexane /EtOAc) afforded the title compound. 326 mg (21 % yield). LC-MS Rt_{H8}= 1.27 min. (90 % purity, ES+ 350/352). ¹H-NMR (400 MHz, CDCl₃): 8.01(s, 1H), 7.91 (dd, 2H), 7.77 (d, 1H), 7.64 (t, 1 H), 7.53 (t, 2H), 6.56 (d, 1H).

### g) 6-Bromo-5-trideuteromethoxy-dideuteromethyl-pyrrolo[2,3-b]pyridin

To a solution of (6-Bromo-5-trideuteromethoxy-dideuteromethyl-pyrrolo[2,3-b]pyridin-1-yl)-phenyl-methanone (320 mg, 0.91 mmol) in methanol (8 ml) was added 2M NaOH solution (4.57 ml, 9.14 mmol) and the reaction mixture was stirred at rt for 2 days. The white solid formed in the reaction was filtered off and dried. 124 mg. The filtrate was evaporated in vacuo, diluted with ethyl acetate and washed with sat. sodium bicarbonate solution and brine. The organic layer was dried over sodium sulfate, filtered and evaporated. 55 mg. Combined solids: 179 mg (80% yield). LC-MS Rt_{H8}= 0.84 min. (85 % purity, ES+ 246/248). ¹H-NMR (400 MHz, CDCl₃): 10.78 (broad s, 1H), 8.06(s, 1H), 7.44 (m, 1H), 6.53 (m, 1H).

### h) 6-Cyano-5-trideuteromethoxy-dideuteromethyl-pyrrolo[2,3-b]pyridin

A mixture of 6-Bromo-5-trideuteromethoxy-dideuteromethyl-pyrrolo[2,3-b]pyridin (120 mg, 0.488 mmol), zinc (0.319 mg, 4.88 µmol) and zinc cyanide (34.4 mg, 0.293 mmol) in dry DMF (1.5 ml) was degassed with argon in a 4 ml microwave vial for 20 minutes. Bis(tri-t-butylphosphine)palladium(0) (24.92 mg, 0.049 mmol) was added and the vial was sealed and heated at 80 °C for 4 h. The reaction mixture was poured into a water/ice/ethyl acetate mixture and the organic layer was washed twice with brine, dried over sodium sulfate, filtered and evaporated. Silica gel chromatography (cyclohexane /EtOAc) of the crude product (164 mg) afforded the title compound as a white solid. 71 mg (76 % yield). LC-MS Rt_{H8}= 0.73 min. (100 % purity, ES+ 193). ¹H-NMR (400 MHz, CDCl₃): 10.14 (broad s, 1H), 8.15 (s, 1H), 7.66 (m, 1H), 6.64 (m, 1H).

### i) 5-Trideuteromethoxy-dideuteromethyl-pyrrolo[2,3-b]pyridin-6-carboxylic acid

A suspension of 6-cyano-5-trideuteromethoxy-dideuteromethyl-pyrrolo[2,3-b]pyridin (70 mg, 0.364 mmol) in 2M NaOH (2 ml) was stirred at 100 °C for 18 h. The reaction mixture was washed with diethyl ether and the aqueous layer was set acidic (pH 6-7) with 2M HCl solution. The white solid formed was filtered off and washed with water. 63 mg white solid. The filtrate was extracted with ethyl acetate, washed with brine, dried ofer sodium sulfate, filtered and evaporated. 12 mg white solid. Combined solids: 75 mg white solid (98% yield). LC-MS Rt_{H8}= 0.60 min. (100 % purity, ES+ 212). ¹H-NMR (400 MHz, CDCl₃): 8.48 (s, 1H), 7.60 (m, 1H), 6.67 (m, 1H).

### j) 3-Chloro-5-trideuteromethoxy-dideuteromethyl-pyrrolo[2,3-b]pyridin-6-carboxylic acid

To a solution of 5-Trideuteromethoxy-dideuteromethyl-pyrrolo[2,3-b]pyridin-6-carboxylic acid (70 mg, 0.331 mmol) in dry DMF (12 ml) was added NCS (44.3 mg, 0.331 mmol) and the reaction mixture was stirred at rt for 20 hours under argon. The reaction mixture was diluted with ethyl acetate and washed with brine. The precipitate formed was filtered off, washed with ethyl acetate and dried in vacuo. The residue (300 mg) was stirred in water and the insoluble part was filtered off affording 20 mg (25 % yield) of a light yellow solid. LC-MS Rt_{H8}= 0.78 min. (100 % purity, ES- 244). ¹H-NMR (400 MHz, DMSO-D₆): 13.04 (s, 1H), 12.28 (broad s, 1 H, NH), 8.06 (s, 1 H), 7.91 (d, 1 H).

### Acid-21: 5-Trideuteromethoxy-3-methyl-pyridine-2-carboxylic acid

5-Trideuteromethoxy-3-methyl-pyridine-2-carboxylic acid was prepared by an analogous reaction sequence to Acid-6 using CD₃OD instead of methanol in the first step; HPLC: Rt_{H10} = 0.42 min; ESIMS: 171 [(M+H)⁺];
¹H NMR (400 MHz, MeOD): δ 8.13 (d, 1H), 7.36 (d, 1H), 2.65 (s, 3H).

### Acid-22: 5-Fluoro-3-methyl-pyridine-2-carboxylic acid

### a) 5-Fluoro-3-methyl-pyridine-2-carbonitrile

A soln. of 2-chloro-5-fluoro-3-methyl-pyridine (CAS 38186-84-4, 408 mg, 2.750 mmol), Zn(CN)₂ (230 mg, 1.923 mmol) and Pd(PPh₃)₄ (190 mg, 0.165 mmol) in DMF (8 ml) was stirred at 120°C for 0.5 h in a microwave. The reaction mixture was filtered over hyflo, diluted with TBME and H₂O and extracted with brine. The aq. phases were reextracted with TBME, the combined org. phases were dried over Na₂SO₄, filtered and concentrated. Flash chromatography on silica gel (hexane-EtOAc 100:0 to 80:20) yielded the title compound. HPLC: Rt_{H12}= 0.72 min; ¹H NMR (400 MHz, CDCl₃): δ 8.42 (d, 1H), 7.42-7.39 (m, 1H), 2.61 (s, 3H).

### b) 5-Fluoro-3-methyl-pyridine-2-carboxylic acid

5-Fluoro-3-methyl-pyridine-2-carbonitrile (254 mg, 1.866 mmol) in conc. aq. HCl (1.5 ml) was stirred in a sealed glass vial at 120°C for 2 h. The reaction mixture was basified with solid NaOH and extracted twice with TBME. The combined org. phases were washed with H₂O. The combined aq. phases were acidified to pH 2 with 2M aq. HCl, and were three times extracted with TMBE, dried over Na₂SO₄, filtered and concentrated. The crude product was used in the next step without further purification.
HPLC: Rt_{H12}= 0.48 min; ESIMS [M+H]⁺= 156; ¹H NMR (400 MHz, CD₃OD): δ 8.37 (d, 1H), 7.63 (dd, 1 H), 2.64 (s, 3H).

### Catalyst 1: 3,5-Bis-trifluoromethyl-benzoic acid (S)-(6-hydroxy-quinolin-4-yl)-((1S,2R,4S,5R)-5-vinyl-1-aza-bicyclo[2.2.2]oct-2-yl)-methyl ester

### a) 3,5-Bis-trifluoromethyl-benzoic acid (S)-(6-triisopropylsilanyloxy-quinolin-4-yl)-((1S,2R,4S,5R)-5-vinyl-1-aza-bicyclo[2.2.2]oct-2-yl)-methyl ester

To a solution of (S)-(6-triisopropylsilanyloxy-quinolin-4-yl)-((1S,2R,4S,5R)-5-vinyl-1-azabicyclo[2.2.2]oct-2-yl)-methanol (Deng et al., J. Amer. Chem. Soc. 2006, 128, 732; CAS Nr.: 876269-55-5; 3.22 g, 6.90 mmol) and Et₃N (1.442 ml, 10.35 mmol) in DCM was added dropwise 3,5-bis-trifluoromethyl-benzoyl chloride (2.480 g, 8.97 mmol). TLC (hexanes/ (EtOAc/MeOH 95:5) 1:1): Rf = 0.44; HPLC: Rt_{H5}= 3.256 min; ESIMS [M+H]⁺= 707; ¹H-NMR (DMSO-d6, 360 MHz): δ 8.76 (d, 1H), 8.53 (s, 2H), 8.00 (d, 1H), 7.73 (d, 1H), 7.59 (s, 1H), 7.41 (d, 1H), 6.49 (d, 1H), 6.09 (ddd, 1H), 5.11 (d, 1H), 5.07 (d, 1H), 3.59 (q, 1H), 2.85-2.73 (m, 2H), 2.65-2.56 (m, 2H), 2.33-2.23 (m, 1H), 1.96-1.50 (m, 5H), 1.38-1.25 (m, 3H), 1.10 (d, 18H).

### b) 3,5-Bis-trifluoromethyl-benzoic acid (S)-(6-hydroxy-quinolin-4-yl)-((1S,2R,4S,5R)-5-vinyl-1-aza-bicyclo[2.2.2]oct-2-yl)-methyl ester

To a solution of compound catalyst 1a) (4.88 g, 6.90 mmol) in 50 ml THF was added dropwise HF-Py (1.8 ml, 68 mmol). The reaction was slightly exothermic and the resulting yellow solution was stirred at rt for 30 min. The mixture was diluted with EtOAc and washed with sat aq. NaHCO3 (3x) and brine. The org layer was dried with Na2SO4 and evaporated. The crude product was purified by column chromatography on silica gel (hexanes/ (EtOAc/ MeOH 20:1) 30-75%) to give the title compound as a pale yellow solid. TLC (hexanes/ (EtOAc/ MeOH 5%] 1:3): Rf = 0.28; HPLC: Rt_{H3}= 2.464 min [M+H]⁺ 551; ¹H-NMR (DMSO-d6, 600 MHz): δ 10.20 (s, 1 H), 8.72 (d, 1 H), 8.58 (s, 2H), 8.51 (s, 1 H), 7.89 (d, 1H), 7.61 (d, 1 H), 7.49 (s, 1 H), 7.33 (d, 1H), 6.49 (d, 1H), 6.07 (ddd, 1H), 5.10 (d, 1H), 5.07 (d, 1H), 3.50-3.43 (m, 1H), 2.88-2.73 (m, 2H), 2.67-2.50 (m, 2H), 2.23 (q, 1H), 1.93-1.83 (m, 1H), 1.78 (s, 1 H), 1.70-1.61 (m 1H), 1.60-1.52 (m, 1H), 1.50-1.44 (m, 1H).

## Claims

1. A compound of formula (I) 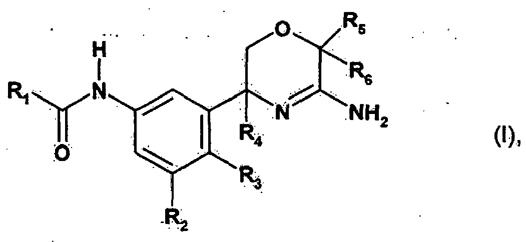 wherein R₁, R₂, R₃, R₄, R₅ and R₆ are defined so as to provide a compound selected from:
5-chloro-3-methoxymethyl-pyridine-2-carboxylic acid [3(5-amino-3-difluoromethyl-3,6-dihydro-2H-[1,4] oxazin-3-yl)-4-fluoro-phenyl]-amide;
3-Chloro-5-cyano-pyridine-2-carboxylic acid [3-(5-amino-3-difluoromethyl-3,6-dihydro-2H-[1,4]oxazin-3-yl)-4-fluoro-phenyl]-amide;
3-Amino-5-tris-deutero-methoxy-pyrazine-2-carboxylic acid [3-(5-amino-3-difluoromethyl-3,6-dihydro-2H-[1,4]oxazin-3-yl)-4-fluoro-phenyl]-amide;
3-Amino-5-prop-2-ynyloxy-pyrazine-2-carboxylic acid [3-(5-amino-3-difluoromethyl-3,6-dihydro-2H-[1,4]oxazin-3-yl)4-fluoro-phenyl]-amide;
3-Chloro-1H-pyrrolo[2,3-b]pyridine-6-carboxylic acid [3-(5-amino-3-difluoromethyl-3,6-dihydro-2H-[1,4]oxazin-3-yl)-4-fluoro-phenyl]-amide;
3-Amino-5-difluoromethyl-pyrazine-2-carboxylic acid [3-(5-amino-3-difluoromethyl-3,6-dihydro-2H-[1,4]oxazin-3-yl)-4-fluoro-phenyl]-amide;
5-Methoxy-3-methyl-pyridine-2-carboxylic acid [3-(6-amino-3-difluoromethyl-3,6-dihydro-2H-[1,4]oxazin-3-yl)-4-fluoro-phenyl]-amide;
5-Difluoromethyl-3-methyl-pyridine-2-carboxylic acid [3-(5-amino-3-difluoromethyl-3,6-dihydro-2H-[1,4]oxazin-3-yl)-4-fluoro-phenyl]-amide;
5-Fluoro-3-trideuteromethoxymethyl-pyridin-2-carboxylic-acid [3-(5-amino-3-difluoromethyl-3,6-dihydro-2H-[1,4]oxazin-3-yl)-4-fluoro-phenyl]-amide;
5-Trideuteromethoxy-3-trideuteromethoxymethyl-pyridine-2-carboxylic acid [3-(5-amino-3-difluoromethyl-3,6-dihydro-2H-[1,4]oxazin-3-yl)-4-fluoro-phenyl]-amide;
3-Amino-5-cyano-pyridine-2-carboxylic acid [3-(5-amino-3-difluoromethyl-3,6-dihydro-2H-[1,4]oxazin-3-yl)-4-fluoro-phenyl]-amide;
3-Amino-5-difluoromethyl-pyridine-2-carboxylic acid [3-(5-amino-3-difluoromethyl-3,6 dihydro-2H-[1,4]oxazin-3-yl)-4-fluoro-phenyl]-amide;
5-Trideuteromethoxy-3-methyl-pyridine-2-carboxylic acid [3-(5-amino-3-difluoromethyl-3,6-dihydro-2H-[1,4]oxazin-3-yl)-4-fluoro-phenyl]-amide;
5-cyano-3-methyl-pyridine-2-carboxylic acid [3-(5-amino-3-difluoromethyl-3,6-dihydro-2H-[1,4]oxazin-3-yl)-4,5-difluoro-phenyl]-amide;
3-Chloro-5-trideuteromethoxy-pyridine-2-carboxylic acid [3-(5-amino-3-difluoromethyl-3,6-dihydro-2H-[1,4]oxazin-3-yl)-4,5-difluoro-phenyl]-amide;
4,6-Dideutero-5-chloro-3-trideuteromethyl-pyridine-2-carboxylic acid [3-(5-amino-3-difluoromethyl-3,6-dihydro-2H-[1,4]oxazin-3-yl)-4,5-difluoro-phenyl]-amide;
3-Chloro-5-cyano-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-methoxy-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-difluoromethoxy-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
5-Chloro-3-methyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
5-Chloro-3-fluoro-pyridine-2-carboxylic acid [3-(5-amino-3,6-dimethyl-6-terfluoromethyl-3,6-dihydro-2H-[1,4]oxazin-3-yl)-4-fluoro-phenyl]-amide;
3-Chloro-5-trideutero-methoxy-pyridine-2-carboxylic acid [3-(5-amino-3,6-dimethyl-6-trifludromethyl-3,6-dihydro-2H-[1,4]oxazin-3-yl)-4-fluoro-phenyl]-amide;
2,5-Dimethyl-oxazole-4-carboxylic acid [3-(5-amino-3,6-dimethyl-6-trifluoromethyl-3,6= dihydro-2H-[1,4]oxazin-3-yl)-4-fluoro-phenyl]-amide;
5-Difluoromethoxy-3-methyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Amino-5-prop-2-ynyloxy-pyrazine-2-carboxylic acid [3-(5-amino-3,6-dimethyl-6-trifluoromethyl-3,6-dihydro-2H-[1,4]oxazin-3-yl)-4-fluoro-phenyl]-amide;
3-Amino-5-cyano-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
5-Difluoromethyl-3-methyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Amino-5-difluoromethyl-pyrazine-2-carboxylic acid [3-(5-amino-3,6-dimethyl-6-trifluoromethyl-3,6-dihydro-2H-[1,4]oxazin-3-yl)-4-fluoro-phenyl]-amide;
3-Amino-5-difluoromethyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
4-Difluoromethyl-6-methoxy-pyridazine-3-carboxylic acid [3-(5-amino-3,6-dimethyl-6-trifluoromethyl-3,6-dihydro-2H-[1,4]oxazin-3-yl)-4-fluoro-phenyl]-amide;
5-Cyano-3-trideuteromethoxymethyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-difluormethyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-trideuteromethoxy-dideuteromethyl-1H-pyrrolo[2,3-b]pyridine-6-carboxylic acid [3-(5-amino-3,6-dimethyl-6-trifluoromethyl-3,6-dihydro-2H-[1,4]oxazin-3-yl)-4-fluoro-phenyl]-amide;
3-Chloro-5-trifluoromethyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
5-Fluoro-3-methyl-pyridine-2-carboxylic acid [3-(5-amino-3,6-dimethyl-6-trifluoro-methyl-3,6-dihydro-2H-[1,4]oxazin-3-yl)-4-fluoro-phenyl]-amide;
5-Trideuteromethoxy-3-methyl-pyridine-2-carboxylic acid [3-(5-amino-3,6-dimethyl-6-trifluoromethyl-3,6-dihydro-2H-[1,4]oxazin-3-yl)-4-fluoro-phenyl]-amide;
5-Cyano-3-methyl-pyridine-2-carboxylic acid [3-(5-amino-6,6-bis-fluoromethyl-3-methyl-3,6-dihydro-2H-[1,4]oxozin-3-yl)-4-fluloro-phenyl]-amide;
5-Difluoromethoxy-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
5-Chloro-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
5-Cyano-3-methyl-pyridine-2-carboxylic acid [3-(5-amino-3,6,6-tris-fluoromethyl-3,6-dihydro-2H-[1,4]oxazin-3-yl)-4-fluoro-phenyl]-amide;
5-Difluoromethyl-3-methyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
3-Amino-5-difluoromethyl-pyrazine-2-carboxylic acid [3-(5-amino-6,6-bis-fluoromethyl-3-methyl-3,6-dihydro-2H-[1,4]oxazin-3-yl)-4-fluoro-phenyl]-amide;
3-Amino-5-difluoromethyl-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
3-Amino-5-cyano-pyridine-2-carboxylic acid [3-(5-amino-6,6-bis-fluoromethyl-3-methyl-3,6-dihydro-2H-[1,4]oxazin-3-yl)-4-fluoro-phenyl]-amide;
5-Difluoromethyl-3-methyl-pyridine-2-carboxylic acid [3-(5-amino-3,6,6-tris-fluoromethyl-3,6-dihydro-2H-[1,4]oxazin-3-yl)-4-fluoro-phenyl]-amide;
3-Chloro-5-difluoromethyl-pyridine-2-carboxylic acid [3-(5-amino-3,6,6-tris-fluoromethyl-3,6-dihydro-2H-[1,4]oxazin-3-yl)-4-fluoro-phenyl]-amide;
3-Chloro-5-trifluofomethyl-pyridine-2-carboxylic acid [3-(5-amino-3)6,6-tris-fluoromethyl-3,6-dihydro-2H-[1,4]oxazin-3-yl)-4-fluoro-phenyl]-amide;
3,5-Dichloro-pyridine-2-carboxylic acid [3-(5-amino-3,6,6-tris-fluoromethyl-3,6-dihydro-2H-[1,4]oxazin-3-yl)-4-fluoro-phenyl]-amide;
3-Amino-5-cyano-pyridine-2-carboxylic acid [3-(5-amino-3,6,6-tris-fluoromethyl-3,6-dihydro-2H-[1,4]oxazin-3-yl)-4-fluoro-phenyl]-amide;
3-Chloro-5-cyano-pyridine-2-carboxylic acid [3-(5-amino-3,6,6-tris-fluoromethyl-3,6-dihydro-2H-[1,4]oxazin-3-yl)-4-fluoro-phenyl]-amide;
N-[3-(5-Amino-3,6-dimethyl-6-trifluoromethyl-3,6-dihydro-2H-[1,4]oxazin-3-yl)-4-fluoro-phenyl]-6-methoxy-2-methyl-nicotinamide;
N-[3-(5-Amino-3,6-dimethyl-6-trifluoromethyl-3,6-dihydro-2H-[1,4]oxazin-3-yl)-4-fluoro-phenyl]-6-trideuteromethoxy-2-methyl-nicotinamide;
2-Amino-N-[3-(5-amino-3,6-dimethyl-6-trifluoromethyl-3,6-dihydro-2H-[1,4]oxazin-3-yl)-4-fluoro-phenyl]-6-ethoxy-nicotinamide;
2-Amino-N-[3-(5-amino-3,6-dimethyl-6-trifluoromethyl-3,6-dihydro-2H-[1,4]oxazin-3-yl)-4-fluoro-phenyl]-6-methoxy-nicotinamide;
2-Amino-N-[3-(5-amino-3,6-dimethyl-6-trifluoromethyl-3,6-dihydro-2H-[1,4]oxazin-3-yl)-4-fluoro-phenyl]-6-trideuteromethoxy-nicotinamide;
2-Amino-N-[3-(5-amino-3,6-dimethyl-6-trifluoromethyl-3,6-dihydro-2H-[1,4]oxazin-3-yl)-4-fluoro-phenyl]-6-pentadeuteroethoxy-nicotinamide;
N-[3-(5-Amino-3,6-dimethyl-6-trifluoromethyl-3,6-dihydro-2H-[1,4]oxazin-3-yl)-4-fluoro-phenyl]-2-chloro-6-methoxy-nicotinamide;
N-[3-(5-Amino-3,6-dimethyl-6-trifluoromethyl-3,6-dihydro-2H-[1,4]oxazin-3-yl)-4-fluoro-phenyl]-2-chloro-6-ethoxy-nicotinamide;
2-Amino-N-[3-(5-amino-3,6-dimethyl-6-trifluoromethyl-3,6-dihydro-2H-[1,4]oxazin-3-yl)-4-fluoro-phenyl]-6-cyclopropylmethoxy-nicotinamide; and
2-Amino-N-[3-(5-amino-3,6-dimethyl-6-trifluoromethyl-3,6-dihydro-2H-[1,4]oxazin-3-yl)-4-fluoro-phenyl]-6-(2,2,2-trifluoro-ethoxy)-nicotinamide;
and pharmaceutically acceptable salts thereof.

2. A compound according to Claim 1 of the formula (I) 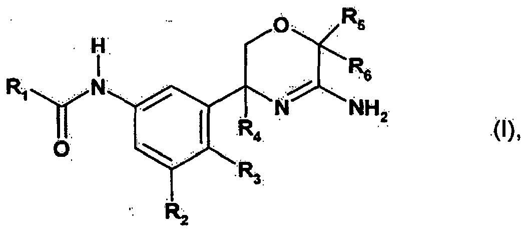 wherein R₁, R₂, R₃, R₄, R₅ and R₆ are defined so as to provide a compound selected from:
5-Chloro-3-methoxymethyl-pyridine-2-carboxylic acid [3-((R)-5-amino-3-difluoromethyl-3,6-dihydro-2H-[1,4]oxazin-3-yl)-4-fluoro-phenyl]-amide;
3-Chloro-5-cyano-pyridine-2-carboxylic acid [3-((R)-5-amino-3-difluoromethyl-3,6-dihydro-2H-[1,4]oxazin-3-yl)-4-fluoro-phenyl]-amide;
3-Amino-5-tris-deutero-methoxy-pyrazine-2-carboxylic acid [3-((R)-5-amino-3-difluoromethyl-3,6-dihydro-2H-[1,4]oxazin-3-yl)-4-fluoro-phenyl]-amide;
3-Amino-5-prop-2-ynyloxy-pyrazine-2-carboxylic acad [3-((R)-5-amino-3-difluoromethyl-3,6-dihydro-2H-[1,4]oxazin-3-yl)-4-fluoro-phenyl]-amide;
3-Chloro-1H-pyrrolo[2,3-b]pyridine-6-carboxylic acid [3-((R)-5-amino-3-difluoromethyl-3,6-dihydro-2H-[1,4]oxazin-3-yl)-4-fluoro-phenyl]-amide;
3-Amino-5-difluoromethyl-pyrazine-2-carboxylic acid [3-((R)-5-amino-3-difluoromethyl-3,6-dihydro-2H-[1,4]oxazin-3-yl)-4-fluoro-phenyl]-amide;
5-Methoxy-3-methyl-pyridine-2-carboxylic acid [3-((R)-5-amino-3-difluoromethyl-3,6-dihydro-2H-[1,4]oxazin-3-yl)-4-fluoro-phenyl]-amide;
5-Difluoromethyl-3-methyl-pyridine-2-carboxylic acid [3-((R)-5-amino-3-difluoromethyl-3,6-dihydro-2H-[1,4]oxazin-3-yl)-4-fluoro-phenyl]-amide;
5-Fluoro-3-trideuteromethoxymethyl-pyridine-2-carboxylic acid [3-((R)-5-amino-3-difluoromethyl-3,6-dihydro-2H-[1,4]oxazin-3-yl)-4-fluoro-phenyl]-amide;
5-Trideuteromethoxy-3-trideuteromethoxymethyl-pyridine-2-carboxylic acid [3-((R)-5-amino-3-difluoromethyl-3,6-dihydro-2H-[1,4]oxazin-3-yl)-4-fluoro-phenyl]-amide;
3-Amino-5-cyano-pyridine-2-carboxylic acid [3-((R)-5-amino-3-difluoromethyl-3,6-dihydro-2H-[1,4]oxazin-3-yl)-4-fluoro-phenyl]-amide;
3-Amino-5-difluofomethyl-pyridine-2-carboxylic acid [3-((R)-5-amino-3-difluoromethyl-3,6-dihydro-2H-[1,4]oxazin-3-yl)-4-fluoro-phenyl]-amide;
5-Trideuteromethoxy-3-methyl-pyridine-2-carboxylic acid [3-((R)-5-amino-3-difluoromethyl-3,6-dihydro-2H-[1,4]oxazin-3-yl)-4-fluoro-phenyl]-amide;
5-Cyano-3-methyl-pyridin-2-carboxylic acid [3-((R)-5-amino-3-difluoromethyl-3,6-dihydro-2H-[1,4]oxazin-3-yl)-4,5-difluoro-phenyl]-amide;
3-Chloro-5-trideuteromethoxy-pyridine-2-carboxylic acid [3-((R)-5-amino-3-difluoromethyl-3,6-dihydro-2H-[1,4]oxazin-3-yl)-4,5-difluoro-phenyl]-amide;
4,6-Dideutero-5-chloro-3-trideuteromethyl-pyridine-2-carboxylic acid [3-((R)-5-amino-3-difluoromethyl-3,6-dihydro-2H-[1,4]oxazin-3-yl)-4,5-difluoro-phenyl]-amide;
3-Chloro-5-cyano-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Chloro-5-methoxy-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Chloro-5-difluoromethoxy-pyridine-2-carboxylic acid [3-((3R,6R)-5-amino3,6-dimethyl-6-trifluoromethyl-3,6-dihydro-2H-[1,4]oxazin-3-yl)-4-fluoro-phenyl]-amide;
5-Chloro-3-methyl-pyndine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Chloro-3-fluoro-pyridine-2-carboxylic acid [3-((3R,6R)-5-amino-3,6-dimethyl-6-trifluoromethyl-3,6-dihydro-2H-[1,4]oxazin-3-yl)-4-fluorophenyl]-amide;
3-Chloro-5-trideutero-methoxy-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
2,5-Dimethyl-oxazole-4-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Difluoromethoxy-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Amino-5-prop-2-ynyloxy-pyrazine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Amino-5-cyano-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Difluoromethyl-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Amino-5-difluoromethyl-pyrazine-2-carboxylic acid [3-((3R,6R)-5-amino-3,6-dimethyl-6-trifluoromethyl-3,6-dihydro-2H-[1,4]oxazin-3-yl)4-fluoro-phenyl]-amide;
3-Amino-5-difluoromethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
4-Difluoromethyl-6-methoxy-pyridazine-3-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Cyano-3-trideuteromethoxymethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Chloro-5-difluormethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Chloro-5-trideuteromethoxy-dideuteromethyl-1H-pyrrolo[2,3-b]pyridine-6-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
3-Chloro-5-trifluoromethyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Fluoro-3-melhyl-pyridine-2-carboxylic acid [3-((3R,6R)-5-amino-3,6-dimethyl-6-trifluoro-methyl-3,6-dihydro-2H-[1,4]oxazin-3-yl)-4-fluoro-phenyl]-amide;
5-Trideuteromethoxy-3-methyl-pyridine-2-carboxylic acid [3-((3R,6R)-5-amino-3,6-dimethyl-6-trifluoromethyl-3,6-dihydro-2H-[1,4]oxazin-3-yl)-4-fluoro-phenyl]-amide;
5-Cyano-3-methyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide;
5-Difluoromethoxy-3-methyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide;
5-Cyano-3-methyl-pyridine-2-carboxylic acid [3-((R)-5-amino-3,6,6-tris-fluoromethyl-3,6-dihydro-2H-[1,4]oxazin-3-yl)-4-fluoro-phenyl]-amide;
5-Difluoromethyl-3-methyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide;
3-Amino-5-difluoromethyl-pyrazine-2-carboxylic acid [3-((R)-5-amino-6,6-bis-fluoromethyl-3-methyl-3,6-dihydro-2H-[1,4]oxazin-3-yl)-4-fluoro-phenyl]-amide;
3-Amino-5-difluoromethyl-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide;
3-Amino-5-cyano-pyridine-2-carboxylic acid [3-((R)-5-amino-6,6-bis-fluoromethyl-3-methyl-3,6-dihydro-2H-[1,4]oxazin-3-yl)-4-fluoro-phenyl]-amide;
5-Difluoromethyl-3-methyl-pyridine-2-carboxylic acid [3-((R)-5-amino-3,6,6-tris-fluoromethyl-3,6-dihydro-2H-[1,5]oxazin-5-yl)-fluoro-phenyl]-amide;
3-Chloro-5-difluoromethyl-pyridine-2-carboxylic acid [3-((R)-5-amino-3,6,6-tris-fluoromethyl-3,6-dihydro-2H-[1,4]oxazin-3-yl)-4-fluoro-phenyl]-amide;
3-Chloro-5-trifluoromethyl-pyridine-2-carboxylic acid [3-((R)-5-amino-3,6,6-tris-fluoromethyl-3,6-dihydro-2H-[1,4]oxazin-3-yl)-4-fluoro-phenyl]-amide;
3,5-Dichloro-pyridine-2-carboxylic acid [3-((R)-5-amino-3,6,6-tris-fluoromethyl-3,6-dihydro-2H-[1,4]oxazin-3-yl)-4-fluoro-phenyl]-amide;
3-Amino-5-cyano-pyridine-2-carboxylic acid [3-((R)-5-amino-3,6,6-tris-fluoromethyl-3,6-dihydro-2H-[1,4]oxazin-3-yl)-4-fluoro-phenyl]-amide;
3-Chloro-5-cyano-pyridine-2-carboxylic acid [3-((R)-5-amino-3,6,6-tris-fluoromethyl-3,6-dihydro-2H-[1,4]oxazin-3-yl)-4-fluoro-phenyl]-amide;
N-[3-((3R,6R)-5-Amino-3,6-dimethyl-6-trifluoromethyl-3,6-dihydro-2H-[1,4]oxazin-3-yl)-4-fluoro-phenyl]-6-methoxy-2-methyl-nicotinamide;
N-[3-((3R,6R)-5-Amino-3,6-dimethyl-6-trifluoromethyl-3,6-dihydro-2H-[1,4]oxazin-3-yl)-4-fluoro-phenyl]-6-trideuteromethoxy-2-methyl-nicotinamide;
2-Amino-N-[3-((3R,6R)-5-amino-3,6-dimethyl-6-trifluoromethyl-3,6-dihydro-2H-[1,4]oxazin-3-yl)-4-fluoro-phenyl]-6-ethoxy-nicotinamide;
2-Amino-N-[5-((3R,6R)-5-amino-3,6-dimethyl-6-trifluoromethyl-3,6-dihydro-2H-[1,4]oxazin-3-yl)-4-fluoro-phenyl]-6-methoxy-nicotinamide;
2-Amino-N-[3-((3R,6R)-5-amino-3,6-dimethyl-6-trifluoromethyl-3,6-dihydro-2H-[1,4]oxazin-3-yl)-4-fluoro-phenyl]-6-trideuteromethoxy-nicotinamide;
2-Amino-N-[3-((3R,6R)-5-amino-3,6-dimethyl-6-trifluoromethyl-3,6-dihydro-2H-[1,4]oxazin-3-yl)-4-fluoro-phenyl]-6-pentadeuteroethoxy-nicotinamide;
N-[3-((3R,6R)-5-Amino-3,6-dimethyl-6-trifluoromethyl-3,6-dihydro-2H-[1,4]oxazin-3-yl)-4-fluoro-phenyl]-2-chloro-6-methoxy-nicotinamide;
N-[3-((3R,6R)-5-Amino-3,6-dimethyl-6-trifluoromethyl-3,6-dihydro-2H-[1,4]oxazin-3-yl)-4-fluoro-phenyl]-2-chloro-6-ethoxy-nicotinamide;
2-Amino-N-[3-((3R,6R)-5-amino-3,6-dimethyl-6-trifluoromethyl-3,6-dihydro-2H-[1,4]oxazin-3-yl)-4-fluoro-phenyl]-6-cyclopropylmethoxy-nicotinamide; and
2-Amino-N-[3-((3R,6R)-5-amino-3,6-dimethyl-6-trifluoromethyl-3,6-dihydro-2H-[1,4]oxazin-3-yl)-4-fluoro-phenyl]-6-(2,2,2-trifluoro-ethoxy)-nicotinamide;
and pharmaceutically acceptable salts thereof.

3. A compound according to Claim 1 or Claim 2, or a pharmaceutically acceptable salt thereof, for use as a medicament.

4. A compound according to Claim 1 or Claim 2, or a pharmaceutically acceptable salt thereof, for use in the treatment or prevention of Alzheimer's disease or mild cognitive impairment.

5. A pharmaceutical composition comprising a compound according to Claim 1 or Claim 2, or a pharmaceutically acceptable salt thereof, as active ingredient and a pharmaceutically acceptable carrier or diluent.

6. A combination comprising a therapeutically effective amount of a compound according to Claim 1 or Claim 2, or a pharmaceutically acceptable salt thereof; and a second drug substance, for simultaneous or sequential administration.

## Patentansprüche

1. Verbindung der Formel (I) 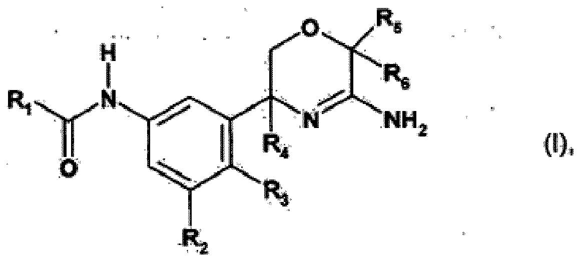 wobei R₁, R₂, R₃, R₄, R₅ und R₆ so definiert sind, dass sie eine Verbindung bereitstellen, die ausgewählt ist aus:
5-Chlor-3-methoxymethyl-pyridin-2-carbonsäure-[3-(5-amino-3-difluormethyl-3,6-dihydro-2H-[1,4]oxazin-3-yl)-4-fluor-phenyl]-amid;
3-Chlor-5-cyano-pyridin-2-carbonsäure-[3-(5-amino-3-difluormethyl-3,6-dihydro-2H-[1,4]oxazin-3-yl)-4-fluor-phenyl]-amid;
3-Amino-5-tris-deutero-methoxy-pyrazin-2-carbonsäure-[3-(5-amino-3-difluormethyl-3,6-dihydro-2H-[1,4]oxazin-3-yl)-4-fluor-phenyl]-amid;
3-Amino-5-prop-2-ynyloxy-pyrazin-2-carbonsäure-[3-(5-amino-3-difluormethyl-3,6-dihydro-2H-[1,4]oxazin-3-yl)-4-fluor-phenyl]-amid;
3-Chlor-1H-pyrrolo[2,3-b]pyridin-6-carbonsäure-[3-(5-amino-3-difluormethyl-3,6-dihydro-2H-[1,4]oxazin-3-yl)-4-fluor-phenyl]-amid;
3-Amino-5-difluormethyl-pyrazin-2-carbonsäure-[3-(5-amino-3-difluormethyl-3,6-dihydro-2H-[1,4]oxazin-3-yl)-4-fluor-phenyl]-amid;
5-Methoxy-3-methyl-pyridin-2-carbonsäure-[3-(5-amino-3-difluormethyl-3,6-dihydro-2H-[1,4]oxazin-3-yl)-4-fluor-phenyl]-amid;
5-Difluormethyl-3-methyl-pyridin-2-carbonsäure-[3-(5-amino-3-difluormethyl-3,6-dihydro-2H-[1,4]oxazin-3-yl)-4-fluor-phenyl]-amid;
5-Fluor-3-trideuteromethoxymethyl-pyridin-2-carbonsäure-[3-(5-amino-3-difluormethyl-3,6-dihydro-2H-[1,4]oxazin-3-yl)-4-fluor-phenyl]-amid;
5-Trideuteromethoxy-3-trideuteromethoxymethyl-pyridin-2-carbonsäure-[3-(5-amino-3-difluormethyl-3,6-dihydro-2H-[1,4]oxazin-3-yl)-4-fluor-phenyl]-amid;
3-Amino-5-cyano-pyridin-2-carbonsäure-[3-(5-amino-3-difluormethyl-3,6-dihydro-2H-[1,4]oxazin-3-yl)-4-fluor-phenyl]-amid;
3-Amino-5-difluormethyl-pyridin-2-carbonsäure-[3-(5-amino-3-difluormethyl-3,6-dihydro-2H-[1,4]oxazin-3-yl)-4-fluor-phenyl]-amid;
5-Trideuteromethoxy-3-methyl-pyridin-2-carbonsäure-[3-(5-amino-3-difluormethyl-3,6-dihydro-2H-[1,4]oxazin-3-yl)-4-fluor-phenyl]-amid;
5-Cyano-3-methyl-pyridin-2-carbonsäure-[3-(5-amino-3-difluormethyl-3,6-dihydro-2H-[1,4]oxazin-3-yl)-4,5-difluor-phenyl]-amid;
3-Chlor-5-trideuteromethoxy-pyridin-2-carbonsäure-[3-(5-amino-3-difluormethyl-3,6-dihydro-2H-[1,4]oxazin-3-yl)-4,5-difluor-phenyl]-amid;
4,6-Dideutero-5-chlor-3-trideuteromethyl-pyridin-2-carbonsäure-[3-(5-amino-3-difluormethyl-3,6-dihydro-2H-[1,4]oxazin-3-yl)-4,5-difluor-phenyl]-amid;
3-Chlor-5-cyano-pyridin-2-carbonsäure-[3-(5-amino-3,6-dimethyl-6-trifluormethyl-3,6-dihydro-2H-[1,4]oxazin-3-yl)-4-fluor-phenyl]-amid;
3-Chlor-5-methoxy-pyridin-2-carbonsäure-[3-(5-amino-3,6-dimethyl-6-trifluormethyl-3,6-dihydro-2H-[1,4]oxazin-3-yl)-4-fluor-phenyl]-amid;
3-Chlor-5-difluormethoxy-pyridin-2-carbonsäure-[3-(5-amino-3,6-dimethyl-6-trifluormethyl-3,6-dihydro-2H-[1,4]oxazin-3-yl)-4-fluor-phenyl]-amid;
5-Chlor-3-methyl-pyridin-2-carbonsäure-[3-(5-amino-3,6-dimethyl-6-trifluormethyl-3,6-dihydro-2H-[1,4]oxazin-3-yl)-4-fluor-phenyl]-amid;
5-Chlor-3-fluor-pyridin-2-carbonsäure-[3-(5-amino-3,6-dimethyl-6-trifluormethyl-3,6-dihydro-2H-[1,4]oxazin-3-yl)-4-fluor-phenyl]-amid;
3-Chlor-5-trideutero-methoxy-pyridin-2-carbonsäure-[3-(5-amino-3,6-dimethyl-6-trifluormethyl-3,6-dihydro-2H-[1,4]oxazin-3-yl)-4-fluor-phenyl]-amid;
2,5-Dimethyl-oxazole-4-carbonsäure-[3-(5-amino-3,6-dimethyl-6-trifluormethyl-3,6-dihydro-2H-[1,4]oxazin-3-yl)-4-fluor-phenyl]-amid;
5-Difluormethoxy-3-methyl-pyridin-2-carbonsäure-[3-(5-amino-3,6-dimethyl-6-trifluoimethyl-3,6-dihydro-2H-[l,4]oxazin-3-yl)-4-fluor-phenyl]-amid;
3-Amino-5-prop-2-ynyloxy-pyrazin-2-carbonsäure-[3-(5-amino-3,6-dimethyl-6-trifluormethyl-3,6-dihydro-2H-[1,4]oxazin-3-yl)-4-fluor-phenyl]-amid;
3-Amino-5-cyano-pyridin-2-carbonsäure-[3-(5-amino-3,6-dimethyl-6-trifluormethyl-3,6-dihydro-2H-[1,4]oxazin-3-yl)-4-fluor-phenyl]-amid;
5-Difluormethyl-3-methyl-pyridin-2-carbonsäure-[3-(5-amino-3,6-dimethyl-6-trifluormethyl-3,6-dihydro-2H-[1,4]oxazin-3-yl)-4-fluor-phenyl]-amid;
3-Amino-5-difluormethyl-pyrazin-2-carbonsäure-[3-(5-amino-3,6-dimethyl-6-trifluormethyl-3,6-dihydro-2H-[1,4]oxazin-3-yl)-4-fluor-phenyl]-amid;
3-Amino-5-difluormethyl-pyridin-2-carbonsäure-[3-(5-amino-3,6-dimethyl-6-trifluormethyl-3,6-dihydro-2H-[1,4]oxazin-3-yl)-4-fluor-phenyl]-amid;
4-Difluormethyl-6-methoxy-pyridazine-3-carbonsäure-[3-(5-amino-3,6-dimethyl-6-trifluormethyl-3,6-dihydro-2H-[1,4]oxazin-3-yl)-4-fluor-phenyl]-amid;
5-Cyano-3-trideuteromethoxymethyl-pyridin-2-carbonsäure-[3-(5-amino-3,6-dimethyl-6-trifluormethyl-3,6-dihydro-2H-[1,4]oxazin-3-yl)-4-fluor-phenyl]-amid;
3-Chlor-5-difluormethyl-pyridin-2-carbonsäure-[3-(5-amino-3,6-dimethyl-6-trifluormethyl-3,6-dihydro-2H-[1,4]oxazin-3-yl)-4-fluor-phenyl]-amid;
3-Chlor-5-trideuteromethoxy-dideuteromethyl-1H-pyrrolo[2,3-b]pyridin-6-carbonsäure-[3-(5-amino-3,6-dimethyl-6-trifluormethyl-3,6-dihydro-2H-[1,4]oxazin-3-yl)-4-fluor-phenyl]-amid;
3-Chlor-5-trifluormethyl-pyridin-2-carbonsäure-[3-(5-amino-3,6-dimethyl-6-trifluormethyl-3,6-dihydro-2H-[1,4]oxazin-3-yl)-4-fluor-phenyl]-amid;
5-Fluor-3-methyl-pyridin-2-carbonsäure-[3-(5-amino-3,6-dimethyl-6-trifluor-methyl-3,6-dihydro-2H-[1,4]oxazin-3-yl)-4-fluor-phenyl]-amid;
5-Trideuteromethoxy-3-methyl-pyridin-2-carbonsäure-[3-(5-amino-3,6-dimethyl-6-trifluormethyl-3,6-dihydro-2H-[1,4]oxazin-3-yl)-4-fluor-phenyl]-amid;
5-Cyano-3-methyl-pyridin-2-carbonsäure-[3-(5-amino-6,6-bis-fluormethyl-3-methyl-3,6-dihydro-2H-[1,4]oxazin-3-yl)-4-fluor-phenyl]-amid;
5-Difluormethoxy-3-methyl-pyridin-2-carbonsäure-[3-(5-amino-6,6-bis-fluormethyl-3-methyl-3,6-dihydro-2H-[1,4]oxazin-3-yl)-4-fluor-phenyl]-amid;
5-Chlor-3-methyl-pyridin-2-carbonsäure-[3-(5-amino-6,6-bis-fluormethyl-3-methyl-3,6-dihydro-2H-[1,4]oxazin-3-yl)-4-fluor-phenyl]-amid;
5-Cyano-3-methyl-pyridin-2-carbonsäure-[3-(5-amino-3,6,6-tris-fluormethyl-3,6-dihydro-2H-[1,4]oxazin-3-yl)-4-fluor-phenyl]-amid;
5-Difluormethyl-3-methyl-pyridin-2-carbonsäure-[3-(5-amino-6,6-bis-fluormethyl-3-methyl-3,6-dihydro-2H-[1,4]oxazin-3-yl)-4-fluor-phenyl]-amid;
3-Amino-5-difluormethyl-pyrazin-2-carbonsäure-[3-(5-amino-6,6-bis-fluormethyl-3-methyl-3,6-dihydro-2H-[1,4]oxazin-3-yl)-4-fluor-phenyl]-amid;
3-Amino-5-difluormethyl-pyridin-2-carbonsäure-[3-(5-amino-6,6-bis-fluormethyl-3-methyl-3,6-dihydro-2H-[1,4]oxazin-3-yl)-4-fluor-phenyl]-amid;
3-Amino-5-cyano-pyridin-2-carbonsäure-[3-(5-amino-6,6-bis-fluormethyl-3-methyl-3,6-dihydro-2H-[1,4]oxazin-3-yl)-4-fluor-phenyl]-amid;
5-Difluormethyl-3-methyl-pyridin-2-carbonsäure-[3-(5-amino-3,6,6-tris-fluormethyl-3,6-dihydro-2H-[1,4]oxazin-3-yl)-4-fluor-phenyl]-amid;
3-Chlor-5-difluormethyl-pyridin-2-carbonsäure-[3-(5-amino-3,6,6-tris-fluormethyl-3,6-dihydro-2H-[1,4]oxazin-3-yl)-4-fluor-phenyl]-amid;
3-Chlor-5-trifluormethyl-pyridin-2-carbonsäure-[3-(5-amino-3,6,6-tris-fluormethyl-3,6-dihydro-2H-[1,4]oxazin-3-yl)-4-fluor-phenyl]-amid;
3,5-Dichlor-pyridin-2-carbonsäure-[3-(5-amino-3,6,6-tris-fluormethyl-3,6-dihydro-2H-[1,4]oxazin-3-yl)-4-fluor-phenyl]-amid;
3-Amino-5-cyano-pyridin-2-carbonsäure-[3-(5-amino-3,6,6-tris-fluormethyl-3,6-dihydro-2H-[1,4]oxazin-3-yl)-4-fluor-phenyl]-amid;
3-Chlor-5-cyano-pyridin-2-carbonsäure-[3-(5-amino-3,6,6-tris-fluormethyl-3,6-dihydro-2H-[1,4]oxazin-3-yl)-4-fluor-phenyl]-amid;
N-[3-(5-Amino-3,6-dimethyl-6-trifluormethyl-3,6-dihydro-2H-[1,4]oxazin-3-yl)-4-fluorphenyl]-6-methoxy-2-methyl-nicotinamid;
N-[3-(5-Amino-3,6-dimethyl-6-trifluormethyl-3,6-dihydro-2H-[1,4]oxazin-3-yl)-4-fluorphenyl]-6-trideuteromethoxy-2-methyl-nicotinamid;
2-Amino-N-[3-(5-amino-3,6-dimethyl-6-trifluormethyl-3,6-dihydro-2H-[1,4]oxazin-3-yl)-4-fluor-phenyl]-6-ethoxy-nicotinamid;
2-Amino-N-[3-(5-amino-3,6-dimethyl-6-trifluormethyl-3,6-dihydro-2H-[1,4]oxazin-3-yl)-4-fluor-phenyl]-6-methoxy-nicotinamid;
2-Amino-N-[3-(5-amino-3,6-dimethyl-6-trifluormethyl-3,6-dihydro-2H-[1,4]oxazin-3-yl)-4-fluor-phenyl]-6-trideuteromethoxy-nicotinamid;
2-Amino-N-[3-(5-amino-3,6-dimethyl-6-trifluormethyl-3,6-dihydro-2H-[1,4]oxazin-3-yl)-4-fluor-phenyl]-6-pentadeuteroethoxy-nicotinamid;
N-[3-(5-Amino-3,6-dimethyl-6-trifluormethyl-3,6-dihydro-2H-[1,4]oxazin-3-yl)-4-fluor-phenyl]-2-chlor-6-methoxy-nicotinamid;
N-[3-(5-Amino-3,6-dimethyl-6-trifluormethyl-3,6-dihydro-2H-[1,4]oxazin-3-yl)-4-fluor-phenyl]-2-chlor-6-ethoxy-nicotinamid;
2-Amino-N-[3-(5-amino-3,6-dimethyl-6-trifluormethyl-3,6-dihydro-2H-[1,4]oxazin-3-yl)-4-fluor-phenyl]-6-cyclopropylmethoxy-nicotinamid; und
2-Amino-N-[3-(5-amino-3,6-dimethyl-6-trifluormethyl-3,6-dihydro-2H-[1,4]oxazin-3-yl)-4-fluor-phenyl]-6-(2,2,2-trifluor-ethoxy)-nicotinamid;
und pharmazeutisch unbedenkliche Salze davon.

2. Verbindung nach Anspruch 1 der Formel (I) 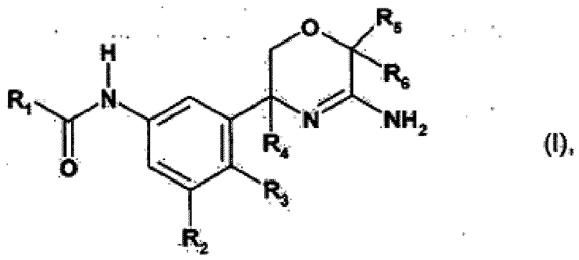 wobei R₁, R₂, R₃, R₄, R₅ und R₆ so definiert sind, dass sie eine Verbindung bereitstellen, die ausgewählt ist aus:
5-Chlor-3-methoxymethyl-pyridin-2-carbonsäure-[3-((R)-5-amino-3-difluormethyl-3,6-dihydro-2H-[1,4]oxazin-3-yl)-4-fluor-phenyl]-amid;
3-Chlor-5-cyano-pyridin-2-carbonsäure-[3-((R)-5-amino-3-difluormethyl-3,6-dihydro-2H-[1,4]oxazin-3-yl)-4-fluor-phenyl]-amid;
3-Amino-5-tris-deutero-methoxy-pyrazin-2-carbonsäure-[3-((R)-5-amino-3-difluormethyl-3,6-dihydro-2H-[1,4]oxazin-3-yl)-4-fluor-phenyl]-amid;
3-Amino-5-prop-2-ynyloxy-pyrazin-2-carbonsäure-[3-((R)-5-amino-3-difluormethyl-3,6-dihydro-2H-[1,4]oxazin-3-yl)-4-fluor-phenyl]-amid;
3-Chlor-1H-pyrrolo[2,3-b]pyridin-6-carbonsäure-[3-((R)-5-amino-3-difluormethyl-3,6-dihydro-2H-[1,4]oxazin-3-yl)-4-fluor-phenyl]-amid;
3-Amino-5-difluormethyl-pyrazin-2-carbonsäure-[3-((R)-5-amino-3-difluormethyl-3,6-dihydro-2H-[1,4]oxazin-3-yl)-4-fluor-phenyl]-amid;
5-Methoxy-3-methyl-pyridin-2-carbonsäure-[3-((R)-5-amino-3-difluormethyl-3,6-dihydro-2H-[1,4]oxazin-3-yl)-4-fluor-phenyl]-amid;
5-Difluormethyl-3-methyl-pyridin-2-carbonsäure-[3-((R)-5-amino-3-difluormethyl-3,6-dihydro-2H-[1,4]oxazin-3-yl)-4-fluor-phenyl]-amid;
5-Fluor-3-trideuteromethoxymethyl-pyridin-2-carbonsäure-[3-((R)-5-amino-3-difluormethyl-3,6-dihydro-2H-[1,4]oxazin-3-yl)-4-fluor-phenyl]-amid;
5-Trideuteromethoxy-3-trideuteromethoxymethyl-pyridin-2-carbonsäure-[3-((R)-5-amino-3-difluormethyl-3,6-dihydro-2H-[1,4]oxazin-3-yl)-4-fluor-phenyl]-amid;
3-Amino-5-cyano-pyridin-2-carbonsäure-[3-((R)-5-amino-3-difluormethyl-3,6-dihydro-2H-[1,4]oxazin-3-yl)-4-fluor-phenyl]-amid;
3-Amino-5-difluormethyl-pyridin-2-carbonsäure-[3-((R)-5-amino-3-difluormethyl-3,6-dihydro-2H-[1,4]oxazin-3-yl)-4-fluor-phenyl]-amid;
5-Trideuteromethoxy-3-methyl-pyridin-2-carbonsäure-[3-((R)-5-amino-3-difluormethyl-3,6-dihydro-2H-[1,4]oxazin-3-yl)-4-fluor-phenyl]-amid;
5-Cyano-3-methyl-pyridin-2-carbonsäure-[3-((R)-5-amino-3-difluormethyl-3,6-dihydro-2H-[1,4]oxazin-3-yl)-4,5-difluor-phenyl]-amid;
3-Chlor-5-trideuteromethoxy-pyridin-2-carbonsäure-[3((R)-5-amino-3-difluormethyl-3,6-dihydro-2H-[1,4]oxazin-3-yl)-4,5-difluor-phenyl]-amid;
4,6-Dideutero-5-chlor-3-trideuteromethyl-pyridin-2-carbonsäure-[3-((R)-5-amino-3-difluormethyl-3,6-dihydro-2H-[1,4]oxazin-3-yl)-4,5-difluor-phenyl]-amid;
3-Chlor-5-cyano-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
3-Chlor-5-methoxy-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
3-Chlor-5-difluormethoxy-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
5-Chlor-3-methyl-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
5-Chlor-3-fluor-pyridin-2-carbonsäure-[3-((3R,6R)-5-amino-3,6-dimethyl-6-trifluonnethyl-3,6-dihydro-2H-[1,4]oxazin-3-yl)-4-fluor-phenyl]-amid;
3-Chlor-5-trideutero-methoxy-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
2,5-Dimethyl-oxazole-4-carbonsäure-[3-((3R,6R)-5-amino-3,6-dimethyl-6-trifluormethyl-3,6-dihydro-2H-[1,4]oxazin-3-yl)-4-fluor-phenyl]-amid;
5-Difluormethoxy-3-methyl-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
3-Amino-5-prop-2-ynyloxy-pyrazin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
3-Amino-5-cyano-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
5-Difluormethyl-3-methyl-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
3-Amino-5-difluormethyl-pyrazin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
3-Amino-5-difluormethyl-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
4-Difluormethyl-6-methoxy-pyridazine-3-carbonsäure-[3-((3R,6R)-5-amino-3,6-dimethyl-6-trifluormethyl-3,6-dihydro-2H-[1,4]oxazin-3-yl)-4-fluor-phenyl]-amid;
5-Cyano-3-trideuteromethoxymethyl-pyrdin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
3-Chlor-5-difluormethyl-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
3-Chlor-5-trideuteromethoxy-dideuteromethyl-1H-pyrrolo[2,3-b]pyridin-6-carbonsäure-[3-((3R,6R)-5-amino-3,6-dimethyl-6-trifluormethyl-3,6-dihydro-2H-[1,4]oxazin-3-yl)-4-fluor-phenyl]-amid;
3-Chlor-5-trifluormethyl-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
5-Fluor-3-methyl-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
5-Trideuteromethoxy-3-methyl-pyridin-2-carbonsäure-[3-((3R,6R)-5-amino-3,6-dimethyl-6-trifluormethyl-3,6-dihydro-2H-[1,4]oxazin-3-yl)-4-fluor-phenyl]-amid;
5-Cyano-3-methyl-pyridin-2-carbonsäure-[3-((R)-5-amino-6,6-bis-fluormethyl-3-methyl-3,6-dihydro-2H-[1,4]oxazin-3-yl)-4-fluor-phenyl]-amid;
5-Difluormethoxy-3-methyl-pyridin-2-carbonsäure-[3-((R)-5-amino-6,6-bis-fluormethyl-3-methyl-3,6-dihydro-2H-[1,4]oxazin-3-yl)-4-fluor-phenyl]-amid;
5-Cyano-3-methyl-pyridin-2-carbonsäure-[3-((R)-5-amino-3,6,6-tris-fluormethyl-3,6-dihydro-2H-[1,4]oxazin-3-yl)-4-fluor-phenyl]-amid;
5-Difluormethyl-3-methyl-pyridin-2-carbonsäure-[3-((R)-5-amino-6,6-bis-fluormethyl-3-methyl-3,6-dihydro-2H-[1,4]oxazin-3-yl)-4-fluor-phenyl]-amid;
3-Amino-5-difluormethyl-pyrazin-2-carbonsäure-[3-((R)-5-amino-6,6-bis-fluormethyl-3-methyl-3,6-dihydro-2H-[1,4]oxazin-3-yl)-4-fluor-phenyl]-amid;
3-Amino-5-difluormethyl-pyridin-2-carbonsäure-[3-((R)-5-amino-6,6-bis-fluormethyl-3-methyl-3,6-dihydro-2H-[1,4]oxazin-3-yl)-4-fluor-phenyl]-amid;
3-Amino-5-cyano-pyridin-2-carbonsäure-[3-((R)-5-amino-6,6-bis-fluormethyl-3-methyl-3,6-dihydro-2H-[1,4]oxazin-3-yl)-4-fluor-phenyl]-amid;
5-Difluormethyl-3-methyl-pyridin-2-carbonsäure-[3-((R)-5-amino-3,6,6-tris-fluormethyl-3,6-dihydro-2H-[1,4]oxazin-3-yl)-4-fluor-phenyl]-amid;
3-Chlor-5-difluormethyl-pyridin-2-carbonsäure-[3-((R)-5-amino-3,6,6-tris-fluormethyl-3,6-dihydro-2H-[1,4]oxazin-3-yl)-4-fluor-phenyl]-amid;
3-Chlor-5-trifluormethyl-pyridin-2-carbonsäure-[3-((R)-5-amino-3,6,6-tris-fluormethyl-3,6-dihydro-2H-[1,4]oxazin-3-yl)-4-fluor-phenyl]-amid;
3,5-Dichlor-pyridin-2-carbonsäure-[3-((R)-5-amino-3,6,6-tris-fluormethyl-3,6-dihydro-2H-[1,4]oxazin-3-yl)-4-fluor-phenyl]-amid;
3-Amino-5-cyano-pyridin-2-carbonsäure-[3-((R)-5-amino-3,6,6-tris-fluormethyl-3,6-dihydro-2H-[1,4]oxazin-3-yl)-4-fluor-phenyl]-amid;
3-Chlor-5-cyano-pyridin-2-carbonsäure-[3-((R)-5-amino-3,6,6-tris-fluormethyl-3,6-dihydro-2H-[1,4]oxazin-3-yl)-4-fluor-phenyl]-amid;
N-[3-((3R,6R)-5-Amino-3,6-dimethyl-6-trifluoimethyl-3,6-dihydro-2H-[1,4]oxazin-3-yl)-4-fluor-phenyl]-6-methoxy-2-methyl-nicotinamid;
N-[3-((3R,6R)-5-Amino-3,6-dimethyl-6-trifluormethyl-3,6-dihydro-2H-[1,4]oxazin-3-yl)-4-fluor-phenyl]-6-trideuteromethoxy-2-methyl-nicotinamid;
2-Amino-N-[3-((3R,6R)-5-amino-3,6-dimethyl-6-trifluormethyl-3,6-dihydro-2H-[1,4]oxazin-3-yl)-4-fluor-phenyl]-6-ethoxy-nicotinamid;
2-Amino-N-[3-((3R,6R)-5-amino-3,6-dimethyl-6-trifluormethyl-3,6-dihydro-2H-[1,4]oxazin-3-yl)-4-fluor-phenyl]-6-methoxy-nicotinamid;
2-Amino-N-[3-((3R,6R)-5-amino-3,6-dimethyl-6-trifluormethyl-3,6-dihydro-2H-[1,4]oxazin-3-yl)-4-fluor-phenyl]-6-trideuteromethoxy-nicotinamid;
2-Amino-N-[3-((3R,6R)-5-amino-3,6-dimethyl-6-trifluormethyl-3,6-dihydro-2H-[1,4]oxazin-3-yl)-4-fluor-phenyl]-6-pentadeuteroethoxy-nicotinamid;
N-[3-((3R,6R)-5-Amino-3,6-dimethyl-6-trifluormethyl-3,6-dihydro-2H-[1,4]oxazin-3-yl)-4-fluor-phenyl]-2-chlor-6-methoxy-nicotinamid;
N-[3-((3R,6R)-5-Amino-3,6-dimethyl-6-trifluormethyl-3,6-dihydro-2H-[1,4]oxazin-3-yl)-4-fluor-phenyl]-2-chlor-6-ethoxy-nicotinamid;
2-Amino-N-[3-((3R,6R)-5-amino-3,6-dimethyl-6-trifluormethyl-3,6-dihydro-2H-[1,4]oxazin-3-yl)-4-fluor-phenyl]-6-cyclopropylmethoxy-nicotinamid; und
2-Amino-N-[3-((3R,6R)-5-amino-3,6-dimethyl-6-trifluormethyl-3,6-dihydro-2H-[1,4]oxazin-3-yl)-4-fluor-phenyl]-6-(2,2,2-trifluor-ethoxy)-nicotinamid;
und pharmazeutisch unbedenkliche Salze davon.

3. Verbindung nach Anspruch 1 oder Anspruch 2 oder ein pharmazeutisch unbedenkliches Salz davon zur Verwendung als Medikament.

4. Verbindung nach Anspruch 1 oder Anspruch 2 oder ein pharmazeutisch unbedenkliches Salz davon zur Verwendung bei der Behandlung oder Prävention von Morbus Alzheimer oder schwacher kognitiver Beeinträchtigung.

5. Pharmazeutische Zusammensetzung, die eine Verbindung nach Anspruch 1 oder Anspruch 2 oder ein pharmazeutisch unbedenkliches Salz davon als Wirkstoff und einen pharmazeutisch unbedenklichen Träger oder ein pharmazeutisch unbedenkliches Verdünnungsmittel umfasst.

6. Kombination, die eine therapeutisch wirksame Menge einer Verbindung nach Anspruch 1 oder Anspruch 2 oder eines pharmazeutisch unbedenklichen Salzes davon und eine zweite Arzneimittelsubstanz zur gleichen oder sequentiellen Verabreichung umfasst.

## Revendications

1. Composé de la formule (I) 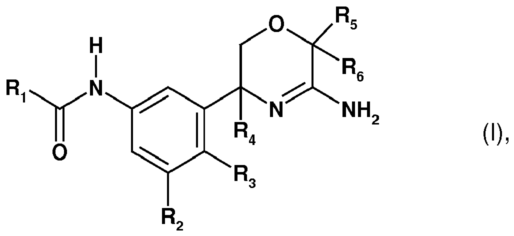 dans laquelle R₁, R₂, R₃, R₄, R₅ et R₆ sont définis de façon à produire un composé choisi parmi :
le [3-(5-amino-3-difluorométhyl-3,6-dihydro-2H-[1,4]oxazine-3-yl)-4-fluoro-phényl]-amide de l'acide 5-chloro-3-méthoxyméthyl-pyridine-2-carboxylique ;
le [3-(5-amino-3-difluorométhyl-3,6-dihydro-2H-[1,4]oxazine-3-yl)-4-fluoro-phényl]-amide de l'acide 3-chloro-5-cyano-pyridine-2-carboxylique ;
le [3-(5-amino-3-difluorométhyl-3,6-dihydro-2H-[1,4]oxazine-3-yl)-4-fluoro-phényl]-amide de l'acide 3-amino-5-tris-deutéro-méthoxy-pyrazine-2-carboxylique ;
le [3-(5-amino-3-difluorométhyl-3,6-dihydro-2H-[1,4]oxazine-3-yl)-4-fluoro-phényl]-amide de l'acide 3-amino-5-prop-2-ynyloxy-pyrazine-2-carboxylique ;
le [3-(5-amino-3-difluorométhyl-3,6-dihydro-2H-[1,4]oxazine-3-yl)-4-fluoro-phényl]-amide de l'acide 3-chloro-1H-pyrrolo [2,3-b]pyridine-6-carboxylique ;
le [3-(5-amino-3-difluorométhyl-3,6-dihydro-2H-[1,4]oxazine-3-yl)-4-fluoro-phényl]-amide de l'acide 3-amino-5-difluorométhyl-pyrazine-2-carboxylique ;
le [3-(5-amino-3-difluorométhyl-3,6-dihydro-2H-[1,4]oxazine-3-yl)-4-fluoro-phényl]-amide de l'acide 5-méthoxy-3-méthyl-pyridine-2-carboxylique ;
le [3-(5-amino-3-difluorométhyl-3,6-dihydro-2H-[1,4]oxazine-3-yl)-4-fluoro-phényl]-amide de l'acide 5-difluorométhyl-3-méthyl-pyridine-2-carboxylique ;
le [3-(5-amino-3-difluorométhyl-3,6-dihydro-2H-[1,4]oxazine-3-yl)-4-fluoro-phényl]-amide de l'acide 5-fluoro-3-trideutérométhoxyméthyl-pyridine-2-carboxylique ;
le [3-(5-amino-3-difluorométhyl-3,6-dihydro-2H-[1,4]oxazine-3-yl)-4-fluoro-phényl]-amide de l'acide 5-trideutérométhoxy-3-trideutérométhoxyméthyl-pyridine-2-carboxylique ;
le [3-(5-amino-3-difluorométhyl-3,6-dihydro-2H-[1,4]oxazine-3-yl)-4-fluoro-phényl]-amide de l'acide 3-amino-5-cyano-pyridine-2-carboxylique ;
le [3-(5-amino-3-difluorométhyl-3,6-dihydro-2H-[1,4]oxazine-3-yl)-4-fluoro-phényl]-amide de l'acide 3-amino-5-difluorométhyl-pyridine-2-carboxylique ;
le [3-(5-amino-3-difluorométhyl-3,6-dihydro-2H-[1,4]oxazine-3-yl)-4-fluoro-phényl]-amide de l'acide 5-trideutérométhoxy-3-méthyl-pyridine-2-carboxylique ;
le [3-(5-amino-3-difluorométhyl-3,6-dihydro-2H-[1,4]oxazine-3-yl)-4,5-difluoro-phényl]-amide de l'acide 5-cyano-3-méthyl-pyridine-2-carboxylique ;
le [3-(5-amino-3-difluorométhyl-3,6-dihydro-2H-[1,4]oxazine-3-yl)-4,5-difluoro-phényl]-amide de l'acide 3-chloro-5-trideutérométhoxy-pyridine-2-carboxylique ;
le [3-(5-amino-3-difluorométhyl-3,6-dihydro-2H-[1,4]oxazine-3-yl)-4,5-difluoro-phényl]-amide de l'acide 4,6-dideutéro-5-chloro-3-trideutérométhyl-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-chloro-5-cyano-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-chloro-5-méthoxy-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-chloro-5-difluorométhoxy-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 5-chloro-3-méthyl-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 5-chloro-3-fluoro-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-chloro-5-trideutéro-méthoxy-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 2,5-diméthyl-oxazole-4-carboxylique ;
le [3-(5-amino-3,6-diméthyl-6-trifluorométhyl-3,6-dihydro-2H-[1,4]oxazine-3-yl)-4-fluoro-phényl]-amide de l'acide 5-difluorométhoxy-3-méthyl-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-amino-5-prop-2-ynyloxy-pyrazine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-amino-5-cyano-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 5-difluorométhyl-3-méthyl-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-amino-5-difluorométhyl-pyrazine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-amino-5-difluorométhyl-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 4-difluorométhyl-6-méthoxy-pyridazine-3-carboxylique ;
le [3-(5-amino-3,6-diméthyl-6-trifluorométhyl-3,6-dihydro-2H-[1,4]oxazine-3-yl)-4-fluoro-phényl]-amide de l'acide 5-cyano-3-trideutérométhoxyméthyl-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-chloro-5-difluorométhyl-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-chloro-5-trideutérométhoxy-dideutérométhyl-1H-pyrrolo[2,3-b]pyridine-6-carboxylique ;
le [3-(5-amino-3,6-diméthyl-6-trifluorométhyl-3,6-dihydro-2H-[1,4]oxazine-3-yl)-4-fluoro-phényl]-amide de l'acide 3-chloro-5-trifluorométhyl-pyridine-2-carboxylique ;
le [3-(5-amino-3,6-diméthyl-6-trifluoro-méthyl-3,6-dihydro-2H-[1,4]oxazine-3-yl)-4-fluoro-phényl]-amide de l'acide 5-fluoro-3-méthyl-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 5-trideutérométhoxy-3-méthyl-pyridine-2-carboxylique ;
le [3-(5-amino-6,6-bis-fluorométhyl-3-méthyl-3,6-dihydro-2H-[1,4]oxazine-3-yl)-4-fluoro-phényl]-amide de l'acide 5-cyano-3-méthyl-pyridine-2-carboxylique ;
le [3-(5-amino-6,6-bis-fluorométhyl-3-méthyl-3,6-dihydro-2H-[1,4]oxazine-3-yl)-4-fluoro-phényl]-amide de l'acide 5-difluorométhoxy-3-méthyl-pyridine-2-carboxylique ;
le [3-(5-amino-6,6-bis-fluorométhyl-3-méthyl-3,6-dihydro-2H-[1,4]oxazine-3-yl)-4-fluoro-phényl]-amide de l'acide 5-chloro-3-méthyl-pyridine-2-carboxylique ;
le [3-(5-amino-3,6,6-tris-fluorométhyl-3,6-dihydro-2H-[1,4]oxazine-3-yl)-4-fluoro-phényl]-amide de l'acide 5-cyano-3-méthyl-pyridine-2-carboxylique ;
le [3-(5-amino-6,6-bis-fluorométhyl-3-méthyl-3,6-dihydro-2H-[1,4]oxazine-3-yl)-4-fluoro-phényl]-amide de l'acide 5-difluorométhyl-3-méthyl-pyridine-2-carboxylique ;
le [3-(5-amino-6,6-bis-fluorométhyl-3-méthyl-3,6-dihydro-2H-[1,4]oxazine-3-yl)-4-fluoro-phényl]-amide de l'acide 3-amino-5-difluorométhyl-pyrazine-2-carboxylique ;
le [3-(5-amino-6,6-bis-fluorométhyl-3-méthyl-3,6-dihydro-2H-[1,4]oxazine-3-yl)-4-fluoro-phényl]-amide de l'acide 3-amino-5-difluorométhyl-pyridine-2-carboxylique ;
le [3-(5-amino-6,6-bis-fluorométhyl-3-méthyl-3,6-dihydro-2H-[1,4]oxazine-3-yl)-4-fluoro-phényl]-amide de l'acide 3-amino-5-cyano-pyridine-2-carboxylique ;
le [3-(5-amino-3,6,6-tris-fluorométhyl-3,6-dihydro-2H-[1,4]oxazine-3-yl)-4-fluoro-phényl]-amide de l'acide 5-difluorométhyl-3-méthyl-pyridine-2-carboxylique ;
le [3-(5-amino-3,6,6-tris-fluorométhyl-3,6-dihydro-2H-[1,4]oxazine-3-yl)-4-fluoro-phényl]-amide de l'acide 3-chloro-5-difluorométhyl-pyridine-2-carboxylique ;
le [3-(5-amino-3,6,6-tris-fluorométhyl-3,6-dihydro-2H-[1,4]oxazine-3-yl)-4-fluoro-phényl]-amide de l'acide 3-chloro-5-trifluorométhyl-pyridine-2-carboxylique ;
le [3-(5-amino-3,6,6-tris-fluorométhyl-3,6-dihydro-2H-[1,4]oxazine-3-yl)-4-fluoro-phényl]-amide de l'acide 3,5-dichloro-pyridine-2-carboxylique ;
le [3-(5-amino-3,6,6-tris-fluorométhyl-3,6-dihydro-2H-[1,4]oxazine-3-yl)-4-fluoro-phényl]-amide de l'acide 3-amino-5-cyano-pyridine-2-carboxylique ;
le [3-(5-amino-3,6,6-tris-fluorométhyl-3,6-dihydro-2H-[1,4]oxazine-3-yl)-4-fluoro-phényl]-amide de l'acide 3-chloro-5-cyano-pyridine-2-carboxylique ;
le N-[3-(5-amino-3,6-diméthyl-6-trifluorométhyl-3,6-dihydro-2H-[1,4]oxazine-3-yl)-4-fluoro-phényl]-6-méthoxy-2-méthyl-nicotinamide ;
le N-[3-(5-amino-3,6-diméthyl-6-trifluorométhyl-3,6-dihydro-2H-[1,4]oxazine-3-yl)-4-fluoro-phényl]-6-trideutérométhoxy-2-méthyl-nicotinamide ;
le 2-amino-N-[3-(5-amino-3,6-diméthyl-6-trifluorométhyl-3,6-dihydro-2H-[1,4]oxazine-3-yl)-4-fluoro-phényl]-6-éthoxy-nicotinamide ;
le 2-amino-N-[3-(5-amino-3,6-diméthyl-6-trifluorométhyl-3,6-dihydro-2H-[1,4]oxazine-3-yl)-4-fluoro-phényl]-6-méthoxy-nicotinamide ;
2-amino-N-[3-(5-amino-3,6-diméthyl-6-trifluorométhyl-3,6-le dihydro-2H-[1,4]oxazine-3-yl)-4-fluoro-phényl]-6-trideutérométhoxy-nicotinamide ;
le 2-amino-N-[3-(5-amino-3,6-diméthyl-6-trifluorométhyl-3,6-dihydro-2H-[1,4]oxazine-3-yl)-4-fluoro-phényl]-6-pentadeutéroéthoxy-nicotinamide ;
le N-[3-(5-amino-3,6-diméthyl-6-trifluorométhyl-3,6-dihydro-2H-[1,4]oxazine-3-yl)-4-fluoro-phényl]-2-chloro-6-méthoxy-nicotinamide ;
le N-[3-(5-amino-3,6-diméthyl-6-trifluorométhyl-3,6-dihydro-2H-[1,4]oxazine-3-yl)-4-fluoro-phényl]-2-chloro-6-éthoxy-nicotinamide ;
le 2-amino-N-[3-(5-amino-3,6-diméthyl-6-trifluorométhyl-3,6-dihydro-2H-[1,4]oxazine-3-yl)-4-fluoro-phényl]-6-cyclopropylméthoxy-nicotinamide ; et
le 2-amino-N-[3-(5-amino-3,6-diméthyl-6-trifluorométhyl-3,6-dihydro-2H-[1,4]oxazine-3-yl)-4-fluoro-phényl]-6-(2,2,2-trifluoro-éthoxy)-nicotinamide ;
et leurs sels pharmaceutiquement acceptables.

2. Composé selon la revendication 1 de la formule (I) 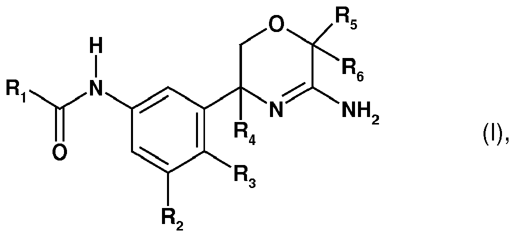 dans laquelle R₁, R₂, R₃, R₄, R₅ et R₆ sont définis de façon à produire un composé choisi parmi :
le [3-((R)-5-amino-3-difluorométhyl-3,6-dihydro-2H-[1,4]oxazine-3-yl)-4-fluoro-phényl]-amide de l'acide 5-chloro-3-méthoxyméthyl-pyridine-2-carboxylique ;
le [3-((R)-5-amino-3-difluorométhyl-3,6-dihydro-2H-[1,4]oxazine-3-yl)-4-fluoro-phényl]-amide de l'acide 3-chloro-5-cyano-pyridine-2-carboxylique ;
le [3-((R)-5-amino-3-difluorométhyl-3,6-dihydro-2H-[1,4]oxazine-3-yl)-4-fluoro-phényl]-amide de l'acide 3-amino-5-tris-deutéro-méthoxy-pyrazine-2-carboxylique ;
le [3-((R)-5-amino-3-difluorométhyl-3,6-dihydro-2H-[1,4]oxazine-3-yl)-4-fluoro-phényl]-amide de l'acide 3-amino-5-prop-2-ynyloxy-pyrazine-2-carboxylique ;
le [3-((R)-5-amino-3-difluorométhyl-3,6-dihydro-2H-[1,4]oxazine-3-yl)-4-fluoro-phényl]-amide de l'acide 3-chloro-1H-pyrrolo [2,3-b]pyridine-6-carboxylique ;
le [3-((R)-5-amino-3-difluorométhyl-3,6-dihydro-2H-[1,4]oxazine-3-yl)-4-fluoro-phényl]-amide de l'acide 3-amino-5-difluorométhyl-pyrazine-2-carboxylique ;
le [3-((R)-amino-3-difluorométhyl-3,6-dihydro-2H-[1,4]oxazine-3-yl)-4-fluoro-phényl]-amide de l'acide 5-méthoxy-3-méthyl-pyridine-2-carboxylique ;
le [3-((R)-amino-3-difluorométhyl-3,6-dihydro-2H-[1,4]oxazine-3-yl)-4-fluoro-phényl]-amide de l'acide 5-difluorométhyl-3-méthyl-pyridine-2-carboxylique ;
le [3-((R)-amino-3-difluorométhyl-3,6-dihydro-2H-[1,4]oxazine-3-yl)-4-fluoro-phényl]-amide de l'acide 5-fluoro-3-trideutérométhoxyméthyl-pyridine-2-carboxylique ;
le [3-((R)-amino-3-difluorométhyl-3,6-dihydro-2H-[1,4]oxazine-3-yl)-4-fluoro-phényl]-amide de l'acide 5-trideutérométhoxy-3-trideutérométhoxyméthyl-pyridine-2-carboxylique ;
le [3-((R)-amino-3-difluorométhyl-3,6-dihydro-2H-[1,4]oxazine-3-yl)-4-fluoro-phényl]-amide de l'acide 3-amino-5-cyano-pyridine-2-carboxylique ;
le [3-((R)-amino-3-difluorométhyl-3,6-dihydro-2H-[1,4]oxazine-3-yl)-4-fluoro-phényl]-amide de l'acide 3-amino-5-difluorométhyl-pyridine-2-carboxylique ;
le [3-((R)-amino-3-difluorométhyl-3,6-dihydro-2H-[1,4]oxazine-3-yl)-4-fluoro-phényl]-amide de l'acide 5-trideutérométhoxy-3-méthyl-pyridine-2-carboxylique ;
le [3-((R)-amino-3-difluorométhyl-3,6-dihydro-2H-[1,4]oxazine-3-yl)-4,5-difluoro-phényl]-amide de l'acide 5-cyano-3-méthyl-pyridine-2-carboxylique ;
le [3-((R)-amino-3-difluorométhyl-3,6-dihydro-2H-[1,4]oxazine-3-yl)-4,5-difluoro-phényl]-amide de l'acide 3-chloro-5-trideutérométhoxy-pyridine-2-carboxylique ;
le [3-((R)-amino-3-difluorométhyl-3,6-dihydro-2H-[1,4]oxazine-3-yl)-4,5-difluoro-phényl]-amide de l'acide 4,6-dideutéro-5-chloro-3-trideutérométhyl-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-chloro-5-cyano-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-chloro-5-méthoxy-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-chloro-5-difluorométhoxy-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 5-chloro-3-méthyl-pyridine-2-carboxylique ;
le [3-(5-amino-3,6-diméthyl-6-trifluorométhyl-3,6-dihydro-2H-[1,4]oxazine-3-yl)-4-fluoro-phényl]-amide de l'acide 5-chloro-3-fluoro-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-chloro-5-trideutéro-méthoxy-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 2,5-diméthyl-oxazole-4-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 5-difluorométhoxy-3-méthyl-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-amino-5-prop-2-ynyloxy-pyrazine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-amino-5-cyano-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 5-difluorométhyl-3-méthyl-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-amino-5-difluorométhyl-pyrazine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-amino-5-difluorométhyl-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 4-difluorométhyl-6-méthoxy-pyridazine-3-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 5-cyano-3-trideutérométhoxyméthyl-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-chloro-5-difluorométhyl-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-chloro-5-trideutérométhoxy-dideutérométhyl-1H-pyrrolo[2,3-b]pyridine-6-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 3-chloro-5-trifluorométhyl-pyridine-2-carboxylique ;
le [3-((3R,6R)-5-amino-3,6-diméthyl-6-trifluoro-méthyl-3,6-dihydro-2H-[1,4]oxazine-3-yl)-4-fluoro-phényl]-amide de l'acide 5-fluoro-3-méthyl-pyridine-2-carboxylique ;
le [3-((3R,6R)-5-amino-3,6-diméthyl-6-trifluorométhyl-3,6-dihydro-2H-[1,4]oxazine-3-yl)-4-fluoro-phényl]-amide de l'acide 5-trideutérométhoxy-3-méthyl-pyridine-2-carboxylique ;
le [3-(((R)-5-amino-6,6-bis-fluorométhyl-3-méthyl-3,6-dihydro-2H-[1,4]oxazine-3-yl)-4-fluoro-phényl]-amide de l'acide 5-cyano-3-méthyl-pyridine-2-carboxylique ;
le [3-(((R)-5-amino-6,6-bis-fluorométhyl-3-méthyl-3,6-dihydro-2H-[1,4]oxazine-3-yl)-4-fluoro-phényl]-amide de l'acide 5-difluorométhoxy-3-méthyl-pyridine-2-carboxylique ;
le [3-(((R)-5-amino-6,6-bis-fluorométhyl-3-méthyl-3,6-dihydro-2H-[1,4]oxazine-3-yl)-4-fluoro-phényl]-amide de l'acide 5-chloro-3-méthyl-pyridine-2-carboxylique ;
le [3-(((R)-5-amino-3,6,6-tris-fluorométhyl-3,6-dihydro-2H-[1,4]oxazine-3-yl)-4-fluoro-phényl]-amide de l'acide 5-cyano-3-méthyl-pyridine-2-carboxylique ;
le [3-(((R)-5-amino-6,6-bis-fluorométhyl-3-méthyl-3,6-dihydro-2H-[1,4]oxazine-3-yl)-4-fluoro-phényl]-amide de l'acide 5-difluorométhyl-3-méthyl-pyridine-2-carboxylique ;
le [3-(((R)-5-amino-6,6-bis-fluorométhyl-3-méthyl-3,6-dihydro-2H-[1,4]oxazine-3-yl)-4-fluoro-phényl]-amide de l'acide 3-amino-5-difluorométhyl-pyrazine-2-carboxylique ;
le [3-(((R)-5-amino-6,6-bis-fluorométhyl-3-méthyl-3,6-dihydro-2H-[1,4]oxazine-3-yl)-4-fluoro-phényl]-amide de l'acide 3-amino-5-difluorométhyl-pyridine-2-carboxylique ;
le [3-(((R)-5-amino-6,6-bis-fluorométhyl-3-méthyl-3,6-dihydro-2H-[1,4]oxazine-3-yl)-4-fluoro-phényl]-amide de l'acide 3-amino-5-cyano-pyridine-2-carboxylique ;
le [3-(((R)-5-amino-3,6,6-tris-fluorométhyl-3,6-dihydro-2H-[1,4]oxazine-3-yl)-4-fluoro-phényl]-amide de l'acide 5-difluorométhyl-3-méthyl-pyridine-2-carboxylique ;
le [3-(((R)-5-amino-3,6,6-tris-fluorométhyl-3,6-dihydro-2H-[1,4]oxazine-3-yl)-4-fluoro-phényl]-amide de l'acide 3-chloro-5-difluorométhyl-pyridine-2-carboxylique ;
le [3-(((R)-5-amino-3,6,6-tris-fluorométhyl-3,6-dihydro-2H-[1,4]oxazine-3-yl)-4-fluoro-phényl]-amide de l'acide 3-chloro-5-trifluorométhyl-pyridine-2-carboxylique ;
le [3-(((R)-5-amino-3,6,6-tris-fluorométhyl-3,6-dihydro-2H-[1,4]oxazine-3-yl)-4-fluoro-phényl]-amide de l'acide 3,5-dichloro-pyridine-2-carboxylique ;
le [3-(((R)-5-amino-3,6,6-tris-fluorométhyl-3,6-dihydro-2H-[1,4]oxazine-3-yl)-4-fluoro-phényl]-amide de l'acide 3-amino-5-cyano-pyridine-2-carboxylique ;
le [3-(((R)-5-amino-3,6,6-tris-fluorométhyl-3,6-dihydro-2H-[1,4]oxazine-3-yl)-4-fluoro-phényl]-amide de l'acide 3-chloro-5-cyano-pyridine-2-carboxylique ;
le N-[3-(((3R,6R)-5-amino-3,6-diméthyl-6-trifluorométhyl-3,6-dihydro-2H-[1,4]oxazine-3-yl)-4-fluoro-phényl]-6-méthoxy-2-méthyl-nicotinamide ;
le N-[3-(((3R,6R)-5-amino-3,6-diméthyl-6-trifluorométhyl-3,6-dihydro-2H-[1,4]oxazine-3-yl)-4-fluoro-phényl]-6-trideutérométhoxy-2-méthyl-nicotinamide ;
le 2-amino-N-[3-(((3R,6R)-5-amino-3,6-diméthyl-6-trifluorométhyl-3,6-dihydro-2H-[1,4]oxazine-3-yl)-4-fluoro-phényl]-6-éthoxy-nicotinamide ;
le 2-amino-N-[3-(((3R,6R)-5-amino-3,6-diméthyl-6-trifluorométhyl-3,6-dihydro-2H-[1,4]oxazine-3-yl)-4-fluoro-phényl]-6-méthoxy-nicotinamide ;
2-amino-N-[3-(((3R,6R)-5-amino-3,6-diméthyl-6-trifluorométhyl-3,6-le dihydro-2H-[1,4]oxazine-3-yl)-4-fluoro-phényl]-6-trideutérométhoxy-nicotinamide ;
le 2-amino-N-[3-(((3R,6R)-5-amino-3,6-diméthyl-6-trifluorométhyl-3,6-dihydro-2H-[1,4]oxazine-3-yl)-4-fluoro-phényl]-6-pentadeutéroéthoxy-nicotinamide ;
le N-[3-(((3R,6R)-5-amino-3,6-diméthyl-6-trifluorométhyl-3,6-dihydro-2H-[1,4]oxazine-3-yl)-4-fluoro-phényl]-2-chloro-6-méthoxy-nicotinamide ;
le N-[3-(((3R,6R)-5-amino-3,6-diméthyl-6-trifluorométhyl-3,6-dihydro-2H-[1,4]oxazine-3-yl)-4-fluoro-phényl]-2-chloro-6-éthoxy-nicotinamide ;
le 2-amino-N-[3-(((3R,6R)-5-amino-3,6-diméthyl-6-trifluorométhyl-3,6-dihydro-2H-[1,4]oxazine-3-yl)-4-fluoro-phényl]-6-cyclopropylméthoxy-nicotinamide ; et
le 2-amino-N-[3-(((3R,6R)-5-amino-3,6-diméthyl-6-trifluorométhyl-3,6-dihydro-2H-[1,4]oxazine-3-yl)-4-fluoro-phényl]-6-(2,2,2-trifluoro-éthoxy)-nicotinamide ;
et leurs sels pharmaceutiquement acceptables.

3. Composé selon la revendication 1 ou la revendication 2, ou un sel pharmaceutiquement acceptable de celui-ci, destiné à être utilisé comme médicament.

4. Composé selon la revendication 1 ou la revendication 2, ou un sel pharmaceutiquement acceptable de celui-ci, destiné à être utilisé dans le traitement ou la prévention de la maladie d'Alzheimer ou l'altération cognitive bénigne.

5. Composition pharmaceutique comprenant un composé selon la revendication 1 ou la revendication 2, ou un sel pharmaceutiquement acceptable de celui-ci, comme principe actif et un support ou diluant pharmaceutiquement acceptable.

6. Combinaison comprenant une quantité thérapeutiquement efficace d'un composé selon la revendication 1 ou la revendication 2, ou un sel pharmaceutiquement acceptable de celui-ci, et une seconde substance médicamenteuse, destinée à une administration simultanée ou séquentielle.
